# EUROPEAN PATENT SPECIFICATION

(11) **EP 3 953 941 B1**
(45) Date of publication and mention of the grant of the patent: **02.07.2025**
(21) Application number: 20788689.6
(22) Date of filing: 11.04.2020
(51) Int. Cl.: G16B 50/20, G16B 5/00

(54) **SYSTEM AND METHOD FOR BIOREMEDIATION OF POLLUTANTS**
SYSTEM UND VERFAHREN ZUR BIOREMEDIATION VON SCHADSTOFFEN
SYSTÈME ET PROCÉDÉ POUR POUR LA BIOREMÉDIATION DE POLLUANTS

(30) Priority: 12.04.2019 IN 201921014894
(43) Date of publication of application: 16.02.2022
(73) Proprietor: TATA CONSULTANVY SERVICES, Maharashtra, Mumbai 400021 (IN)
(72) Inventor: ANAND, Swadha, Hadapsar, Pune 411013 (IN); MERCHANT, Mitali, Maharashtra, Pune 411013 (IN); MOHAPATRA, Anwesha, Maharashtra, Pune 411013 (IN); MANDE, Sharmila Shekhar, Maharashtra, Pune 411013 (IN); BHATT, Vineet, Maharashtra, Pune 411013 (IN); SAMPATH, Preethi Alagarai, Maharashtra, Pune 411013 (IN)
(74) Representative: Goddar, Heinz J.
(86) International application number: PCT/IN2020/050346
(87) International publication number: WO 2020/208657

(56) References cited:
- US-A1- 2013 233 094
- US-A1- 2015 290 840
- US-A1- 2018 181 702
- PAZOS F.: "MetaRouter: bioinformatics for bioremediation", NUCLEIC ACIDS RESEARCH, vol. 33, no. Database issue, 17 December 2004 (2004-12-17), GB, pages D588 - D592, XP055791489, ISSN: 0305-1048, [retrieved on 20221121], DOI: 10.1093/nar/gki068
- DVORÁK PAVEL ET AL: "Bioremediation 3.0: Engineering pollutant-removing bacteria in the times of systemic biology", BIOTECHNOLOGY ADVANCES, ELSEVIER PUBLISHING, BARKING, GB, vol. 35, no. 7, 5 August 2017 (2017-08-05), pages 845 - 866, XP085194795, ISSN: 0734-9750, [retrieved on 20221122], DOI: 10.1016/J.BIOTECHADV.2017.08.001
- RON CASPI ET AL: "The MetaCyc database of metabolic pathways and enzymes and the BioCyc collection of Pathway/Genome Databases", NUCLEIC ACIDS RESEARCH, vol. 42, no. D1, 12 November 2013 (2013-11-12), GB, pages D459 - D471, XP055294204, ISSN: 0305-1048, DOI: 10.1093/nar/gkt1103
- WICKER JÖRG ET AL: "enviPath - The environmental contaminant biotransformation pathway resource", NUCLEIC ACIDS RESEARCH, vol. 44, no. D1, 17 November 2015 (2015-11-17), GB, pages D502 - D508, XP055982953, ISSN: 0305-1048, Retrieved from the Internet <URL:https://watermark.silverchair.com/gkv1229.pdf?token=AQECAHi208BE49Ooan9kkhW_Ercy7Dm3ZL_9Cf3qfKAc485ysgAAAtowggLWBgkqhkiG9w0BBwagggLHMIICwwIBADCCArwGCSqGSIb3DQEHATAeBglghkgBZQMEAS4wEQQMZAyi3KJgIlJWTl5CAgEQgIICjdoCaXfGPAdftyyENWyOvfjVoqAuDAx_toLPMBn63H4sjx4jdjzx2qvhknW9M5kUoxlgImDB5lKrE_NNR0OsIMVgJQcp> [retrieved on 20221121], DOI: 10.1093/nar/gkv1229
- "Environmental Waste Management", 19 April 2016, CRC PRESS, ISBN: 978-0-429-08348-8, article KAPLEY ATYA ET AL: "Microbial Genomics and Bioremediation of Industrial Wastewater", pages: 185 - 216, XP093000025, DOI: 10.1201/b19243-9
- ARORA ET AL.: "Integration of bioinformatics to biodegradation", BIOLOGICAL PROCEDURES ONLINE, vol. 16, no. 8, 27 April 2014 (2014-04-27), pages 1 - 10, XP021184971, Retrieved from the Internet <URL:http://www.biologicalproceduresonline.eom/content/16/1/8> [retrieved on 20200925]
- CERQUEIRA ET AL.: "Biodegradation potential of oily sludge by pure and mixed bacterial cultures", BIORESOURCE TECHNOLOGY, vol. 102, 22 September 2011 (2011-09-22), pages 11003 - 11010, XP028326136, DOI: 10.1016/j.biortech.2011.09.074
- CHEN ET AL.: "Biodegradation of Carbon Nanotubes, Graphene, and Their Derivatives Trends in Biotechnology", TRENDS IN BIOTECHNOLOGY, vol. 35, no. 9, September 2017 (2017-09-01), pages 836 - 846, XP085171379
- WANG ET AL.: "Molecular Analysis of Isophthalate and Terephthalate Degradation by Comamonas testosteroni YZW-D", ENVIRONMENTAL HEALTH PERSPECTIVES, vol. 103, no. 5, June 2995 (2995-06-01), pages 9 - 12, XP055761122
- CHAUHAN ET AL.: "Bacterial metabolism of polycyclic aromatic hydrocarbons: strategies for bioremediation", INDIAN J. MICROBIOL, vol. 48, no. 1, 1 March 2008 (2008-03-01), pages 95 - 113, XP055761128
- WEILAND-BRAUER ET AL.: "Polychlorinated Biphenyl (PCB)-Degrading Potential of Microbes Present in a Cryoconite of Jamtalferner Glacier", FRONT. MICROBIOL., vol. 8, no. 1108, 15 June 2017 (2017-06-15), pages 1 - 17, XP055761131

## Description

### CROSS-REFERENCE TO RELATED APPLICATIONS AND PRIORITY

### TECHNICAL FIELD

The embodiments herein generally relate to the field of waste management, and, more particularly, to a method and system for bioremediation of pollutants by designing microbial communities capable of complete degradation of pollutants.

### BACKGROUND

Contamination of environment by a multitude of pollutants, most of which are produced by industrial and agricultural practices, is becoming a global health concern. Global industrialization has increased the production of several products with harmful chemical composition including plastics, pesticides, synthetic fertilizers, electronic waste, industrial waste, food additives, cleaning products, cosmetics, dyes etc. Many of these products are released into the environment and eventually enter the human food chain primarily through gastrointestinal tract but may also use other routes like airway or skin. Therefore, it is imperative to devise methods which can help in removal of pollutants from the environment as well as elimination of those that enter into the human system. A set of these pollutants have also been termed as endocrine disrupting chemicals (EDCs) as they have impact on hormonal makeup of an individual and have been associated with several metabolic disorders. Further, the exposure to chemicals might show effects on the microbes that reside in our body which are termed as 'human microbiome'.

Various physical and chemical methods are being used for the degradation of pollutants. In the existing physical and chemical methods (e.g. thermo-oxidative, photo-oxidative), the rate of the process compared to the extent of debris' accumulation is slow and the cost of implementation is high. Most of the current methods for bioremediation search for the initial enzyme within a microbe, capable of degrading the pollutant and classify such a microbe as a potential degrader. These methods do not take the entire degradation pathway into consideration. However, in most cases such enzymes are promiscuous as they bind to a range of substrates and tend to occur as multiple copies on the microbial genome. Identification of the enzyme alone therefore does not definitively establish that the pollutant can be completely degraded. Such an approach leads to increased false positive results and misleading conclusions. Moreover, it is also essential to identify the presence of key intermediates and their criticality within the pathway which is usually ignored by existing methods. Many of these intermediates and by-products (like phthalates and bisphenol A (BPA)) are left in the environment which not only pollute the environment (soil, water and air) but also affect humans by entering into the food chain and by altering the hormone levels in both males and females.

Further, in order to overcome the shortcomings of the physical and chemical methods a few bioremediation methods have also been utilized for waste management. Bioremediation is the process of using naturally occurring or deliberately introduced microorganisms such as bacteria, fungi etc. to degrade environmental pollutants from a polluted site. Microbes possess the remarkable ability to degrade a plethora of organic compounds by consuming them as their main source of energy and further assimilating them without releasing any harmful by-products. Biodegradation methods have been a preferred choice of pollution management as they are safer, inexpensive and sustainable method of remediation than the chemical and physical methods.

Majority of the bioremediation methods have been implemented to get the desired degraded products. Most of these methods fail due to incomplete or no information of the pathways involved in the degradation of pollutants by the microbes. These degrading pathways can be present in parts in different microbes of a single community. An individual microbe may not completely degrade a pollutant all by itself but it may be able to do so when it is with other microbes possessing the remaining part of degradation pathway in them. In other words a set of microbes may be able to take the intermediates produced by another set of microbes and degrade the same. Document PAZOS F.: "MetaRouter: bioinformatics for bioremediation" discloses bioremediation, the exploitation of biological catalysts (mostly microorganisms) for removing pollutants from the environment, requires the integration of huge amounts of data from different sources. Developed MetaRouter, a system for maintaining heterogeneous information related to bioremediation in a framework that allows its query, administration and mining (application of methods for extracting new knowledge). MetaRouter is an application intended for laboratories working in biodegradation and bioremediation, which need to maintain and consult public and private data, linked internally and with external databases, and to extract new information from it. Among the data-mining features is a program included for locating biodegradative pathways for chemical compounds according to a given set of constraints and requirements. The integration of biodegradation information with the corresponding protein and genome data provides a suitable framework for studying the global properties of the bioremediation network. The system can be accessed and administrated through a web interface. Document DVORAK PAVEL ET AL: "Bioremediation 3.0: Engineering pollutant-removing bacteria in the times of systemic biology" discloses elimination or mitigation of the toxic effects of chemical waste released to the environment by industrial and urban activities relies largely on the catalytic activities of microorganisms-specifically bacteria. Given their capacity to evolve rapidly, they have the biochemical power to tackle a large number of molecules mobilized from their geological repositories through human action (e.g., hydrocarbons, heavy metals) or generated through chemical synthesis (e.g., xenobiotic compounds). Whereas naturally occurring microbes already have considerable ability to remove many environmental pollutants with no external intervention, the onset of genetic engineering in the 1980s allowed the possibility of rational design of bacteria to catabolize specific compounds, which could eventually be released into the environment as bioremediation agents. The complexity of this endeavor and the lack of fundamental knowledge nonetheless led to the virtual abandonment of such a recombinant DNA-based bioremediation only a decade later. In a twist of events, the last few years have witnessed the emergence of new systemic fields (including systems and synthetic biology, and metabolic engineering) that allow revisiting the same environmental pollution challenges through fresh and far more powerful approaches. The focus on contaminated sites and chemicals has been broadened by the phenomenal problems of anthropogenic emissions of greenhouse gases and the accumulation of plastic waste on a global scale. Analyzed how contemporary systemic biology is helping to take the design of bioremediation agents back to the core of environmental biotechnology. Inspect a number of recent strategies for catabolic pathway construction and optimization and bring them together by proposing an engineering workflow. The above mentioned documents do not disclose the use of protein domains in discovery of bioremediation pathways, nor do they disclose the data structures as claimed that are used to identify microbial communities of bioremediation.

### SUMMARY

Embodiments of the present disclosure present technological improvements as solutions to one or more of the above-mentioned technical problems recognized by the inventors in conventional systems. For example, in one embodiment, a system for bioremediation of one or more pollutants is as defined in claim 13.

In another aspect, a method for bioremediation of one or more pollutants is as defined in claim 1.

In yet another aspect, one or more non-transitory machine readable information storage mediums comprising one or more instructions which when executed by one or more hardware processors cause bioremediation of one or more pollutants is as defined in claim 14.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
Fig. 1 illustrates a block diagram of a system for bioremediation of pollutants according to an embodiment of the present disclosure.
Fig. 2A-2C is a flowchart illustrating the steps involved in the bioremediation of pollutants according to an embodiment of the present disclosure.
Fig. 3A-3C is a flowchart illustrating the steps involved in the creation of knowledgebase according to an embodiment of the present disclosure.
Fig. 4 illustrates the various categories of the pollutants which can be subjected to bioremediation using the methods according to an embodiment of the present disclosure.
Fig. 5 illustrates the proximal and distal active sites present in bi-functional catalase-peroxidase enzyme in bacteria according to an embodiment of the present disclosure.
Fig. 6A-6C illustrates the schematic representation of degradation pathways of carbon-based pollutants PAH, PCB and PET respectively according to an embodiment of the present disclosure.
Fig. 7A-7B is a flowchart illustrating the steps involved in the bioremediation of carbon based pollutants according to an embodiment of the present disclosure.

### DETAILED DESCRIPTION

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts.

### GLOSSARY - TERMS USED IN THE EMBODIMENTS

The expressions microbes or organisms refers to living organisms which may include bacteria, fungi, algae, protists and viruses etc.'

The expression 'complete degrader' in the context of present disclosure refers to 'Complete Pollutant Degrader' and the expression 'partial degrader' in the context of present disclosure refers to 'Partial Pollutant degrader'.

The expression "microbial genome" in the context of present disclosure refers to microbial genome and the corresponding protein and nucleotide sequence of the genome.

The expression "degradation pathway" in the context of the present disclosure refers to the genetic machinery for degradation/elimination of these plurality of pollutants present in genomes of microbes wherein, degradation refers to conversion of a pollutant either to compounds which are assimilated into the metabolic machinery of the microbe itself or when released cause no harm to the environment,

The expression "critical intermediate" in the context of the present disclosure refers to that intermediate formed during the degradation of a pollutant which divides the pathway for the degradation of the pollutant to its constituent subpathways.

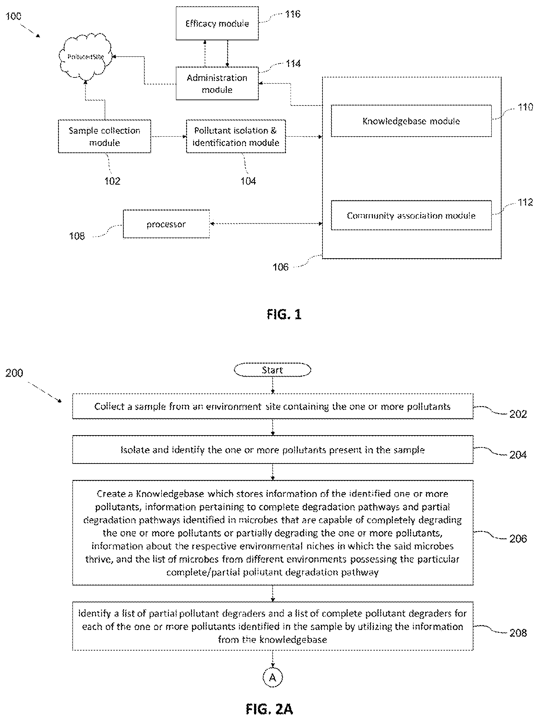

Referring now to the drawings, and more particularly to Fig. 1 through Fig. 7B, where similar reference characters denote corresponding features consistently throughout the figures, there are shown preferred embodiments and these embodiments are described in the context of the following exemplary system and/or method.

According to an embodiment of the disclosure, a system 100 for bioremediation of pollutants is shown in the block diagram of Fig. 1. The system 100 is capable of identifying different pollutants from any environmental polluted/contaminated site such as but not limited to soil, sediment, water, landfill, oil spill etc. and administration of microbial communities for their complete bioremediation of the pollutants. The system 100 helps in complete degradation of pollutants and its intermediates by a microbial community without causing any harm to the environment. Complete degradation of a pollutant refers to conversion of a pollutant to either compounds which cause no harm to the environment or those compounds which can be assimilated within microbes which reside in a given environmental site. Complete degradation may be brought about by a single microbe or a consortia/community of microbes which can combinatorially bring about complete degradation of a pollutant. The system 100 is using a method to determine the pollutant degradation potential in microbe by identifying genetic machinery in the form of degradation pathways/sub-pathways comprising of genes and/or proteins and enzymes on a microbial genome which are capable of performing these reactions for degradation of the one or more of the plurality of pollutants, determines the final products obtained from these pathways/subpathways and therefore, the extent to which the organism can degrade or change a pollutant. The sub-pathways are determined such that these subsets of complete degradation pathway for the pollutant and the genes/ proteins/ enzymes comprised thereof independently exist within certain microbes and can metabolize the pollutant to release intermediates which can be released out into the environment by a microbe and taken up by another microbe capable of metabolizing this intermediate compound to another intermediate and release it. A combination of different microbes can be designed such that this process can continue till the complete degradation of the pollutant is achieved.

This information is utilized to create a backend knowledgebase. Subsequently the knowledgebase is used to build a customized microbial community which combinatorially contribute to the pollutant degradation potential as a whole where each constituent microbe/organism augments the capacity to completely degrade the pollutant into products that can be assimilated by the microbes within an environment or can be released to the environment without any harmful effects.

In an embodiment of system 100, the community can also be designed in a way that the consortium of microbes together degrade the pollutant to an intermediate compound or metabolite and this intermediate/ product hence obtained can be utilized for several industrial and other applications. The microbes possessing sub-pathways capable of degrading the pollutant to different intermediate compounds or metabolites are partial degraders. A combination of partial microbial degraders which can together contribute all sub-pathways within a degradation pathway can form a consortia/ cocktail combinatorially capable of complete degradation of a pollutant. The system 100 can identify and establish the pollutant degradation potential of the microbes present at the environmental site, it can enhance the overall capacity of the community as well as design a community that can survive in the environment and is capable of degrading the target pollutants. The various pollutants may include plastics, organic pollutants, inorganic pollutants, aromatic pollutants, gaseous and particulate pollutant, heavy metal, carbon based nanomaterials and radioactive pollutants etc.

According to an embodiment of the disclosure, the system 100 consists of a sample collection module 102, a pollutant isolation and identification module 104, a memory 106 and a processor 108 as shown in Fig. 1. The processor 108 is in communication with the memory 106. The processor 108 is configured to execute a plurality of algorithms stored in the memory 106. The memory 106 further includes a plurality of modules for performing various functions. The memory 106 includes a knowledgebase module 110 and a community association module 112. The system 100 further comprises an administration module 114 and an efficacy module 116 as shown in the block diagram of Fig. 1.

According to an embodiment of the disclosure, the sample is collected from a site using the sample collection module 102. The sample may be collected from diverse pollutant sites such as soil industrial wastelands, soil from textile effluents drainage site, and soil dumped with sewage sludge, sediment, water bodies etc. The pollutant can also be collected from any other pollution affected site. The sample is collected using site specific methods. Sample collection methods vary on the basis of the type of environment/environmental niche (e.g. soil, sediment and water) and the type of material being collected.

Soil samples between two regions vary in terms of absorption properties, texture, density, humidity, geological setting of the site, type and population of microorganisms. Depending on the type of sampling site (e.g. cultivable disturbed land vs. uncultivable undisturbed land), the sampling depth changes. Sample extraction is carried out by tools such as augers, vehicle-mounted hydraulic auger, core barrel, trowel, Brass Sample Sleeves and solid-tube samplers. Pollutants and pollutant debris from water bodies are collected using tools like hydrological bottles, manta net, Neuston net and drift nets behind either a stationary or moving boat. The use of any other method for sample extraction is well within the scope of this disclosure.

Factors such as the type of collection site and its associated parameters of pH, salinity, temperature, pollutant concentration are all recorded during sampling. These parameters are important as the pollutant concentration and microbial distribution varies with the spatial or temporal properties. Similar such sampling techniques can be applied in various other environments for different types of pollutants.

According to an embodiment of the disclosure, the system 100 further comprises the pollutant isolation and identification module 104. The pollutant isolation and identification module 104 is configured to isolate and identify a pollutant or a plurality of pollutants from the collected sample. There are different types of pollutants present in the collected sample. The isolation and identification of different types of pollutants is done either by physical methods or by chemical methods. Pollutants are generally chemically inert and durable, thus physical methods are more frequently utilized as compared to chemical approaches. Sorting of the various pollutant entities from the sample usually forms the first step in pollutant identification and can be classified into categories such as optical or visual based sensors, floatation techniques, densitometry based sorting methods, etc. On the basis of the underlying principle, different physical methods such as near infrared sensors (NIR), electrostatic approach (e.g. tribo-electrostatic separator), Hyper-spectral imaging technology, pressurized fluid extraction (PFE), differential scanning calorimetric (DSC) and laser induced breakdown spectroscopic (LIBS) techniques can also be used to identify the different pollutants in the collected sample. The process of separating the pollutants from the sediment sample is done by density separation. The use of any other method for pollutant isolation is well within the scope of this disclosure.

Pollutant samples collected from various sites are filtered and then subjected to methods such as wet peroxide oxidation (WPO), density separation and gravimetric analysis for identifying the different types of micro-pollutants. Identification of the type of pollutant can be done by Fourier-transform infrared (FTIR) spectroscopy, Fourier transform Raman spectroscopy (FT Raman), infrared or Raman spectroscopy etc. Another method "attenuated total reflectance" (ATR) is equally adept at identifying various pollutant constituents. Toxic substances, POPs and chemicals used as additives to the pollutant material can be determined using methods like chromatography, spectrometry etc. Identification of heteroatoms (e.g. nitrogen, chlorine, sulphur etc.) in the compounds can be carried out by Laboratory heteroatoms identification techniques such as Lassaigne method, Beilstein test etc. From the results of such tests useful conclusions can be drawn, and further the results help in distinguishing between pollutant types in the query sample. GC-MS (gas chromatography-mass spectrometry) and GC-ECD (gas chromatography-electron capture detection) based methods can also be used for detection of several pollutants. Another aspect of detecting pollutants effecting human health involves immunoassays including ELISA or cell based assays. Isolation and extraction of certain pollutants follow techniques such as repetitive fractional distillation methods, ultrasonic waves in Sonication/ Ultrasonic agitation, Mechanical Agitation, Pressurized fluid extraction (PFE), rotating disk sorptive extraction (RDSE) etc. Certain pollutants like carbon based nano-materials (CBNMs) can be identified using electron microscopy. Other methods include optical detection methods, assaying the elemental ratios or isotopic signatures to determine the presence and type of CBNMs. The use of any other method for pollutant identification is well within the scope of this disclosure.

The identified pollutant or plurality of pollutants (Pᵢ) from the collected sample can be degraded by many approaches such as photo oxidation, thermo-oxidative degradation, biodegradation etc. In the present disclosure, the prospects of biodegradation are considered for the complete and efficient bioremediation of an environmental sample from all pollutants.

According to an embodiment of the disclosure, the memory 106 comprises the knowledgebase module 110. The knowledgebase module 110 creates multiple maps and matrices as explained below. All of them in collection are referred as the knowledgebase. The knowledgebase module 110 is configured to create a knowledgebase wherein the knowledgebase stores information of the identified pollutant or the plurality of pollutants, information pertaining to complete degradation pathways and partial degradation pathways identified in microbes that are capable of completely degrading the one or the plurality of said pollutants or partially degrading the one or the plurality of said pollutants, information about the respective environmental niches in which the said microbes thrive, and the list of microbes from different environments possessing the particular complete/partial pollutant degradation pathway. The complete degradation pathway refers to a set of genes on the genome of a microbe and/or proteins encoded by the microbe wherein the said set of genes and/ or encoded proteins or enzymes are responsible for complete degradation of a pollutant to either compounds that are not harmful to the environment or to compounds that can be assimilated by other microbe(s) residing within the said environment. The partial degradation pathway in a microbe refers to a set of genes or encoded proteins or enzymes that constitute one or more subpathways, wherein a sub-pathway is a subset of the complete degradation pathway encoded within genome of the said microbe. The sub-pathway degrades the pollutant to an intermediate compound which can be released out into the environment by the said microbe and is subsequently taken up by another microbe within the environment, wherein the another microbe possesses another subpathway that metabolizes the released intermediate compound.

The knowledgebase is the backend data which is used to build a customized microbial community for pollutant degradation. The knowledgebase provides information about which microbes can degrade a given pollutant and the final products formed thereof. The knowledgebase also provides the information for a particular microbe as well as pollutants that can be degraded by that microbe i.e. the total pollutant degradation potential of the microbe. In addition, the knowledgebase also provides information about the environmental conditions in which the organism has evolved to survive or colonizes in.

According to an embodiment of the disclosure, the knowledgebase module 110 is configured to create the multi-dimensional pollutant pathway organism matrix (PPOM). Following steps are being used for the same. A pathway can be defined as a series of enzymatically catalysed reactions, where the product formed in the previous reaction becomes the substrate for the subsequent reaction.

In the first step, the degradation pathways for each of the plurality of isolated pollutants (PᵢDP) is obtained by literature mining techniques. In an example, a query string (Qᵢₙ) is generated as follows for the isolated pollutant (Pᵢ). ['NameString'] + [(Aerobic) OR (Anaerobic)] + [Microbe] where NameString = (Pi) + [Degradation OR Metabolism]

The Query string (Qᵢₙ) is used as input to search against curated literature search engine like Pubmed and pathway databases such as KEGG Pathways / EAWAG (BBD/PPS)/ MetaCyc. The result set obtained from literature search engine and pathway databases contains a list of abstracts Aₒᵤₜ as output along with a pathway result set (PRSₒᵤₜ) and the list of Organisms (Oₒᵤₜ) in which the pathway is experimentally characterized.

Further, a manual search of the pathway PᵢDP (which is a series of steps of conversion of substrate pollutant to final product or intermediate compounds/metabolites) for the degradation of each of the target pollutants Pᵢ and the genes/ enzymes involved in the process is done by using the list Aₒᵤₜ and the pathway result set obtained for each input query string Qᵢₙ in the previous step. Then a manual curation and identification of all the sub-pathways is performed. Sub-pathways are identified such that the product of one sub-pathway can be taken as the initial substrate for the next sub-pathway. Therefore all sub-pathways put together can bring about complete degradation of a pollutant/compound. The criteria involved in identifying sub-pathways may include existence of the subpathway (and the constituent genes/proteins thereof) by itself on the genome of a microbe and formation of a product which can be released into the environment and made available to the microbes in the community possessing the next sub-pathway capable of utilizing this released product. This product is hereby termed as 'Critical intermediate metabolite/s' (CIMs). All the possible sub-pathways [PᵢSP1, PᵢSP2, PᵢSP3... PᵢSPn] together can construct the pollutant degradation pathway PᵢDP. The same can be explained with the help of the following example: Consider a hypothetical pathway PᵢDPwhich comprises of the following steps. Enzymes catalysing each reaction in the pathway are E1, E2... E9

| | | | | | | | |
|---|---|---|---|---|---|---|---|
| E1 | E2 | E3 | E4 | E5 | E6 | E7 | E9 |

The sub-pathways in this pathway are PᵢSP₁, PᵢSP₂ and PᵢSP₃
where PᵢSP₁ = S 1 --> S4 catalysed by (E1, E2, E3) present in organism O1
   PᵢSP₂ = S4 --> S6 catalysed by (E4, E5) present in organism O2
   PᵢSP₃ = S6 --> S10 catalysed by (E6, E7, E8, E9) present in organism O3
and PᵢDP = PᵢSP₁ + PᵢSP₂ + PᵢSP₃ O1, O2 and O3cumulatively contribute the complete degradation pathway PᵢDP for pollutant Pᵢ

These sub-pathways can be defined as subsets of the complete degradation pathway PᵢDP and the genes/ proteins/ enzymes comprised thereof that are encoded within the genomes of microbes and bring about biosynthesis of intermediates (like those depicted as S4, S6 etc.) which can be released out into the environment by the microbe and taken up by another microbe within the environment which possesses the pathway to utilize the released product.

Thus the complete degradation pathway for a pollutant Pᵢ refers to the set of genes on the genome of a microbe and/or proteins encoded by them which are responsible for complete degradation of a pollutant to compounds that are not harmful to the environment or compounds that can be assimilated by microbes residing within the said environment. The partial degradation pathway in a microbe refers to set of genes and/or encoded proteins or enzymes forming one or more subpathways (subset of the complete degradation pathway) encoded within genome of a microbe that can degrade the pollutant to an intermediate compound which can be taken up by another microbe possessing the sub-pathway for degradation of the said intermediate compound. The process can continue as a chain till a product is formed which can be assimilated by the microbes residing within the environment without a concomitant release of harmful substances into the environment. In summary, a sub-pathway is a subset of the complete degradation pathway encoded within the genome of a microbe, and wherein the sub-pathway degrades the pollutant to an intermediate compound which can be released out into the environment by the said microbe and is subsequently taken up by another microbe within the environment (206), wherein the another microbe possesses another subpathway that metabolizes the released intermediate compound. The presence of all the sub-pathways and the corresponding genes/proteins/enzymes thereof for degradation of a pollutant within a single microbe confers upon the said microbe termed as a complete degrader or a complete pollutant degrader (CPD), the capability of degrading the pollutant completely to compounds or metabolites that are not harmful to the environment or assimilate the final product within its own metabolic processes. On the contrary, partial degraders or partial pollutant degraders (PPD) refer to microbes that contribute one or more sub-pathways and the corresponding genes and/or encoded proteins or enzymes that convert a pollutant to an intermediate compound, and wherein multiple partial degraders can combinatorially contribute all sub-pathways for complete degradation of the said pollutant identified in the collected sample. Complete degradation refers to degradation of a pollutant to either a compound(s) or metabolite(s) which are not harmful to the environment or compound(s) or metabolite(s) which can be assimilated within the microbes residing the environment from where the sample has been collected. The intermediate compound(s) or metabolite(s) biosynthesized by these partial degraders can also be obtained separately using any of industrial scale methods or laboratory experimental procedures and utilized for multiple industrial and commercial applications.

Further, using the list Aₒᵤₜ obtained for each input query string Qin, searching of the Organism (Oₒᵤₜ) and the extent of pathway presence is performed. Literature validation of the sub-pathways [PᵢSP₁, PᵢSP₂, PᵢSP₃ ....PᵢSPₙ] is performed and the 'Critical intermediate metabolite(s)' (CIMs) are identified. The CIM is that metabolite formed in the course of the pathway, which can be released from one microbe within a microbial community and taken up and utilized by another complementary microbe within the community. This complementary microbe can utilize or metabolize this product as a nutrient source, substrate for secondary metabolites etc. This process can continue like a relay chain with a set of microbes acting as a consortium or community such that the intermediates released by one set of microbes within the consortia are utilized/metabolized by another set of microbes within the consortia and this sharing of released intermediates continues till the final products released are not harmful to environment or the final products are assimilated within the microbes thriving in the environment from where the sample is collected from.Later, a multi-dimensional pollutant pathway organism matrix (PPOM) is created which comprises of the target pollutant, its complete degradation pathway, the validated sub-pathways of the complete degradation pathway, the organisms where the pathway/ sub-pathway have been experimentally characterized and the literature and manually curated information about the environmental niche (e.g. soil, water, sediment etc.) from which the organism was isolated.

According to an embodiment of the disclosure, the knowledgebase module 110 is further configured to create the Genome-Pathway Master Map (GPM). The GPM is a multi-dimensional map that gives information on which subpathways for a given pollutant degradation pathway are present within genome of a candidate microbes. Creation of GPM depends on the following steps. Initially, using literature mining and manual curation techniques, microbes found in abundance in various environmental samples (e.g. soil, water surface, sediment etc.) and residing in multiple environmental niches are curated and the Database of most abundant Environmental Bacteria-Microbes (DEBG) and their corresponding environment(s)/environmental niche(s) where they are known to thrive is created. Environmental niche in present disclosure refers to the environmental conditions which an organism is known to reside or colonize in (obtained by literature mining) and has evolved to survive in. Information regarding genes sequence and location on the available sequenced bacterial genomes is obtained from National Centre of Biotechnology Information (NCBI). These bacterial genomes are functionally annotated to identify protein domains within each gene on the genome using multiple methods which may include but are not limited to gene homology (BLAST etc.), HMM based identification (Protein Family or PFAM Database etc.), Position Specific Scoring matrices (PSSM) etc. In one embodiment, the database PfamDB (or protein domain family database) comprising HMMs corresponding to all protein domains can be obtained as taught in PFAM database.

The protein domains corresponding to each of the candidate enzymes in each of the sub-pathways and pathways listed in PPOM is identified. In one embodiment, a Hidden Markov Model (HMM) based profiling of the candidate enzymes was done to procure the functional protein domain information within each protein using PfamDB as a database. Any other method can be used for obtaining functional information. This information is used to create a map namely - Pathway Domain Map (PDM) comprises of all sub-pathways within a pathway for degradation of a particular pollutant and their associated protein domains, across all the microbes stored in DEBG. For each genome sequence corresponding to microbes in DEBG, information on the location and genomic arrangement of its constituent genes is listed. The list of genes arranged as per their order on the genomes in terms of the genomic locations for each bacterial genome as obtained from NCBI is put in a map termed as Genome Map (GM). GM also contains information about functional annotations of each of these genes in the form of constituent protein domains in each gene

Further, for a sub-pathway which is the key found in the hash PDM, the value is a list of corresponding domains. The genes of a pathway/sub-pathway are often found to occur in proximity on the microbial genomes and are termed as gene clusters. The distance on the genome within which the set of domains forming a pathway or a sub-pathway should lie in order to form a functional gene cluster varies and is often defined using manual and literature based curation. This distance in this embodiment is defined in terms of the number of genes based on their genomic locations (termed as window size) within which the domains should lie in order to indicate a gene cluster and therefore a pathway/sub-pathway presence. Each associated protein domains (pfams) within PDM for each key sub-pathway, is searched in the genome map (GM) to find if the protein domains forming a subpathway occur together as a gene cluster on the genome, thereby located within a defined window size on the genome. In this embodiment, a window of 20 genes both upstream and downstream of the query protein domain (which refers to any one protein domain within a sub-pathway) on the genome is utilized. The presence of other protein domains in a sub-pathway within the window (20 in this case) is recorded in the form of gene name or pfam database based domain assignment. Window size can be variable depending on various factors like the candidate pathways and domains involved. A sub-pathway is considered to be present if the number of domains in the genome contributing to this sub-pathway and occurring within the window size (e.g. if 20 genes is window size that +20 and -20 of the query protein domain) crosses a threshold value (variable for each sub-pathway and obtained using literature mining and manual curation). The threshold value refers to the ratio corresponding to threshold minimum number of domains required to be present in order to confirm existence of sub-pathway out of the total number of domains corresponding to this sub-pathway in the PDM. From the information recorded, a multi-dimensional matrix is created with genome names and pathways/sub-pathways information for degradation of each pollutant out of the one or the plurality of pollutants identified in the said collected sample. This is called as the genome-pathway master map (GPM).

The GPM map is provided with a value of either 0 or 1 based on a first predefined criterion. The first predefined criterion is for each sub-pathway in a bacterial genome, a value of 0 is assigned to a bacterial genome if the corresponding number of sub-pathway protein domains as recorded in 'PDM' either don't occur or do not reach the threshold value within a window size of 20 genes within this genome. A value of 1 is assigned if number of sub-pathway protein domains are above the threshold value (defined for candidate pathway using literature mining and manual curation) and are present within the window of 20 genes on a microbial genome. Window size can be variable depending on the system and the candidate pathway. Finally, the results are validated against the list of organisms from the PPOM matrix where the pathway has been experimentally characterized to eliminate erroneous results.

The GPM map generated in the previous step provides information on which sub-pathways are present in a genome along with the protein domain data (pfam) and value of 1 or 0 corresponding to each sub-pathway. However, the existence of protein domains in number above the threshold for a sub-pathway is sometimes inconclusive to establish the presence of the pathway within those microbes that can degrade a particular pollutant. Some protein domains corresponding to the enzyme encoded by the gene can be promiscuous and may have multiple copies on the bacterial genome, with each of them involved in different functions and binding different substrates. In such cases further validation is necessary to annotate gene/protein function which is done by performing active site analysis of the enzymes in the present embodiment. Some protein domains belong to categories which are involved in multiple functions within a microbe. Further, these domains do not form parts of gene clusters or operons and therefore, cannot be distinguished on the basis of their genomic neighbourhood from their other homologs. In order to understand substrate specificity of these domains, the amino acid patterns corresponding to their active sites need to be accounted.

According to an embodiment of the disclosure, the knowledgebase module 110 is further configured to create genome pathway enzyme map (GPE map). The GPE map along with the GPM map helps in identification of the complete and partial pollutant degraders.

Initially, all the sub-pathways having a value of 1 for at least one genome in the GPM map for the chosen threshold value, are the filtered candidate pathways (CP), for further validation by active site analysis. The sub-pathways having all values as 0 in GPM are rejected. For each Candidate Pathway (CP) and its constituent enzymes, literature mining is done to list out the patterns specific to the active site of the query enzyme within that candidate pathway. Assume the pattern set for each candidate enzyme for a pathway (ECP) is P_{ecp}.

An active site is the region of the enzyme (protein) which binds to a specific substrate for the reaction to occur and comprises of patterns called motifs. These motifs act as signature sequences which help in identifying whether the enzyme is functionally capable to bind to a substrate and are conserved across all enzymes having similar functionality. In the present embodiment for a candidate enzyme (ECP), multiple sequence alignment (MSA) is done across all the possible functionally similar enzymes. A list of all the homologs of the ECP are identified by sequence similarity methods. These homologs of the candidate enzyme are then subjected to Multiple Sequence Alignment (MSA) to identify the conserved patterns across the enzymes. These conserved patterns are validated in literature to assess its functional importance. Patterns lying in the active site and validated by literature are termed as P_{ecp.}

Further, the Genome-Pathway-Enzyme Map (GPE map) is created. The GPE map stores the active site information for each enzyme (information about P_{ecp}.) corresponding to catalysis of each step in each sub-pathways corresponding to the degradation pathway of the said one or the plurality of pollutants identified in the collected sample. This information is obtained and recorded in GPE map for each of the bacteria (and their genomes) included in DEBG. A value of either 0 or 1 is assigned to the GPE map corresponding to each genome based on a second predefined condition/criterion. The second predefined condition/criterion is, the value of 1 is assigned to those enzymes where the corresponding active site pattern for that enzyme is found and 0 in case the pattern for the enzyme is not found.

Some of the pollutants cannot be taken in by the microbes due to their large size. Therefore, as the candidate enzymes may be secreted to extracellular environment for degrading a polymer to its monomeric or semi degraded state, it becomes necessary to test for the presence of signal peptides in the candidate enzyme ECP to ascertain its secretion capacity. Some enzymes are to be secreted outside the bacterial cell to work on the pollutant before its monomer or semi degraded intermediate is absorbed by the bacterial cell. These secreted enzymes are identified by the presence of signal peptide within the protein sequence. In the present embodiment, the testing is done using SignalP 4.1 server which is further validated using literature mining. A value of 1 is assigned to those enzymes where the secretion capability is found within the enzyme and 0 in case the secretion capability is not found. This information for each P_{ecp} is stored in GPE.

Thus for example a given pathway PᵢDP for an isolated Pollutant Pᵢ comprising of 3 sub-pathways (PᵢSP₁, PᵢSP₂ and PᵢSP₃), a microbe would be termed as complete pollutant degraders (CPD) if the value of all sub-pathways of for this microbe in GPM map is 1 and the value in GPE map is 1 for the enzymes constituent of these sub-pathways. The microbes possessing a genome having one or more of the sub-pathways for degradation of a pollutant but not the complete pathway would be termed as a Partial Pollutant Degrader (PPD). Each PPD will hold a value of 1 in GPM as well as a value of 1 for each enzyme corresponding to these subpathways present on the genome of the said PPD. Multiple organisms tagged as PPDs can contribute individual sub-pathways to combinatorially bring about complete degradation of the pollutant and can together form a microbial consortia/community for complete degradation of a pollutant. In this scenario, one or more microbes will partially degrade the pollutant to a CIM which can be released in the environment and taken up by another set of microbeswhich can utilize/metabolize this CIM and degrade to the other CIM which can be released into the environment. This combinatorial metabolism process involving different sets of microbes can continue until a metabolite is obtained which can be completely assimilated by the microbial consortia or the metabolite causes no harm to the environment when released by the microbe. The combinatorial utilization of a compound by bacterial consortia can also be designed in such a way to bring about degradation to a CIM which can be used for other applications including those in industry and other commercial purposes. This type of consortia will not result in final products that can be assimilated by microbes and completely degrade a compound but can lead to production of intermediate compounds which can be isolated and utilized for multiple industrial applications.

Thus, the pollutant degrading microbial community satisfies the following criteria:
**1.** Presence of the critical functional domains indicative of a sub-pathway derived by literature mining and manual curation.
**2.** Presence of all sub-pathways for pollutant degradation within genomes corresponding to a community of microbes either to bring about complete degradation or partial degradation to obtain intermediate products which can be rechannelled for multiple applications. All sub-pathways need not be present in a single microbial genome, but should be present in a 'community of microbial genomes' in order to consider the microbial community efficient for degradation of the pollutant.
**3.** Presence of active site patterns in the enzymes corresponding to binding of specific substrate involved in each reaction of the identified sub-pathways within microbes where the sub-pathways are identified.
**4.** Presence of the secretion capacity in the secreted involved in the reactions if degradation pathway requires an extracellular digestion of a pollutant substrate.

The DEBG is also updated with the identifier tags for complete and partial degraders of the pollutants. Further, a list for complete pollutant degrader is created and another list for partial pollutant degrader is created. And finally, the DEBG is updated with the tags PPD and CPD for the constituent genomes.

The multi-dimensional pollutant pathway organism matrix (PPOM), Genome-Pathway-Enzyme map (GPE), Genome-Pathway-Master map (GPM) and Database of most abundant Environmental Bacteria-Microbes (DEBG) together form the knowledgebase (Master Backend) in the present implementation. The knowledge base provides information on the sub-pathways present in each genome for degradation of a pollutant as well as intermediates that might be released into the environment by the organism. In addition, it provides information about the total pollutant degradation potential of the genome. The knowledgebase can be pre-created and stored in the memory for a set of well-known and common plurality of pollutants which may include but not be limited to Plastics like Polyethylene Terephthalate(PET), Styrene, Polyurethane etc., Polycyclic Aromatic hydrocarbons (PAHs) like Naphthalene, Anthracene, Pyrene etc., and different congeners of Polychlorobiphenyls (PCBs) etc. The knowledgebase can further be populated and augmented using the knowledgebase module 110 with additional set of plurality of pollutants that are not included in the pre-created knowledgebase and may be identified in the environmental site from where the sample is collected.

According to an embodiment of the disclosure, the memory 106 further comprises of the community association module 112. The community association module 112 is configured to create a map of microbes which comprises information of one or more of the partial pollutant degraders and complete pollutant degraders, capable of degrading each pollutant within the one or a plurality of pollutants identified within the sample to varying degrees of degradation. This information also includes the environmental site from where the sample is collected. This information is gathered using the DEBG, the GPM map and the GPE map. The varying degrees of degradation for a said pollutant refers to the degradation of a pollutant to different intermediate compounds or metabolites which are determined by final product(s) released by the said degrader upon the action of genes or proteins or enzymes corresponding to the sub-pathway(s) present within the genome of the said degrader for the degradation of the said pollutant, These intermediate compounds can either be released into the environment and utilized by other microbes within the environment or can be assimilated within the same microbe which carries out this degradation (210);

The community association module creates/predicts the community of microorganisms that as a whole has the functional capability to completely degrade an isolated Pollutant. The organisms from the GPE matrix having value 1 are filtered for the enzymes for which active site analysis is done. This result set is RS1. Similarly, the organisms from the GPM matrix having value 1 are filtered corresponding to its sub-pathways for an isolated Pollutant (Pᵢ). This result set is RS₂. The organisms having no active site pattern (value 0) for the query enzyme in GPE is filtered out and the organisms having no sub-pathway are filtered out. The combined result is a matrix comprising of candidate organisms result Set (CRS_{com}) which combinatorially have the functional potential to degrade each of the isolated plurality of pollutants. A subset of these organisms can be chosen such that they partially degrade a compound/pollutant to intermediate levels where the products hence formed can be channelled into multiple industrial applications.

Further, environmental information corresponding to each organism in CRS_{com} is obtained from the DEBG. This information is used to create a Pollutant Organism Environment Matrix (POEM). The matrix POEM consists of a pollutant, the organisms capable of degrading it to varying degrees (depending on complete pathway or sub-pathways present) and the environment from where the organism has been sampled. This matrix indicates the organisms which can be combinatorially utilized to completely or partially degrade a pollutant depending on the requirement. Thus, for an environmental sample, all the combinations of organisms capable of surviving in an environment and functionally capable of partially or completely degrading a target pollutant can be concocted and a customized microbial community can be designed. The customized microbial community can comprise of microbes wherein different sub-pathways within each microbe can be combined (cumulatively forming the complete pollutant degradation pathway) to partially/completely degrade the pollutant even in cases where single constituent microbe lacks the degrading capability.

In an embodiment, the method discussed above can be utilized to design microbial communities which possess functional capabilities to degrade multiple pollutants. The minimal community required to degrade multiple pollutants in a polluted site can be identified using the knowledgebase. This minimal community will comprise of a set of microbes which can survive in an environmental condition, possess sub-pathways corresponding to complete degradation of a plurality of pollutants. Thus, microbes belonging to these communities can combinatorially contribute all constituent sub-pathways for complete degradation of each of the pollutants identified in an environmental polluted site. It should be appreciated that one microbe might be responsible for contributing a set of sub-pathways for degradation of more than one pollutant also. The microbial communities so designed which comprise of microbes which are complete or partial degraders of the one or plurality of pollutants present in the collected sample can be used to design first microbial consortia comprising microbes capable of existing together in the said environment and combinatorially degrading the one or the plurality of pollutants in the environmental site the sample has been collected from. The degradation will depend on the presence of all subpathways (genes and/or proteins and enzymes corresponding to the sub-pathway) for degradation of the pollutant(s) identified in the environmental site within the genomes of the set of microbes forming the consortia

The method described in the disclosure can also be re-purposed for applications other than bio-remediation of pollutants. In one embodiment, the method described can be used to produce compounds that are of commercial use within industries. For example, in case of bio-remediation of Polyethylene Terepthalic Acid (PET) by bacteria, terephthalic acid (TPA) and ethylene glycol (EG) are produced as intermediates. Using these intermediates as raw materials, TPA finds applications in multiple industries like those involved in packaging, textiles etc. making use of polymer and polyesters. Another set of bacteria can then be added to this microbial community which can convert EG to compounds of industrial use such as glycolate, which is used extensively in the cosmetic industry. Polyhydroxyalkanoates (PHAs), which are most common class of bioplastics available in the industry can also be made using TPA as a raw material by augmenting the microbial consortia utilized to form TPA from PET with the set of bacteria responsible for converting TPA to PHA as can be obtained from the knowledgebase. In another embodiment, the method described here can be used to convert the intermediates to recycle the parent compound for industrial use. For example, TPA and EG obtained from bio-remediation of PET pollutants can be used to make new PET polymers, which can then be used in the industry for the production of various PET based products. Therefore, the intermediates formed as CIMs in this method can be isolated and utilized for various industrial purposes. In these embodiments, the consortia is designed such that it only degrades a pollutant/compound to an intermediate compound which can be isolated for further industrial uses instead of completely degrading the pollutant.

In another embodiment, the method described herein can be used to identify microbes that causes degradation of important industrial compounds, thereby causing huge loss to the industries. For example, asphalt used in the construction of roads is sometimes degraded by bacteria causing pinholes to form on the surface of the roads thereby hampering their structural stability. Such bacteria with pathways present to degrade asphalt and corresponding sub-pathways can be identified using the method described in the present disclosure and can be targeted. Any other process through which the invention described herein can be used for industrial application is within the scope of this disclosure. These microbial communities which comprise of combination of partial degraders which metabolize or degrade the said one or the plurality of pollutants can be designed such that the intermediate compounds biosynthesized by the partial degraders can be obtained and repurposed for multiple industrial applications. This can be used as a second microbial consortia to partially degrade a pollutant to obtain intermediate compounds of industrial importance which may cater to industries like but not limited to packaging, automotive, oil and gas, food and beverage, textiles, paints and lubricants etc.

According to an embodiment of the disclosure, the system 100 further comprises the administration module 114. The administration module 114 is configured to administer the concocted customized microbial community over the environmental site from where the sample has been collected. The administration results in complete and efficient degradation of the plurality of pollutants from the site.

The method of administration of a bioremediation technique varies depending on the type of contamination site, the degree of pollution, location, cost, and environmental policies specific to the site. Different pollutants have been observed to contaminate various sites such as soil, waste water, sludge from industries and aquatic environments (water bodies especially oceans, lakes and rivers). The administration methods can be broadly categorized as two types: namely *ex-situ* and *in-situ* bioremediation.

In *Ex-situ* method of bioremediation, the pollutants are excavated from a polluted site, transported to another area for processing and then eventually returned to the site post treatment.

In case of *In-situ* method, bioremediation takes place at the contaminated site itself.

While ex-situ methods are more effective, they are not economically viable to do so when large contaminated areas are being targeted. Although multiple types of bioremediation methods exist and are in industrial use, in this embodiment, two categories of bioremediation have been disclosed below based on the site of contamination as well as the type of bioremediation.

*Ex-situ* administration: The method for *Ex-situ* bioremediation differs on the basis of the phase of the contaminated material and can be classified as: (i) solid-phase system (using techniques such as land-farming, soil piles and composting) and (ii) slurry-phase systems (involving treatment of solid-liquid suspensions in bioreactors).

Solid-phases systems are useful for large quantities of waste materials and require favourable conditions such as moisture content, frequent aeration, mixing (mechanical and air mixing), pH and inorganic nutrients for microbial growth. In the process of land-farming the contaminated soil is spread into a lined bed (to prevent leaching) and regular mixing of the soil is done for availability of nutrients and oxygen for the microbes. In case of bio-piling the polluted soil samples are placed as piles over top of a bug vacuum pump. This vacuum pump maintains a steady flow of oxygen keeping the sample well aerated and nutrients are added for hastening the process of bioremediation. The conditions are monitored to ensure efficient bioremediation.

In slurry-phase systems, contaminated solid materials from the site of application along with microorganisms and water (all the components are formulated into slurry) are brought within a bioreactor. A bioreactor is a large vessel which converts the raw materials into different products via a series of biological reactions. The process of bioremediation in a bioreactor is one of the most common methodology by which contaminated soil/water can be treated. In this process, the bioreactor is maintained in the optimal conditions for microbial growth and the pollutant in the raw material (contaminated soil/water) is metabolized. The microbes added here are pollutant degrading microbes or microbial community identified through our pipeline. The raw material can be any sample extracted from any pollutant contaminated site. The bioprocess parameters necessary for the process such as temperature, pH, agitation and aeration rates, substrate and inoculum concentrations can be controlled externally which makes this a preferred technique as the bioremediation rate can be effectively improved. Post treatment, the treated soil/water can be restored into their original site. One advantage of using bioreactor bioremediation is the use of engineered microbial community. Since it is an enclosed system the engineered microorganisms can be destroyed before restoration of treated soil/water, thereby ensuring that the engineered microorganisms do not enter the ecosystem.

*In-situ* administration: *In-situ* bioremediation techniques are comparatively less costly compared to *ex-situ* methods as they do not involve any excavation. However, the method does require sophisticated equipment for improving microbial activities and its cost of design as well as on-site installation increases expenditures incurred for the process. In-situ methods of bioremediation techniques might be naturally attenuated as in intrinsic bioremediation or proceed with some enhancement (bioventing, bio-sparging and phytoremediation). Microbes have the innate capacity to degrade metabolites and consume it as a source of carbon. Bioremediation methods which exploit and manage the existing capabilities of naturally occurring microbes to degrade contaminants without applying any engineering steps to enhance the process are classified as intrinsic bioremediation. Bioventing method, involves a continuous supply of a steady stream of oxygen as well as nutrient and moisture to unsaturated (vadose) zones of the site in order to enhance the activity of the indigenous microbes to degrade the pollutant in the contaminated soil or water.

The microbes identified in the present methodology as pollutant degraders can be applied to achieve efficient bioremediation activity. The above mentioned methods are some of the ways in which bioremediation can be administered to pollutant contaminated environment.

According to an embodiment of the disclosure, the system 100 also comprises the efficacy module 116. In the efficacy module 116, post administration of the pollutant degrading microbe or the microbial community, the contaminated site must be evaluated in frequent intervals to check for the presence of pollutant contamination and the rate of pollutant degradation. Any of the methods discussed in pollutant identification module 104 can be used for assessment of efficacy of designed microbial communities for bioremediation of environment pollutants. Any other accepted methodology of assaying the presence of pollutants is within the scope of this disclosure. Based on the level of contamination present after assaying, necessary modifications can be made to the first and second microbial consortia to accelerate the bioremediation of these pollutants. Modifications include but is not limited to inoculation with multiple titers of bacterial community designed as first and second microbial consortia, modifying the administered bacterial community and augmenting with additional CPD and PPD microbes which can survive in the said environment niche based on the information stored in knowledgebase, addition of essential nutrients, better aeration to the contaminated environment etc., to further boost the pollutant degradation.

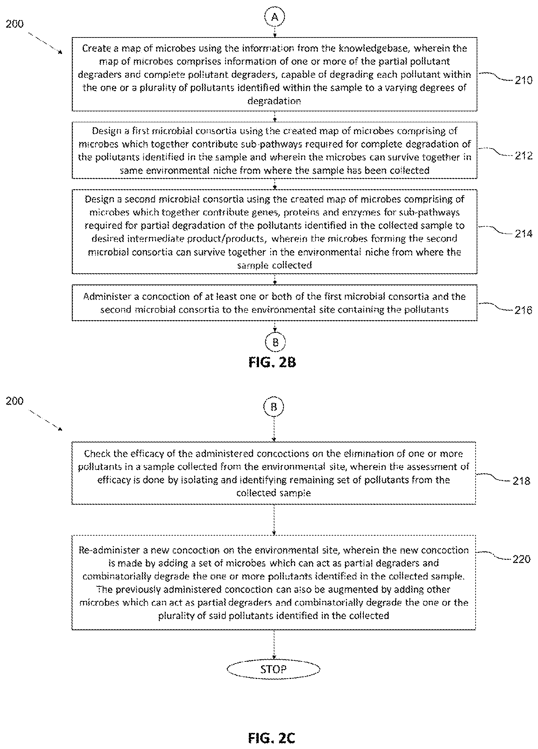

In operation, a flowchart 200 illustrating the steps involved for bioremediation of pollutants is shown in Fig. 2A-2C. Initially at step 202, the sample is collected from an environment site containing the one or more pollutants. At step 204, the one or more pollutants present in the sample are isolated and identified.

At step 206, the knowledgebase is created. The knowledgebase stores information of the identified one or more pollutants, information pertaining to complete degradation pathways and partial degradation pathways identified in microbes that are capable of completely degrading the one or more pollutants or partially degrading the one or more pollutants, information about the respective environmental niches in which the said microbes thrive, and the list of microbes from different environments possessing the particular complete/partial pollutant degradation pathway. The complete degradation pathway refers to a set of genes on a genome of a microbe and/or proteins encoded by the microbe wherein the set of genes and/ or encoded proteins are responsible for complete degradation of a pollutant either to compounds that are safe for the environment or to compounds that can be assimilated by other microbe(s) residing within the environment. The partial degradation pathway in the microbe refers to a set of genes or encoded proteins that constitute one or more sub-pathways. A sub-pathway is a subset of the complete degradation pathway encoded within genome of the microbe, and the subpathway degrades the pollutant to an intermediate compound which can be released out into the environment by the microbe and is subsequently taken up by another microbe within the environment, wherein the another microbe possesses another sub-pathway that metabolizes the released intermediate compound.

At step 208, the list of partial pollutant degraders and the list of complete pollutant degraders are identified for each of the one or more pollutants identified in the sample by utilizing the information from the knowledgebase. The partial pollutant degraders refer to microbes that contribute one or more subpathways and the corresponding set of genes, encoded proteins or enzymes that convert a pollutant to an intermediate compound. While the multiple partial degraders can combinatorially contribute all sub-pathways for complete degradation of the pollutant identified in the collected sample. The complete pollutant degraders possess a combination of all sub-pathways and the corresponding set of genes, encoded proteins or enzymes within a single microbe for degradation of the pollutant identified in the collected sample.

At step 210, the map of microbes is created using the information from the knowledgebase. The map of microbes comprises information of one or more of the partial pollutant degraders and complete pollutant degraders, capable of degrading each pollutant within the one or a plurality of pollutants identified within the sample to a varying degrees of degradation. The varying degrees of degradation for the pollutant refers to the degradation of a pollutant to different intermediate compounds or metabolites and the intermediate compounds or metabolites are determined by final product(s) released by the degrader upon the action of genes or proteins or enzymes corresponding to the sub-pathway(s) present within the genome of the degrader for the degradation of the pollutant. The intermediate compounds can either be released into the environment and utilized by other microbes within the environment or can be assimilated within the same microbe which carries out this degradation.

At step 212, the first microbial consortia is designed using the created map of microbes comprising of microbes which together contribute subpathways required for complete degradation of the one or more pollutants identified in the sample and wherein the microbes can survive together in the same environmental niche from where the sample has been collected.

At step 214, the second microbial consortia is designed using the created map of microbes comprising of microbes which together contribute genes, proteins and enzymes for sub-pathways required for partial degradation of the one or the plurality of pollutants identified in the said collected sample to desired intermediate product/products. The microbes forming the second microbial consortia can survive together in the environmental niche from where the sample has been collected.

At step 216, the concoction of at least one or both of the first microbial consortia and the second microbial consortia is administered to the said environmental site containing the one or the plurality of pollutants. The method of administration of a bioremediation technique varies depending on the type of contamination site, the degree of pollution, location, cost, and environmental policies specific to the site. The administration methods can be broadly categorized as two types: namely ex-situ and in-situ bioremediation as explained above.

At step 218, the efficacy of the administered concoctions is checked on the elimination of one or more pollutants in a sample collected from the environmental site. The assessment of efficacy is done by isolating and identifying remaining set of pollutants from the collected sample. At step 220, a new concoction is re-administered on the environmental site. The new concoction is made by adding a set of microbes which can act as partial degraders and combinatorially degrade the one or the plurality of said pollutants identified in the collected sample. The previously administered concoction can also be augmented by adding other microbes which can act as partial degraders and combinatorially degrade the one or the plurality of said pollutants identified in the collected sample.

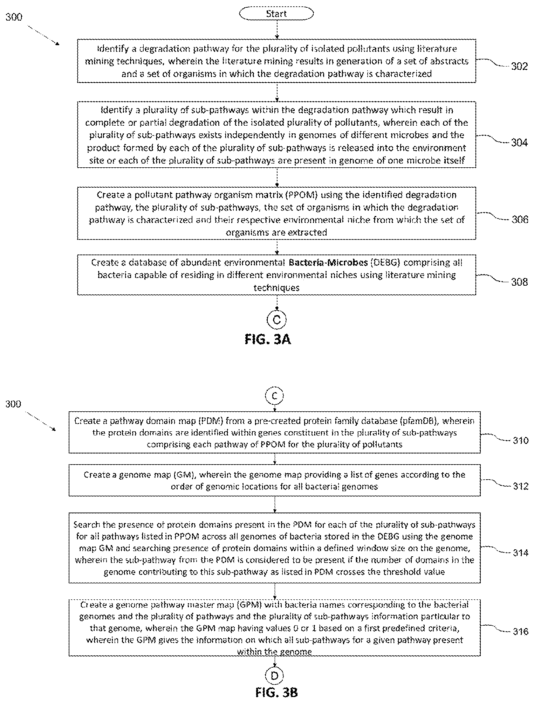

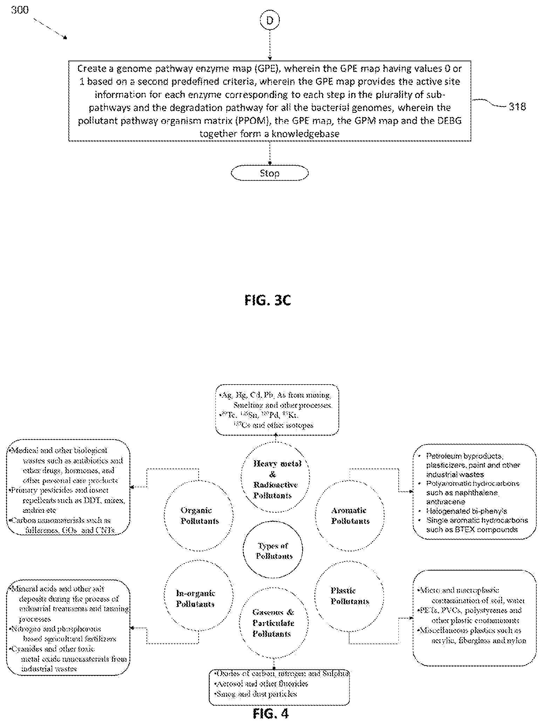

A flowchart 300 for creating the knowledgebase is shown in Fig. 3A-3C. Initially at step 302, the degradation pathway(s) for the plurality of isolated pollutants are identified using literature mining techniques. The literature mining also results in information about a set of microbes in which the pathway(s) have been experimentally characterized as well as the environment niche(s) from where these said microbes have been isolated. Similarly at step 304, the plurality of subpathways are also identified within the degradation pathway which result in partial/complete utilization/assimilation of the isolated plurality of pollutants. In an embodiment, each of the plurality of sub-pathways exists in genomes of different microbes called Partial Pollutant degraders (PPD) and the product formed by each of the plurality of sub-pathways is released into the environment site and is metabolized or is taken up by other microbe(s) inhabiting the environment. In another embodiment, each of the one or the plurality of sub-pathways are present in genome of one microbe itself called as a Complete Pollutant degrader (CPD);

At step 306, the pollutant pathway organism matrix (PPOM) is created using the identified degradation pathway for each one or the plurality of identified pollutants, the plurality of sub-pathways for the degradation pathway, the set of organisms in which the degradation pathway is characterized and information based on literature mining and manual curation about the respective environmental niche/niches from which the said set of microbes are isolated. At step 308 the Database of Abundant Environmental Bacteria-Microbes (DEBG) is created using literature mining techniques. The DEBG comprising information pertaining to all microbes and the different environmental niches they thrive in.

At step 310, the pathway domain map (PDM) is created from a pre-created protein family database (pfamDB), wherein the protein domains included in the PDM are those corresponding to genes/proteins constituting the plurality of sub-pathways that comprise each degradation pathway present in the created PPOM for the plurality of pollutants. At step 312, the genome map (GM) is created, wherein the genome map providing a listing of genes/proteins ordered as per their respective genomic locations in a microbe as well as the constituent protein domains within these genes/proteins. In the next step 314, presence of protein domains included in PDM for each of the plurality of sub-pathways for all pathways listed in PPOM is searched on the genomes of microbes stored in the DEBG to determine occurrence of these sub-pathways on the genomes; wherein the search is performed using the genome map GM as a database and wherein the sub-pathway from the PDM is considered to be present if the number of domains in the genome contributing to this sub-pathway as listed in PDM occur within a window size of genes on the genome and cross a predefined threshold value

At step 316, the genome pathway master map (GPM) is created with microbial names corresponding to the microbial genomes in DEBG, and information about presence or absence of the said plurality of pathways and the said plurality of sub-pathways on the genome, for each of the one or the plurality of pollutants identified in the collected sample, and wherein the GPM map has a value of 0 or 1 based on a first predefined criterion, and wherein the GPM provides the information about all sub-pathways for a given pollutant degradation pathway that are present within each of the microbial genomes listed in the GPM.

At step 318, the genome pathway enzyme map (GPE) is createdwhich comprises of all microbial names listed in the DEBG, information about active site of each enzyme involved in each step of the plurality of sub-pathways on each genome, for each of the one or the plurality of pollutants identified in the collected sample, wherein the GPE map has a value of 0 or 1 based on a second predefined criterion. The pollutant pathway organism matrix (PPOM), the GPE map, the GPM map and the DEBG together form the knowledgebase.

According to an embodiment of the disclosure, the system 100 can be used for bioremediation of plurality of pollutants which may include but not be limited to plastics (Polyethylene terephthalate (PET), Styrene etc.), rubber, pesticides, synthetic fertilizers, electronic waste, industrial waste, food additives, cleaning products, cosmetics, dyes etc. as shown in Fig. 4. In another embodiment, in addition to removal of pollutants, system 100 can also be used for repurposing the products and intermediates within this process for various other applications including those in industry.

In one embodiment of this disclosure, system 100 can be applied for bio-remediation of carbon-based pollutant such as carbon based nanomaterials (CBNMs), PAHs, PCBs and PET. In case of CBNM degradation, the methodology may follow a two-step process to determine if a bacterial community or a bacterium is capable of degrading CBNMs such that the pollutant is either converted to substances which are not harmful to living beings and environment or are completely assimilated within the bacteria residing in the environment from where CBNM is isolated as a pollutant. The first step is to identify the presence of a key secretory peroxidase enzyme in the bacterium or the bacterial community. The second step is to identify the presence of aromatic degradation ability (such as PAHs, single aromatic hydrocarbons (SAHs) and PCBs). Bacteria are known degraders of PAHs and PCBs. The methodology looks for the presence of the genetic machinery in the bacterial genome that are essential for degradation of PAHs and PCBs. The method postulates that if these two features are present in the bacterium or can be combinatorially contributed by members in the bacterial community, then they are capable of completely degrading CBNMs.

CBNMs are the order of magnitude 1-1000nm (although most of them fall in the range of 10-100nm), hence cannot be internalized by the bacteria, which are of the size of 2µm, i.e., 2000nm for degradation. Therefore, it is possible that the initial degradation of CBNMs might happen outside the bacterial cell, mostly by the peroxidase enzyme. However, it is not known what might be the bacterial peroxidase that can possibly degrade nanomaterials. In this embodiment, the bacterial enzyme *catalase-peroxidase* (*kat*) is identified as the potential peroxidase enzyme that may be capable of degrading CBNMs. Additionally, it is identified that the presence of secretory catalase-peroxidase in the bacterial community or the bacterium is essential for degradation of CBNMs.

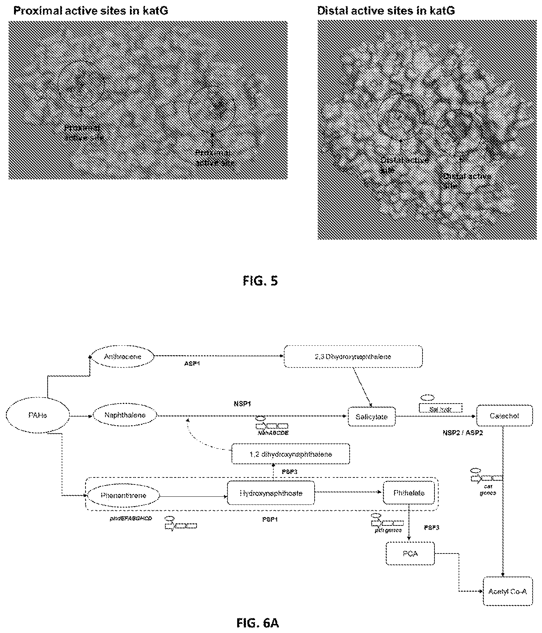

Multiple eukaryotic peroxidases have been experimentally shown to degrade CBNMs, of which plant secretory peroxidase horseraddish peroxidase (HRP) can degrade various types of CBNMs such as single walled carbon nanotube (SWCNT), graphene oxide (GO), reduced graphene oxide (RGO) and multi-walled carbon nanotube (MWCNT) etc. In the present disclosure, it is postulated that bi-functional secretory catalase-peroxidase may possess the capability to degrade CBNMs in bacteria. Catalase-peroxidase, although a prokaryotic peroxidase, shares a high structural similarity with HRP. The presence of a distal active site along with the proximal active site in catalase-peroxidase is known and the CBNM ligands may bind to either the proximal or the distal active sites of the enzyme as shown in Figure 5. Both the proximal and the distal active site cavities are lined with various aromatic amino acid residues such as Tryptophan (Trp), Tyrosine (Tyr) and Phenylalanine (Phe) etc along with various other polar residues such as Arginine (Arg). These amino acid residues may help in stabilizing the binding of CBNM at the proximal and the distal active sites. Additionally, the distal active site of the enzyme is connected to the central heme cavity through a series of non-polar aromatic amino acid residues. The electron transfer from the heme active site to the distal cavity where CBNM binds may occur by electron hopping through the aromatic amino acid bridges in the enzyme especially via W176 residue. These results indicate that secretory bi-functional catalase-peroxidase may be the bacterial peroxidase that can degrade CBNMs.

Bi-functional catalase-peroxidases belong to family III of peroxidase superfamily. The main function of this enzyme is in scavenging H₂O₂, thereby protecting the bacterial cell from oxidative stress. Catalase-peroxidase may be performing the following reactions in the presence of CBNM pollutants.

| | |
|---|---|
| CBNM + H₂O₂ | Oxidized-CBNM + 2 H₂O |
| 2 H₂O₂ | O₂ + 2 H₂O |

In the present disclosure, it has been stated that the bi-functional catalase-peroxidase enzyme is capable of degrading CBNMs, provided they are secreted outside the bacterial cell. This allows them to access the bigger CBNMs for degradation. Since the enzyme catalase-peroxidase is highly conserved across all bacteria containing this enzyme, similar results are expected for all the enzyme homologs which are present in other bacteria. The CBNMs are converted to intermediates of the category PAHs or PCBs which are also pollutants and need to be further degraded in order to bring about complete CBNM degradation. PAHs and PCBs are themselves a part of multiple industrial wastes responsoble for environmental pollution. Therefore, removal of these compounds form environment is also necessary.

PAHs are organic compounds comprising of multiple aromatic rings made of carbon and hydrogen as shown in FIG. 6A. In this disclosure low molecular weight PAHs (LMW PAHs) such as Naphthalene, Anthracene and Phenanthrene have been analyzed as per the methodology described. Degradation methods for other PAHs are also well within the scope of this disclosure. Using the method for literature mining used in our study it was found that LMW PAHsare biodegradable and usually favor aerobic degradation by the action of oxygen-mediated metabolism, followed by dehydrogenases and the subsequent ring cleavage by the dioxygenases to form TCA cycle intermediates which can be easily assimilated by the organism. Literature mining of pathway in literature search engines such as PubMed and Pathway Databases such as KEGG Pathways/ EAWAG (BBD/PPS)/ MetaCyc. The query string can be PAH (e.g. Naphthalene) Degradation + Aerobic + Bacteria. Using the above mechanism, the pathways, their corresponding enzymes, the critical intermediates, model organisms and gene clusters are identified . The cluster of genes (coding for their enzymes) involved in each subpathway is searched across all the genomes. Organisms having the clusters are further validated by the presence of structurally significant patterns found in the active site of that enzyme. Bacterial genomes or consortia of bacteria having all the sub-pathways as well as the active site pattern is deemed as a PAH degrader. Naphthalene degradation pathway as illustrated in FIG. 6A comprises of two subpathways namely NSP1 and NSP2 where i) Naphthalene is converted to Salicylate (NSP1) and ii) Salicylate can further be converted to Catechol (NSP2) which is eventually degraded into compounds that can be assimilated by the bacterial genome via the *cat* gene cluster which is evolutionarily conserved in bacteria. Similarly the three ringed Anthracene, is degraded via a set of two sub-pathways ASP1 and ASP2. The former subpathway (ASP1), as shown in FIG. 6A, involves a series of enzymatic steps which converts i) Anthracene to 2,3-Dihydroxynaphthalene following which the compound so formed is also converted to ii) Salicylate which is further degraded via the catechol pathway (ASP2). The intermediate Salicylate, which is degraded via catechol metabolism, forms the common critical intermediate (CIM) in both naphthalene and anthracene degradation. Degradation pathway of the other three ringed PAH-Phenanthrene analyzed in our study, is divided into three sub-pathways namely i) conversion of phenanthrene to 1-Hydroxy-2-naphthoate, regulated by its gene cluster and forms phthalate (PSP1). ii) Phthalate is degraded to 3,4 Dihydroxybenzoate (Protocatechuic Acid) via the sub-pathway regulated by *pth* gene cluster and eventually gets assimilated into bacterial metabolism via benzoate degradation(PSP2). iii) Phenanthrene can be degraded via the sub-pathway which forms 1,2 Naphthalenediol and further gets degraded via naphthalene metabolism(PSP3).

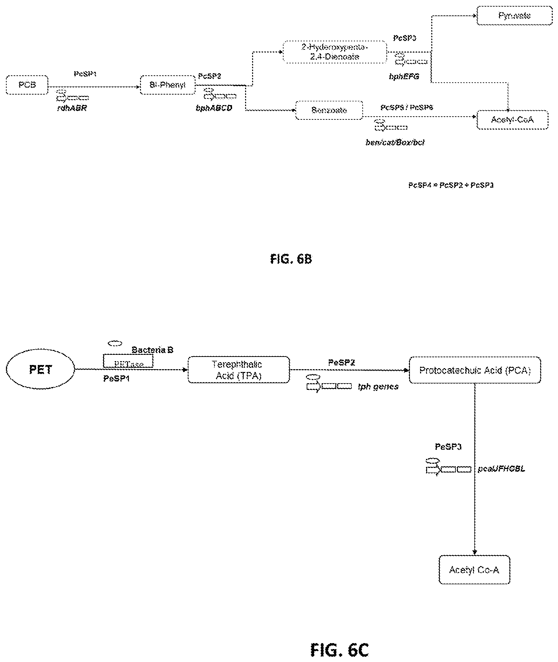

The other aromatic intermediate formed during the degradation of CBNM are biphenyls which are the reduced (dehalogenated) forms of PCBs. The process of reductive dechlorination of higher chlorinated biphenyls to lower chlorinated Biphenyls involving the *rdhABR* gene cluster has been included as a sub-pathway (PcSP1) as illustrated in FIG. 6B and is brought about by specialized community of Organohalide-respiring bacteria under anaerobic conditions. The lower chlorinated biphenyl derivatives are further degraded under aerobic conditions to 2-hydroxypenta-2,4-dienoate and benzoate via action of biphenyl dioxygenase (*bphA*) activity involving gene cluster *bphABCD* well known as upper pathway(PcSP2). 2-hydroxypenta-2,4-dienoate is further degraded to pyruvate via another well studied distinct gene cluster bphEFG known as lower pathway (PcSP3) of degradation. Few organisms possess both PcSP2 and PcSP3 together as a complete gene cluster for biphenyl degradation and termed as PcSP4. These pathways specific to PCB degradation which lead to the formation of intermediates such as Benzoate. Degradation of benzoate proceeds via either catechol or benzoyl CoA metabolism pathway (termed as PcSP5 and PcSP6 respectively).

According to an embodiment of the disclosure, the system 100 can also be explained with the help of following example for Polyethylene terephthalate (PET). The above methodology can be used to infer the PET-pollutant (Polyethylene terephthalate) degradation capacity of bacteria.

Bacterial degradation of PET involves 3 major sub-pathways (a) Hydrolysis of PET to its monomers like Bis-hydroxyethyl terephthalate (BHET), Mono-hydroxyethyl terephthalate (MHET) or Terephthalic acid (TPA). (b) Conversion of MHET to TPA and (c) reduction of TPA to Protocatechuic Acid (PCA). After a thorough literature mining, PETase enzyme fr*om Ideonella sakeins*is is considered in this analysis due to its efficient activity in breaking of the polymer to its monomers. Its remote homologs are identified using PSI-BLAST in this embodiment. The use of other methods of gathering these homologs are also well within the scope of this disclosure. This PETase exists as a monomer and structurally belongs to the α/β hydrolase superfamily, which is strictly conserved across all esterase proteins such as lipases and cutinases. Similar to other α/β hydrolases, the enzyme PETase has the conserved catalytic triad S131-H208-D177 and a serine hydrolase motif of Gly-x1-Ser-x2-Gly on the active site. However, the presence of two intra-molecular disulphide bridges, formed near the catalytic centre - DS1 and DS2, form a unique feature of PETase where other hydrolases have only one disulphide bridge. The remote homologs are further filtered taking into account the presence of DS1 disulphide-bridge along with the conserved patterns of α/β hydrolase superfamily. The sequences of these remote homologs are aligned and their multiple sequence alignment is used to create a Hidden Markov model for PETase (PET-Pfam). PETase is a secretory protein and is known to be secreted to the extracellular environment which in turn provides easy and optimized PET accessibility. The secretion capacity of the potential genes having PET-Pfam was confirmed by using SignalP 4.1 server to identify signal peptides in the query gene.

The conversion of TPA to PCA is a two-step process involving two enzymes. The final output of this step (i.e. PCA) is a functionally important intermediate found to be conserved in bacteria. Thus degradation of PET involves conversion of PET by the action of PETase enzyme leading to the formation of its constituent monomers such as TPA and is denoted as PeSP1. The action of PETase is the rate limiting step and is denoted as the Specific Pathway PeSP1. The subpathway for degrading TPA to form Protocatehuic Acid (PCA) is denoted as PeSP2 . PCA is degraded to form Acetyl CoA and is regulated by *pcaIJFHGBL* gene cluster. This sub-pathway is common to degradation of PET and even in case of Phenanthrene and is denoted as PeSP3 (Illustrated in Figure 6C).

Bacteria having PETase but no TPA sub-pathway is a potential PET degrader. Conversely bacteria having TPA sub-pathway but no PET to TPA conversion is a potential TPA degrader. A group of bacteria which can combinatorially contribute all the sub-pathways (TPA to PCA and PCA to catechol) along with PETases with appropriate active sites and signal peptides are considered to be Complete PET Degrading microbial community. A bacterium having the subpathways, the active site patterns along with the signal peptide is considered to be a Complete PET Degrader. A single microbe can have all the sub-pathways within it or a community of microbes may contribute in the sub-pathways for collective and efficient degradation of the pollutant. Microbial concoctions comprising of different microbial combinations, contributing to each of the sub-pathways identified can be obtained using the list of organisms maintained with inventors due to the size constraint. The sheet can be provided to the examiners based on the request. In another embodiment, these concoctions will lead to complete degradation of PET to products that can be assimilated by the bacteria. Further microbial concoctions can also be designed to comprise of organisms that can degrade PET to different intermediate levels where products formed can have multiple industrial applications. For example, a microbial consortium comprising of multiple organisms capable to degrading PET to TPA and EG can be created using the list of organisms provided. This microbial consortium can lead to formation of these two intermediates TPA and EG which can be isolated and utilized for multiple industrial applications.

According to an embodiment of the disclosure, the system 100 can also be explained with the help of examples of various table utilized in the knowledgebase.

**Pollutant pathway organism matrix (PPOM):** This matrix includes a list of various pollutants, the degradation pathway and the identified subpathways for the respective pollutants, organisms in which these pathways are experimentally characterized, and the environmental niche(s) from which these organisms are identified. Table 1 represents the format of PPOM. While a sample PPOM for pollutant PET is shown in Table 2.

**TABLE 1: Format of PPOM**

| **Pollutants** | **Pathways** | **Pathway description** | **List of organisms** | **Sample type** |
|---|---|---|---|---|
| **Pollutant 1** | Sub-pathway 1 | **pollutant** 1 -> B | org 1, org2 | soil, aquatic |
| | Sub-pathway 2 | B->C | org3,org2 | sediment, aquatic |
| | Sub-pathway 3 | C->D | org2 | aquatic |
| **Pollutant 2** | Sub-pathway 1 | pollutant 2 ->Y | org4, org3 | landfill, sediment |
| | Sub-pathway 2 | Y->Z | org5, org6, org7 | soil, groundwater, soil |
| **Pollutant 3** | Sub-pathway 1 | K->L | org8, org9 | sediment, soil |
| | Sub-pathway 2 | Pollutant 3 ->M | org8, org10 | sediment, soil |
| | Sub-pathway 3 | M->N | org 10 | soil |
| | Sub-pathway 4 | N->O | org 10 | soil |
| .... | ... | ... | ... | ... |

**TABLE 2: A sample PPOM for pollutant PET**

| **Pollutants** | **Pathways** | **Pathway description** | **List of organisms** | **Sample Type** |
|---|---|---|---|---|
| **PET (polyterephthalic acid)** | Subpathway 1 | PET -> TPA | Ideonellasakeinsis | sediment |
| | Subpathway 2 | TPA -> PCA | Ideonellasakeinsis, Comamonas sp. | sediment, soil |
| ..... | ..... | .... | ..... | ..... |

**Database of abundant environmental bacteria-microbes** (DEBG):This database includes the microbial genomes reported till date with the environment in which these microbes were isolated. A sample DEBG is shown in Table 3.

**TABLE 3: A sample database of abundant environmental Bacteria-Microbes (DEBG)**

| **Genome ID** | **Organism Name** | **Status** | **Sample Type** |
|---|---|---|---|
| GCA_001017435.1_ASM10 1743v1 | [Polyangium] brachysporum_::_strain=DS M 7029 | Complete Genome | soil |
| GCA_002116905.1_ASM21 1690v1 | Rhizobactergummiphilus_::_ strain=NS21 | Complete Genome | soil |
| GCA_002196515.1_ASM21 9651v1 | Vibrio gazogenes_::_strain=ATCC 43942 | Complete Genome | Estuarywater |
| GCA_000952685.1_ASM95 268v1 | Pseudomonas stutzeri :: strain=NT0128 | Draft Genome (Scaffold) | Wheatroot |
| GCA 900115905.1 IMG-taxon_2663762775_annotate d_assembly | Pseudomonas formosensis_::_strain=JCM 18415 | Draft Genome (Scaffold) | Compost |
| ..... | ..... | ..... | ..... |

**Database of protein domains (PDM) from the pfamDB:** The protein domains that are associated with genes constituent in the subpathways/pathways listed in PPOM for each pollutant are added in this matrix. PfamDB includes a large database providing information regarding different protein domains or function annotations of various proteins. PDM is made from searching for these domains listed in PfamDB across the desired genes. Table 4 shows a prototype of the PDM and Table 5 shows a sample PDM for pollutant PET.

**TABLE 4: A prototype of the PDM**

| **Pollutants** | **Pathways** | **Pathway description** | **Domains** |
|---|---|---|---|
| **Pollutant 1** | Sub-pathway 1 | pollutant 1 -> B | Domain 2, Domain 4 |
| | Sub-pathway 2 | B->C | Domain 1 |
| | Sub-pathway 3 | C->D | Domain5 |
| **Pollutant 2** | Sub-pathway 1 | pollutant 2 ->Y | Domain 3 |
| | Sub-pathway 2 | Y->Z | Domain 4, Domain 6 |
| **Pollutant 3** | Sub-pathway 1 | K->L | Domain 8 |
| | Sub-pathway 2 | Pollutant 3 ->M | Domain 7, Domain 9 |
| | Sub-pathway 3 | M->N | Domain 10 |
| | Sub-pathway 4 | N->O | Domain 11 |
| .... | ... | ... | ... |

**TABLE 5: A sample PDM for pollutant PET**

| **Pollutants** | **Pathways** | **Pathway description** | **Domains** |
|---|---|---|---|
| **PET (polyterephthalic acid)** | Sub-pathway 1 | PET -> TPA | DLH, AXE1 and Hydrolase_4-Serine aminopeptidase |
| | Sub-pathway 2 | TPA -> PCA | PdxA, rieske, ring hydroxyl, SnoaL, NAD binding, FAD binding, Fer2 |
| ...... | ..... | .... | ..... |

**Genome map (GM):** It represents the genome map providing information of list of genes according to the order of genomic locations for each bacterial genome as well as protein domain composition or functional information of each gene. An example of GM is shown in Table 6

**TABLE 6: A sample genome map**

| **Sr. No.** | **Genome ID** | **Organism name** | **Gene ID** | **Domains** | **Gene location** |
|---|---|---|---|---|---|
| 1 | GCA_00018638 5.1_ASM18638 v1 | Deinococcusmaricopensis DSM 21211 :: strain=DSM 21211 | Gene 1 | Domain1,2 | 12--112 |
| | | | Gene 2 | Domain 3,5 | 114--259 |
| | | | Gene 3 | Domain 1,5 | 312-555 |
| | | | ...... | | ...... |
| 2 | GCA_00211690 5.1_ASM21169 0v1 | Rhizobactergummiphilus _::_strain=NS21 | Gene 1 | Domain 6,7 | 150--306 |
| | | | Gene 2 | Domain 8 | 459--673 |
| | | | Gene 3 | Domains 8,9 | 682-1245 |
| | | | ...... | | ...... |
| ..... | ..... | ..... | ...... | | ...... |

**Genome pathway master map (GPM):** It provides with genome names and corresponding pathway/ sub-pathways information for each of the one or plurality of pollutants, wherein the GPM map having values 0 or 1 based on a first predefined criteria, where the criteria is to search for pathway specific protein domains within a window of 10 neighbouring genes and assign value of 1 if domains above a threshold value are present or 0 if domains are absent, for all the sub-pathways in a genome. Table 7 shows a prototype of GPM. Table 8 shows an example of GPM for PET pollutant.

**TABLE 7: A prototype table of GPM**

| **Genome ID/ organism name** | **Pollutant 1** | | | **Pollutant 2** | | **......** |
|---|---|---|---|---|---|---|
| | **Subpathway 1** | **Subpathway 2** | **Subpathway 3** | **Subpathway 1** | **Subpathway 2** | **...** |
| Genome 1/ Org1 | 1 | 0 | 0 | 0 | 0 | ... |
| Genome 2/ Org2 | 1 | 1 | 1 | 0 | 0 | ... |
| Genome 3/ Org3 | 1 | 0 | 0 | 1 | 1 | ... |
| Genome 4/ Org4 | 0 | 0 | 1 | 0 | 1 | ... |
| Genome 5/ Org5 | 0 | 0 | 0 | 0 | 0 | ... |
| ...... | ...... | ...... | ...... | ...... | ...... | ... |

**TABLE 8: An example of GPM for PET Pollutant**

| **Genome ID/ organism name** | **Pollutant - PET** | |
|---|---|---|
| | **Subpathway 1** | **Subpathway 2** |
| Deinococcusmaricopensis DSM 21211 :: strain=DSM 21211 | 1 | 0 |
| Rhizobactergummiphilus :: strain=NS21 | 1 | 0 |
| Vibrio gazogenes :: strain=ATCC 43942 | 1 | 0 |
| Acidovorax sp. P4 | 0 | 1 |
| Burkholderia sp. HB1 | 0 | 1 |
| Pseudomonas saudimassiliensis | 1 | 1 |
| Marinobacternanhaiticus D15-8W | 1 | 1 |
| .... | .... | .... |

**Genome pathway enzyme map (GPE):** The GPE provides the active site information for each enzyme corresponding to a step in the plurality of sub-pathways for the genome along with the information on the presence of signal peptides for each enzyme within GPE. Table 9 shows a prototype of GPE and Table 10 shows an example of GPE for PET pollutant

**TABLE 9: A prototype of GPE table**

| **Genome ID/ organism name** | **Gene ID** | **Pollutant 1** | | | ...... |
|---|---|---|---|---|---|
| | | **Sub- )athway 1-Enzyme 1** | | | ...... |
| | | **Active-site requirement1** | **Active-site requirement2** | **Active-site requirement3** | ...... |
| Genome 1/ Org1 | Gene 1 | 1 | 1 | 1 | ...... |
| Genome 2/ Org2 | Gene 1 | 0 | 1 | 1 | ...... |
| Genome 3/ Org3 | Gene 1 | 1 | 0 | 1 | ...... |
| Genome 3/ Org3 | Gene 2 | 1 | 1 | 0 | ...... |
| ...... | ...... | ...... | ...... | ...... | ...... |

**TABLE 10: An example of GPE for PET pollutant**

| **Genome ID/ organism name** | **Gene ID** | **Pollutant = PET** | | | ... ... |
|---|---|---|---|---|---|
| | | **Sub-pathway 1-PETase** | | | ... ... |
| | | **Active-site requirement 1 (DS1)** | **Active-site requirement 2 (DS2)** | **Active-site requireme nt 3 (Signal peptide)** | ... ... |
| Deinococcusmaricopensis DSM 21211 | ADV66860.1 | 1 | 0 | 0 | ... ... |
| RhizobactergummiphilusNS21 | ARN19002.1 | 1 | 1 | 1 | ... ... |
| Vibrio gazogenesATCC 43942 | ASA57064.1 | 1 | 1 | 0 | ... ... |
| Acidovorax sp. P4 | NA | 0 | 0 | 0 | ... ... |
| Burkholderia sp. HB 1 | NA | 0 | 0 | 0 | ... ... |
| Pseudomonas saudimassiliensis | CEF27108.1 | 1 | 1 | 1 | ... ... |
| Marinobactemanhaiticus D15-8W | ENO12784.1 | 1 | 1 | 1 | ... ... |
| ...... | ...... | ...... | ...... | ...... | ... ... |

Prototype of POEM matrix representing the sub-pathways of each degradation pathway for each pollutant and the information about organisms which contain these sub-pathways obtained using the methodology discussed in present disclosure. The matrix also shows the complete/partial pollutant degradation abilities of each organism as well as the environmental niche(s) these organisms have been isolated from. The information pertaining to each pollutant identified in the collected sample forms a part of POEM matrix.

**TABLE 11: A sample POEM matrix**

| **Pollutant Name: Pollutant 1** | | | | | |
|---|---|---|---|---|---|
| **Subpathway1** | **Environment** | **Degradation** | **Subpathway 2** | **Environment** | **Degradation** |
| Organism 1/Genome 1 | Envi 1 | Partial /Complete | Organism 5/Genome 5 | Envi 1 | Partial /Complete |
| Organism 2/Genome 2 | Envi 1 | Partial /Complete | Organism 6/Genome 6 | Envi 1 | Partial /Complete |
| Organism 3/Genome 3 | Envi 3 | Partial /Complete | Organism 7/Genome 7 | Envi 3 | Partial /Complete |
| Organism 4/Genome 4 | Envi 4 | Partial /Complete | Organism 8/Genome 8 | Envi 4 | Partial /Complete |

Probable consortia derived from the POEM matrix can be derived on the basis of presence of sub-pathways as well as the microbes possessing these sub-pathways should be capable of surviving in the same environmental niche(s) from where the sample is collected as shown in Table 12, Table 13 and Table 14. The first consortia for complete degradation of Pollutant 1 in Envi 1 can be obtained as under.

**TABLE 12: Consortia 1 for degradation of Pollutant 1 in Environment 1**

| **Sub-pathway 1** | **Environment** | **Degradation** | **Sub-pathway 2** | **Environment** | **Degradation** |
|---|---|---|---|---|---|
| Organism 1/Genome 1 | Envi 1 | Partial | Organism 5/Genome 5 | Envi 1 | Partial |
| Organism 1/Genome 1 | Envi 1 | Partial | Organism 6/Genome 6 | Envi 1 | Partial |

It should be noted that designing a consortia comprising of microbes possessing only sub-pathway 1 which will include Organism 1 and Organism 2 in this case would stop the degradation of the pollutant at the intermediate released/produced after the action of sub-pathway 1 within Organism 1 or Organism 2. The product intermediate so released can be rechanneled for a plurality of industrial applications. Therefore, a consortia comprising of organisms (Organism 1 and 2 in this case) which show presence of sub-pathway 1 can form the second consortia which releases an intermediate useful for industrial applications. Similarly consortia for degradation of pollutant 1 in other environments can be deciphered.

**TABLE 13: Consortia 2 for degradation of Pollutant 1 in Environment 3**

| | | | | | |
|---|---|---|---|---|---|
| Organism 3/Genome 3 | Envi 3 | Partial | Organism 7/Genome 7 | Envi 3 | Partial |

**TABLE 14: Consortia 3 for degradation of Pollutant 1 in Environment 4**

| | | | | | |
|---|---|---|---|---|---|
| Organism 4/Genome 4 | Envi 4 | Partial | Organism 8/Genome 8 | Envi 4 | Partial |

Further, a few examples of a prototype of POEM matrix for the partial and complete degraders of PET is provided in Table 15.

**TABLE 15: A prototype of POEM matrix with few examples for the partial and complete degraders of PET**

| **Sub-pathway 1 PET -> TPA** | **Environment** | **Degradation** | **Subpathway 2 TPA -> PCA** | **Environment** | **Degradation** |
|---|---|---|---|---|---|
| Vibrio gazogenesstrain(ATCC 43942) | Estuary-Water | Partial | Acidovorax sp. NA2 | Soil | Partial |
| Rhizobactergummiphilusstrain(NS21) | Soil | Partial | Acidovorax sp. NA3 | Soil | Partial |
| ... | .... | ... | ... | ... | ... |

Probable consortia for complete degradation of PET contributing sub-pathway 1 and 2 based on POEM matrix is shown in Table 16 and Table 17 for soil and sediments of marine environment respectively.

### Environmental preference: Soil

**TABLE 16: Probable consortia for complete degradation of PET contributing subpathway 1 and 2 based on POEM matrix for couple of examples in Soil**

| **Strains with sub-pathway 1 PET -> TPA** | **Strains with sub-pathway 2 TPA -> PCA** | **Environment** |
|---|---|---|
| | | |
| Rhizobactergummiphilusstrain(NS21) | Comamonastestosteroni CNB-2 | Soil |
| Rhizobactergummiphilusstrain(NS21) | Comamonastestosteroni TK102 | Soil |
| ... | ... | ... |

### Environmental reference: Sediments of marine environment

**TABLE 17: Probable consortia for complete degradation of PET contributing subpathway 1 and 2 based on POEM matrix for a couple of examples in sediments of marine environment**

| **Strains with sub-pathway 1 PET -> TPA** | **Strains with sub-pathway 2 TPA -> PCA** | **Environment** |
|---|---|---|
| Marinobactersegnicrescens strain(CGMCC 1.6489) | Cycloclasticus sp. P1 | Sediment |
| Marinobactersegnicrescens strain(CGMCC 1.6489) | Marinobacternanhaiticus D15-8W | Sediment |
| ... | ... | ... |

The consortia can be predicted using following criteria:
- Presence of sub-pathway 1 and 2 for complete degradation of PET to PCA which can be finally assimilated by multiple bacteria
- Strains comprising the consortium should be isolated from or capable of surviving in the environmental niche where sample is obtained from.

The methodology discussed in this embodiment was used to identify the degradation potential in bacteria for the major industrial pollutants polyethylene terephthalate (PET), polycyclic aromatic hydrocarbons (PAH) and polychlorinated biphenyls (PCB) as well as emerging pollutants such as carbon based nanomaterials (CBNMs). According to the methodology described in this embodiment, complete degradation of PET, CBNMs, PAHs and PCB involve a plurality of sub-pathways as described below.

For example, complete PET degradation involves PETase enzyme sub-pathway which converts PET to its constituent monomers such as TPA. The candidate bacterial family involved in sub-pathway for PETase and sub-pathway for TPA to PCA comprising one or more of bacterial family shown in Table 18. It should be appreciated that family in this case refers to the taxonomic classification according to Linnaean taxonomy and in this disclosure it refer to the strains of microbes within the given family which possess genes/proteins/enzymes for the corresponding sub-pathways. Any other bacterial family having the potential to degrade PET is included in the scope of this disclosure.

**TABLE 18: List of candidate bacterial family for PET degradation corresponding to various pathway**

| PET | | | |
|---|---|---|---|
| | | | |

| Sub-pathways corresponding to a pollutant pathway | PETase | Terepthalic Acid to Protocatechuic acid | Protocatechuic Acid to AcetylCoA |
|---|---|---|---|
| Candidate bacterial families having the gene context based functional potential to degrade pollutant pathways | Polyangiaceae | Comamonadaceae | Actinosynnemataceae |
| | Burkholderiaceae | Bacillaceae | Caulobacteraceae |
| | Burkholderiales_incertae_s edis | Bradyrhizobiaceae | Oxalobacteraceae |
| | Alteromonadaceae | Burkholderiaceae | Streptomycetaceae |
| | Oceanospirillaceae | Piscirickettsiaceae | Micrococcaceae |
| | Pseudomonadaceae | Sphingomonadaceae | Rhizobiaceae |
| | Vibrionaceae | Hyphomicrobiaceae | Myxococcaceae |
| | | Pseudomonadaceae | Nocardiaceae |
| | | Pseudonocardiaceae | Brucellaceae |
| | | Oxalobacteraceae | Nocardiopsaceae |
| | | Rhizobiaceae | Oceanospirillaceae |
| | | Nocardiaceae | Planococcaceae |
| | | Rhodocyclaceae | Pseudonocardiaceae |
| | | Streptomycetaceae | Actinopolysporaceae |
| | | | Streptosporangiaceae |
| | | | Xanthomonadaceae |
| | | | Hyphomicrobiaceae |
| | | | Rhodobacteraceae |
| | | | Mycobacteriaceae |
| | | | Microbacteriaceae |
| | | | Alcaligenaceae |
| | | | Geodermatophilaceae |
| | | | Burkholderiaceae |
| | | | Enterobacteriaceae |
| | | | Halomonadaceae |
| | | | Moraxellaceae |
| | | | Dietziaceae |
| | | | Phyllobacteriaceae |
| | | | Spliingomonadaceae |
| | | | Rhodospirillaceae |
| | | | Micromonosporaceae |
| | | | Comamonadaceae |
| | | | Pseudomonadaceae |
| | | | Aeromonadaceae |
| | | | Alteromonadaceae |
| | | | Aurantimonadaceae |
| | | | Cytophagaceae |
| | | | Neisseriaceae |
| | | | Deinococcaceae |
| | | | Nocardioidaceae |
| | | | Vibrionaceae |
| | | | Kiloniellaceae |
| | | | Gordoniaceae |
| | | | Listeriaceae |
| | | | Bacillaceae |
| | | | Xanthobacteraceae |
| | | | Rubrobacteraceae |
| | | | Tsukamurellaceae |
| | | | Bradyrhizobiaceae |
| | | | Saprospiraceae |
| | | | Sphingobacteriaceae |
| | | | Thermaceae |
| | | | Clostridiaceae |
| | | | Flavobacteriaceae |
| | | | Brevibacteriaceae |
| | | | Corynebacteriaceae |
| | | | Beijerinckiaceae |
| | | | Methylobacteriaceae |
| | | | Cystobacteraceae |
| | | | Granulosicoccaceae |
| | | | Glycomycetaceae |
| | | | Bacillaceae 1 |
| | | | Catenulisporaceae |
| | | | Sphaerobacteraceae |
| | | | unclassified Betaproteobacteria |
| | | | unclassified Burkholderiales |
| | | | unclassified Flavobacteriales |
| | | | Yersiniaceae |
| | | | Vicinamibacteraceae |

According to an embodiment of the disclosure, the system 100 is also configured to identify the key enzyme for the degradation of CBNMs. A key peroxidase enzyme is identified for the initial degradation of CBNMs wherein the presence of the enzyme is essential for CBNM degradation to occur. The enzymatic degradation of CBNM by the key peroxidase forms the initial step of the reaction and the intermediates formed are degraded in the subsequent steps discussed further as below:

Initially, the enzyme capable of degradation of CBNM is identified by performing literature mining techniques. A query string (Qᵢₙ) is generated for the isolated pollutant (Pi). Here the pollutant is any class of CBNM such as SWCNTs, MWCNTs, GO, RGO etc. The Query string (Qᵢₙ) is used as input to mine against curated literature search engines like PubMed and pathway databases such as KEGG Pathways / EAWAG (BBD/PPS)/ MetaCyc. The result set obtained from literature search engine contains a list of abstracts Aₒᵤₜ as output along with enzyme used for degradation of CBNMs (Eₒᵤₜ), wherein the degradation of CBNM is experimentally characterized.

In the next step, the key bacterial enzyme (E_{bac}) capable of degradation of CBNM is identified as follows. A list of all potential bacterial enzyme candidates for degradation of CBNMs is created (Eₗᵢₛₜ) across which the enzyme output from previous step (Eₒᵤₜ) is compared. These bacterial enzyme candidates are identified such that they possess protein domain constitution similar to Eₒᵤₜ enzyme. The factors used for comparison between Eₒᵤₜ and these candidate enzymes include protein and nucleotide sequence level similarity, protein structure level comparison and similarity in residues forming the active site. Scores are assigned for each member of Eₗᵢₛₜ compared against the enzyme capable of degrading CBNM Eₒᵤₜ. The enzyme with maximum similarity to Eₒᵤₜ is picked and is considered as the potential bacterial enzyme candidate (E_{bac}) capable of degradation of CBNMs.

Further, in order to degrade large molecular weight CBNMs the key bacterial enzyme (E_{bac}) in multiple bacterial species needs to be secreted out of the bacteria. The bacterial enzyme (E_{bac}) capable of degradation of CBNM is checked for its secretion capability and presence in extracellular region of bacteria. In an embodiment, the presence of secretion capabilities in E_{bac} is done through two methods involving analysis of presence of an N-terminal signal peptide as well as the analysis for leaderless secretion capabilities based on the amino acid constitution of the enzyme. For each genome Ge of the bacterial species S, containing the enzyme (E_{bac}), the secretion capacity of E_{bac} is analyzed and a score is obtained for each secretion method tested (D-score for presence of N-terminal signal peptide and SP-score for leaderless secretion). Based on the D-score and SP-score of E_{bac} the secretion potential of E_{bac} is determined. Only those bacterial species S for which the secretion potential of E_{bac} is higher than threshold score SOₜₕᵣₑ are considered as potential CBNM degraders. Such bacterial species with secretion score higher than Soₜₕᵣₑ are referred to as S_{NM}. In one embodiment, the threshold score (Soₜₕᵣₑ) of 0.79 is considered, but it can vary depending on methodology utilized and the enzyme system analyzed. Any other method of analyzing secretion capabilities of an enzyme is within scope of this disclosure.

According to an embodiment of this disclosure, the candidate bacterial enzyme capable of degradation of CBNMs (E_{bac}) was identified to be secretory bi-functional catalase-peroxidase enzyme and the candidate bacterial family identified to contain the secretory bi-functional catalase-peroxidase (S_{NM}) are identified. The candidate bacterial family containing secretory catalase-peroxidase enzyme and are involved in degradation of CBNM are listed in Table 19. It should be appreciated that family in this case refers to the taxonomic classification according to Linnaean taxonomy and in this disclosure it refer to the strains of microbes within the given family which possess genes/proteins/enzymes for the corresponding sub-pathways. Any other bacterial family capable of degradation of CBNMs is within the scope of this disclosure.

**TABLE 19 shows detailed candidate bacterial family for CBNM degradation**

| | | | |
|---|---|---|---|
| Acaryochloridaceae | Ectothiorhodospiraceae | Bradyrhizobiaceae | Methylophilaceae |
| Acetobacteraceae | Ferrimonadaceae | Burkholderiaceae | unclassified Alphaproteobacteria |
| Acidobacteriaceae | Flammeovirgaceae | Burkholderiaceae | Sphingobacteriaceae |
| Comamonadaceae | Cryomorphaceae | unclassified Burkholderiales | Nostocaceae |
| Moraxellaceae | Francisellaceae | Caulobacteraceae | Oleiphilaceae |
| Flavobacteriaceae | Frankiaceae | Caulobacteraceae | Pelobacteraceae |
| Aeromonadaceae | Gallionellaceae | Enterobacteriaceae | Phycisphaeraceae |
| Rhizobiaceae | Gemmatimonadaceae | Chitinophagaceae | Pirellulaceae |
| Cyclobacteriaceae | Geobacteraceae | Halomonadaceae | Planococcaceae |
| Marinilabiaceae | Gloeobacteraceae | Xenococcaceae | Chlorobiaceae |
| Erythrobacteraceae | Vibrionaceae | [Weeksellaceae] | Pseudoalteromonadaceae |
| Alteromonadaceae | Hahellaceae | Oxalobacteraceae | unclassified Rhizobiales |
| Phyllobacteriaceae | Saprospiraceae | Colwelliaceae | unclassified Rhizobiales |
| Rhodobacteraceae | Halothiobacillaceae | Puniceicoccaceae | Psychromonadaceae |
| Campylobacteraceae | Hyphomonadaceae | Gomontiellaceae | Nocardiaceae |
| Rhodocyclaceae | Hyphomicrobiaceae | Cyanobacteriaceae | Rivulariaceae |
| Xanthobacteraceae | Idiomarinaceae | Desulfobacteraceae | Salinivirgaceae |
| Pseudomonadaceae | Ignavibacteriaceae | Desulfobulbaceae | Shewanellaceae |
| Bacillaceae | Neisseriaceae | Desulfomicrobiaceae | Synechococcaceae |
| Oceanospirillaceae | Legionellaceae | Syntrophaceae | Gomphosphaeriaceae |
| Bacteroidaceae | Leptospiraceae | Peptococcaceae | Kiloniellaceae |
| Thiotrichaceae | Vicinamibacteraceae | Desulfovibrionaceae | Chromatiaceae |
| Beijerinckiaceae | Rhodospirillaceae | Chrysiogenaceae | unclassified |
| Bernardetiaceae | Methylobacteriaceae | Desulfuromonadaceae | Verrucomicrobiaceae |
| Sphingomonadaceae | Methylococcaceae | Cytophagaceae | Wenzhouxiangellaceae |
| Alcaligenaceae | Piscirickettsiaceae | Xanthomonadaceae | Woeseiaceae |

As a result of the action of catalase-peroxidase (S_{NM}), intermediates so formed during CBNM degradation show structural similarity to PARs, PCBs and SAHs which are aromatic compounds. Due to this reason, in the next step of CBNM degradation, the presence of aromatic degradation in bacterial species S_{NM} is analyzed. For each bacterial species S_{NM} that contains the key bacterial enzyme E_{bac} capable of degradation of CBNMs, the presence of aromatic hydrocarbon (such as PAHs and bi-phenyls) degradation ability is identified as discussed in further details below.

According to an embodiment of the disclosure, the PAHs included in this study include low molecular weight PAHs such as A) Naphthalene, B) Anthracene and C) Phenanthrene. Degradation pathways for each PAH pollutant is divided into sub-pathways. PAH degradation involves Naphthalene to Salicylate sub-pathway, Anthracene to Dihydroxynaphthalene sub-pathway, Catechol to AcetylCoA sub-pathway, Phenanthrene to Phthalate sub-pathway, Phthalatetodihydroxybenzoate sub-pathway and Phenanthrene to naphthalenediol sub-pathway. The candidate bacteria family involved in sub-pathway for Naphthalene to Salicylate comprising one or more the bacterial family is given in detail in Table 20A.
The candidate bacteria family involved in sub-pathway for Anthracene to Dihydroxynaphthalene is described in detail in Table 20A. It should be appreciated that family in this case refers to the taxonomic classification according to Linnaean taxonomy and in this disclosure it refer to the strains of microbes within the given family which possess genes/proteins/enzymes for the corresponding sub-pathways. The candidate bacteria family involved in sub-pathway for Catechol to Acetyl-CoA is described in detail in Table 20A.
The candidate bacteria family involved in sub-pathway for Phenanthrene to Phthalate is described in detail in Table 20B.
The candidate bacteria family involved in sub-pathway for Phthalate to dihydroxybenzoate is described in detail in Table 20B.
The candidate bacteria family involved in sub-pathway for Phenanthrene to naphthalenediol is described in detail in Table 20B.

TABLE 20A and TABLE 20B shows detailed candidate bacterial family for PAH degradation.

**TABLE 20A: List of candidate bacterial family for PAH degradation corresponding to various pathways**

| | **PAH (Naphthalene)** | **PAH (Anthracene)** | **PAH (Naphthalene/Anthracene)** |
|---|---|---|---|
| **Sub-pathways corresponding to a pollutant pathway** | **Naphthalene to Salicylate** | **Anthracene to Dihydroxynaphthalene** | **Catechol to AcetylCoA** |
| **Candidate bacterial families having the gene context based functional potential to degrade pollutant pathways** | Comamonadaceae | Comamonadaceae | Comamonadaceae |
| | Rhizobiaceae | Alcaligenaceae | Moraxellaceae |
| | Alteromonadaceae | Actinosvnnemataceae | Alcaligenaceae |
| | Bacillaceae | Bacillaceae | Alcanivoracaceae |
| | Alcaligenaceae | Geodermatophilaceae | Alicvclobacillaceae |
| | Bradyrhizobiaceae | Sphingomonadaceae | Ectothiorhodospiraceae |
| | Burkholderiaceae | Bradyrhizobiaceae | Actinosvnnemataceae |
| | Rhodobacteraceae | Burkholderiaceae | Phyllobacteriaceae |
| | Erythrobacteraceae | Caulobacteraceae | Neisseriaceae |
| | Piscirickettsiaceae | Rhodobacteraceae | Rhodocyclaceae |
| | Gordoniaceae | Frankiaceae | Pseudomonadaceae |
| | Oceanospirillaceae | Alteromonadaceae | Bacillaceae |
| | Aurantimonadaceae | Phyllobacteriaceae | Thiotrichaceae |
| | Oxalobacteraceae | Mycobacteriaceae | Bradyrhizobiaceae |
| | Phyllobacteriaceae | Hyphomicrobiaceae | Burkholderiaceae |
| | Mycobacteriaceae | Erythrobacteraceae | Clostridiaceae |
| | Sphincomonadaceae | Pseudomonadaceae | Rhodobacteraceae |
| | Hyphomicrobiaceae | Rhizobiaceae | Corynebacteriaceae |
| | Neisseriaceae | Nocardiaceae | Oxalobacteraceae |
| | Pseudomonadaceae | Streptomycetaceae | Piscirickettsiaceae |
| | unclassified Rhizohiales | Gammaproteobacteria_ incertae sedis | Frankiaceae |
| | Nocardiaceae | | Gordoniaceae |
| | Rhodocyclaceae | | Intrasporangiaceae |
| | Streptomycetaceae | | Enterobacteriaceae |
| | | | Planococcaceae |
| | | | Alteromonadaceae |
| | | | Aurantimonadaceae |
| | | | Mvcobacteriaceae |
| | | | Nakamurellaceae |
| | | | Nocardiaceae |
| | | | Nocardioidaceae |
| | | | Sphingomonadaceae |
| | | | Micrococcaceae |
| | | | Rhizobiaceae |
| | | | Streptomycetaceae |
| | | | Sulfobacillaceae |
| | | | Thermomonosporaceae |
| | | | |

**TABLE 20B: List of candidate bacterial family for PAH degradation corresponding to various pathways**

| **PAH (Phenanthrene)** | | |
|---|---|---|
| **Phenanthrene to Phthalate** | **Phthalate to dihydroxybenzoate** | **Phenanthrene to naphthalenediol** |
| Comamonadaceae | Acetobacteraceae | Comamonadaceae |
| Alteromonadaceae | Comamonadaceae | Bacillaceae |
| Phyllobacteriaceae | Alcaligenaceae | Bradyrhizobiaceae |
| Bacillaceae | Bacillaceae | Burkholderiaceae |
| Bradyrhizobiaceae | Bradyrhizobiaceae | Caulobacteraceae |
| Burkholderiaceae | Brucellaceae | Oxalobacteraceae |
| Erythrobacteraceae | Burkholderiaceae | Rhodocyclaceae |
| Oxalobacteraceae | Halomonadaceae | Frankiaceae |
| Mycobacteriaceae | Colwelliaceae | Halomonadaceae |
| Sphingomonadaceae | Corynebacteriaceae | Immundisolibacteraceae |
| Hyphomicrobiaceae | Frankiaceae | Rhodobacteraceae |
| Micrococcaceae | Gordoniaceae | Alteromonadaceae |
| Pseudomonadaceae | Phyllobacteriaceae | Oceanospirillaceae |
| Rhizobiaceae | Rhodobiaceae | Phyllobacteriaceae |
| Nocardiaceae | Mycobacteriaceae | Mycobacteriaceae |
| Streptomycetaceae | Nocardioidaceae | Nocardiaceae |
| | Nostocaceae | Sphingomonadaceae |
| | Sphingomonadaceae | Hyphomicrobiaceae |
| | Rhodobacteraceae | Enterobacteriaceae |
| | Oxalobacteraceae | Erythrobacteraceae |
| | Pseudonocardiaceae | Pseudonocardiaceae |
| | Pseudoalteromonadaceae | Micrococcaceae |
| | Pseudomonadaceae | Pseudomonadaceae |
| | Rhizobiaceae | Rhizobiaceae |
| | Nocardiaceae | Streptomycetaceae |
| | Alteromonadaceae | |
| | Streptomycetaceae | |
| | Gomphosphaeriaceae | |
| | Rhodospirillaceae | |
| | Enterobacteriaceae | |
| | Gammaproteobacteria_incertae_sedis | |

According to an embodiment of the disclosure, PCB degradation involves PCB to Biphenyl sub-pathway, Biphenyl to Acetyl-CoA/Pyruvatesub-pathway, Biphenyl to 2-hydroxypenta-2,4-dienoatesub-pathway, 2-hydroxypenta-2,4-dienoate to Acetyl-CoA/pyruvatesub-pathway, Benzoate to Acetyl-CoA via catecholsub-pathway and Benzoate to Acetyl-CoA via benzoyl-CoAsub-pathway. The candidate bacteria family involved in sub-pathway for PCB to Biphenyl is described in detail in Table 21A. It should be appreciated that family in this case refers to the taxonomic classification according to Linnaean taxonomy and in this disclosure it refer to the strains of microbes within the given family which possess genes/proteins/enzymes for the corresponding sub-pathways. Any other bacterial family capable of degrading PCB to Biphenyl is within the scope of this disclosure. The candidate bacteria family involved in sub-pathway for Biphenyl to Acetyl-CoA/Pyruvatesub-pathway is described in detail in Table 21A. Any other bacterial family capable of degrading PCB to Biphenyl to Acetyl-CoA/Pyruvate is within the scope of this disclosure.
The candidate bacteria family involved in sub-pathway for Biphenyl to 2-hydroxypenta-2, 4-dienoate is described in detail in Table 21A. Any other bacterial family capable of degrading Biphenyl to 2-hydroxypenta-2, 4-dienoate is within the scope of this disclosure.
The candidate bacteria family involved in sub-pathway for 2-hydroxypenta-2,4-dienoate to Acetyl-CoA/pyruvate is described in detail in Table 21B. Any other bacterial family capable of degrading 2-hydroxypenta-2, 4-dienoate to Acetyl-CoA/pyruvate is within the scope of this disclosure.
The candidate bacteria family involved in sub-pathway for Benzoate to Acetyl-CoA via catechol is described in detail in Table 21B. Any other bacterial family capable of degrading Benzoate to Acetyl-CoA via catechol is within the scope of this disclosure.
The candidate bacteria family involved in sub-pathway for Benzoate to Acetyl-CoA via benzoyl-CoA is described in detail in Table 21B. Any other bacterial family capable of degrading Benzoate to Acetyl-CoA via benzoyl-CoA is within the scope of this disclosure.

TABLE 21A and TABLE 21B shows detailed candidate bacterial family for PCB degradation.

**TABLE 21A: List of candidate bacterial family for PCB degradation corresponding to various pathways**

| **Sub-pathways corresponding to a pollutant pathway** | **PCB to Biphenyl** | **Biphenyl to Acetyl-CoA/Pyruvate** | **Biphenyl to 2-hydroxypenta-2,4-dienoate** |
|---|---|---|---|
| **Candidate bacterial families having the gene context based functional potential to degrade pollutant pathways** | Dehalococcoidaceae | Comamonadaceae | Comamonadaceae |
| | Peptococcaceae | Alcaligenaceae | Alcaligenaceae |
| | Campylobacteraceae | Alcanivoracaceae | Rhodocvclaceae |
| | | Rhodocyclaceae | Bacillaceae |
| | | Bacillaceae | Bradyrhizobiaceae |
| | | Burkholderiaceae | Burkholderiaceae |
| | | Conexibacteraceae | Rhodobacteraceae |
| | | Corvnebacteriaceae | Corvnebacteriaceae |
| | | Erythrobacteraceae | Immnndi solibacteraceae |
| | | Frankiaceae | Beijerinckiaceae |
| | | Aurantimonadaceae | Mycobacteriaceae |
| | | Beijerinckiaceae | Nocardioidaceae |
| | | Mycobacteriaceae | Sphingomonadaceae |
| | | Sphingomonadaceae | Pseudomonadaceae |
| | | Paenibacillaceae | Rhizobiaceae |
| | | Hyphomicrobiaceae | Nocardiaceae |
| | | Pseudoalteromonadaceae | Streptomycetaceae |
| | | Pseudomonadaceae | |
| | | Pseudonocardiaceae | |
| | | Xanthomonadaceae | |
| | | Rhizobiaceae | |
| | | Nocardiaceae | |
| | | Alteromonadaceae | |
| | | Planococcaceae | |
| | | Spongiibacteraceae | |

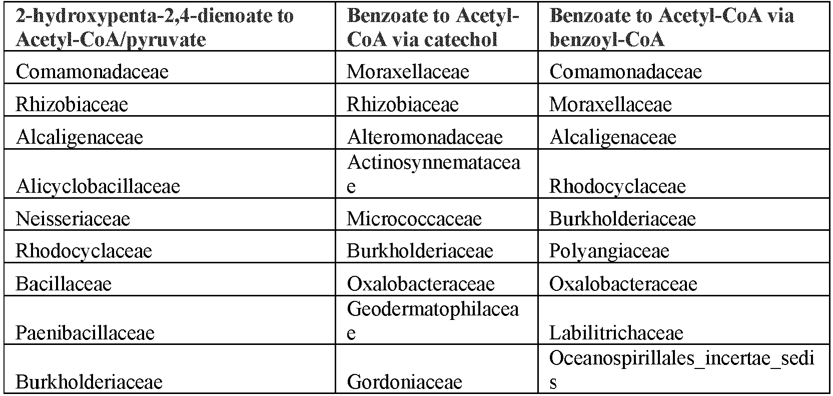

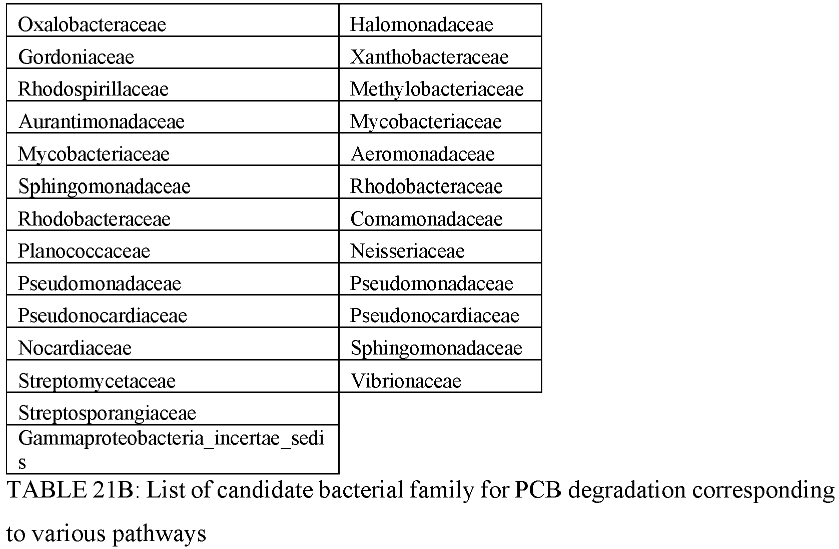

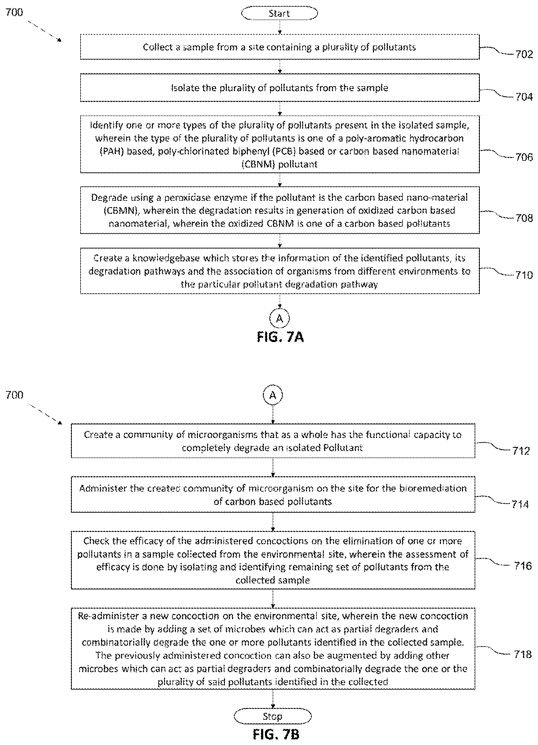

In operation, a flowchart 700 illustrating the steps involved for bioremediation of carbon based pollutants is shown in Fig. 7A-7B. Initially at step 702, the sample is collected from the site containing the plurality of pollutants. At step 704, isolating the plurality of pollutants from the sample. At step 706, one or more types of the plurality of pollutants present in the isolated sample are identified, wherein the type of the plurality of pollutants can be but are not limited to carbon based pollutants such as polyaromatic hydrocarbon (PAH) based, polychlorinated biphenyl (PCB) based, single aromatic hydrocarbon (SAH) based or carbon based nanomaterial (CBNM) pollutant.

In the next step 708, if the identified pollutant is the carbon based nano-material (CBNM) then it is degraded using the peroxidase enzyme, wherein the degradation results in generation of oxidized carbon based nanomaterial, wherein the oxidized CBNM is one of a carbon based pollutants which may lead to generation of intermediates which include PAH, PCB, SAH etc..

In the next step 710, the knowledgebase is created which stores the information of the identified pollutants, its degradation pathways and the association of organisms from different environments to the particular pollutant degradation pathway. The knowledgebase also contains peroxidase for CBNM degradation along with the organisms from different environments possessing this peroxidase enzyme. Further at step 712, a community of microorganisms is created that as a whole has the functional capacity to completely degrade an isolated Pollutant. At step 714, the created community of microorganism is administered on the site for the bioremediation of carbon based pollutants. At step 716, the efficacy of the administered concoctions on the elimination of one or more pollutants in a sample collected from the environmental site is checked and the assessment of efficacy is done by isolating and identifying remaining set of pollutants from the collected sample. And finally at step 718, the new concoction is re-administered at the environmental site by adding a set of microbes which can act as partial degraders and combinatorially degrade the one or more pollutants identified in the collected sample.

According to an embodiment of the disclosure, the system 100 can also be explained with the help of following CBNM degradation example. Certain bacterial family have been shown to degrade CBNM. The present analysis indicates that the hypothesis that catalase-peroxidase is the initial enzyme that degrades CBNM holds true. The study as well as the corresponding analysis have been described in detail below.

**Overview of the Study:** As discussed the initial steps of CBNM degradation involves the presence of bi-functional catalase-peroxidase (katG) enzyme that is secreted out of the bacterial cell. The redox reaction of CBNM catalysed by this enzyme produces aromatic intermediates which include, but is not limited to, various PAH and PCB compounds such as naphthalene, acenaphthene, bi-phenyl, as well as many single ringed aromatic compounds such as, phthalic acid, salicylic acid, benzoic acid, etc. These aromatic intermediates are then further degraded by the essential enzymes for aromatic hydrocarbon degradation bacteria.

**CBNM degrading capability based on Presence of Eₒᵤₜ: As** discussed in the methodology, enzyme Eₒᵤₜ capable of degrading CBNMs was identified as bi-functional catalase-peroxidase (katG). Identification of the bacterial species E_{bac} having Eₒᵤₜ (katG) revealed that many bacterial genera does indeed possess the enzyme katG. The sequence of the protein was taken to analyze the secretion capabilities of the particular enzyme. Accordingly the D-score (for presence of N-terminal signal peptide) and SP-score (for leaderless secretion) were determined. While N-terminal signal peptide was not detected using SignalP software, the possibility of leaderless secretion of these katG enzyme was detected using SecretomeP software and enzymes with D-score and SP-score beyond the threshold score SOₜₕᵣₑ of 0.5 were considered. Thus, we can say that many bacterial genera such as *Pseudomonas sp., Labrys sp.* and *Stenotrophomonas sp.,* etc, indeed contain the necessary secretory bi-functional catalase-peroxidase enzyme to initiate the first step of CBNM degradation.

**Microbial Community Concoction:** A polluted site usually comprises of a concoction of contaminants and presence of the carbon based pollutants and compounds (e.g. CBNMs, PAHs, PCBs, etc.) at these sites is quite pervasive. Effective bio-remediation of a polluted sample thus would require a combination of multiple organisms, capable of degrading each pollutant type, that work towards total degradation of these pollutants. Bacteria are known to live in multispecies communities and exhibit extensive interactions within as well as between species and possess the remarkable ability to degrade a plethora of organic compounds by consuming it as its main source of energy and further assimilating them without releasing any harmful by-products. Thus, in order to bring about complete degradation of the graphene oxide, a microbial community concoction comprising of CBNM degrading bacterial genera as well as other microbes capable of higher aromatic degradation needs to be identified and administered to the polluted site.

According to an embodiment of the disclosure, the system 100 can also be explained with the help of following example for bacterium *Labrys* sp WJW. The bacterium *Labrys* sp WJW has been shown to degrade CBNM, especially graphene oxide (GO). The present analysis indicates that the hypothesis that catalase-peroxidase is the initial enzyme that degrades CBNM holds true. The study as well as the corresponding analysis have been described in detail below.

**Overview of the Study:** As discussed the initial steps of CBNM degradation involves the presence of bi-functional catalase-peroxidase (katG) enzyme in *Labrys* sp WJW that is secreted out of the bacterial cell. The redox reaction of CBNM catalysed by this enzyme produces aromatic intermediates which include, but is not limited to, various PAH and PCB compounds such as naphthalene, acenaphthene, bi-phenyl, as well as many single ringed aromatic compounds such as, phthalic acid, salicylic acid, benzoic acid, etc. These aromatic intermediates are then further degraded by the essential enzymes for aromatic hydrocarbon degradation in *Labrys* sp WJW bacteria.

Case Study of Degradation of GO by a novel bacterial species *Labrys sp.* WJW: In this study, a novel strain of bacteria *Labrys sp.* WJW isolated from soil was seen to utilize GO as the sole carbon source under laboratory conditions. Analysis of the degradation processes through mass spectroscopic methods indicate that many of the intermediates produced in the process are aromatic hydrocarbons. Further, a micro array analysis suggested that many of the aromatic degradation genes of *Labrys* sp WJW has been up-regulated during the process indicating that these intermediates are degraded by the *Labrys* sp WJW.

**CBNM degrading capability based on Presence of Eₒᵤₜ:** As discussed in the methodology, enzyme Eₒᵤₜ capable of degrading CBNMs was identified as bi-functional catalase-peroxidase (katG). Identification of the bacterial species E_{bac} having Eₒᵤₜ (katG) revealed that *Labrys sp* WJW does indeed possess the enzyme katG. The sequence of the protein was taken to analyze the secretion capabilities of the particular enzyme. Accordingly the D-score (for presence of N-terminal signal peptide) and SP-score (for leaderless secretion) were determined. While N-terminal signal peptide was not detected using SignalP software, the possibility of leaderless secretion of *Labrys sp* WJW katG enzyme was detected using SecretomeP software with a SP-score of 0.80 (on a scale of 0-1) which is well beyond the threshold score SOₜₕᵣₑ of 0.5. Thus, we can say that *Labrys sp* WJW does indeed contain the necessary secretory bi-functional catalase-peroxidase enzyme to initiate the first step of CBNM degradation.

**Aromatic hydrocarbon degradation capability:** Aromatic hydrocarbon degradation ability of *Labrys sp* WJW was assayed as discussed in the methodology. It was identified that *Labrys* sp WJW possessed the full gene cluster for only benzoate degradation and therefore may not be able to degrade the higher aromatics released as a part of the intermediate mixture.

**Microbial Community Concoction:** A polluted site usually comprises of a concoction of contaminants and presence of the carbon based pollutants and compounds (e.g. CBNMs, PARs, PCBs, etc.) at these sites is quite pervasive. Effective bio-remediation of a polluted sample thus would require a combination of multiple organisms, capable of degrading each pollutant type, that work towards total degradation of these pollutants. Bacteria are known to live in multispecies communities and exhibit extensive interactions within as well as between species and possess the remarkable ability to degrade a plethora of organic compounds by consuming it as its main source of energy and further assimilating them without releasing any harmful by-products. Thus, in order to bring about complete degradation of the graphene oxide, a microbial community concoction comprising of *Labrys* sp WJW as well as other microbes capable of higher aromatic degradation needs to be identified and administered to the polluted site.

The aromatic hydrocarbon degradation ability of various bacteria was analyzed to determine their ability to degrade intermediates formed during CBNM degradation which show structural similarity to PAH and PCB intermediate compounds. Also, PAH and PCB are by themselves potent contaminants that must be degraded into harmless by-products.

Existing literature suggests that Low Molecular Weight (LMW) PAHs, which include hydrocarbon compounds having less than 4 fused benzene rings, such as Naphthalene, Anthracene and Phenanthrene are biodegradable and usually undergo aerobic degradation. An exhaustive genomic analysis was done across bacterial genomes to ascertain the ability to degrade the above mentioned PAHs. Naphthalene being a PAH is usually favored to be degraded via aerobic degradation by bacteria by the action of oxygen-mediated metabolism, followed by dehydrogenases and the subsequent ring cleavage by the dioxygenases to form TCA cycle intermediates which can be easily assimilated by the organism.

The Naphthalene degradation pathway can be divided into two subpathways; namely conversion of Naphthalene to Salicylate (NSP1) and salicylate degradation via catechol (NSP2) where both the sub-pathways are governed by a Lys-R regulator. Analysis of the pathway via literature mining, manual curation and comparison with the model organism *Pseudomonas stutzeri* helped in identifying the gene clusters involved in the sub-pathways for Naphthalene to Salicylate and Salicyate to Acetyl-CoA conversion. Domain information corresponding to each gene is searched from the Pfam database. In the present methodology the presence of this cluster was identified across bacterial genomes using a Hidden Markov Model based approach using tool such as HMMER. A window of 20 genes up and downstream of the query gene was searched for the presence of the gene cluster. Bacterial genomes such as *Polaromonas naphthalenivorans CJ2, Novosphingobium aromaticivorans DSM 12444, Celeribacter indicus* etc. had both the gene cluster as well as the Lys-R regulator and they occurred within context within their genomes. These organisms were the potential Naphthalene degraders (PAH) which needed further active site pattern validation.

Using literature mining, the patterns specific to the active site of the enzyme naphthalene dioxygenase (NDO) which is involved in initial attack on the ring aromatic structure was identified. The conservation of active sites for enzymes involved was determined using Multiple Sequence Alignment (MSA). All the potential naphthalene degraders (e.g. *Polaromonas naphthalenivorans CJ2, , Celeribacter indicus* etc.) were further validated by searching for the presence of residues critical for enzymes involved in naphthalene degradation. It was observed that organisms such as *Polaromonas naphthalenivorans CJ2, Acidovorax sp. P4 etc.* had these important active site residues conserved (e.g. V-209, N-297, F-352). Thus bacterial organisms possessing the gene cluster along-with its regulator, and having the active site patterns specific to NDO, can be designated as True Naphthalene degraders.

A similar approach is used to handle PCBs as that in Naphthalene degradation as described above. Biphenyls and their lower chlorinated forms are produced under anaerobic conditions by reductive de-halogenation of higher chlorinated PCBs. involving *rdhABR* gene cluster. The domain of RdhB gene was used to identify dehalogenation potential across bacterial genomes. Using our methodology bacteria such as *Dehalococcoides mccartyi, Sulfurospirillum multivorans, Desulfitobacterium dehalogenans* were identified. The cluster for upper pathway degradation of Biphenyls was identified in organisms such as *Acidovorax sp. KKS102, Azoarcus sp. CIB, Celeribacter indicus, Comamonas testosteroni TK102, etc.* The intermediate formed at the end of upper pathway is further degraded within the same organism or transported out and degraded by another organism via lower pathway . The potential organisms containing the lower pathway as found in our analysis to list a few are *Acidovorax sp. JS42, Acidovorax sp. KKS102,, etc..* Many of these bacteria contain both pathways for complete degradation of PCBs.

Hence, a microbial community cocktail capable of degradation of CBNMs as well as other higher and lower aromatic compounds will bring about complete and effective degradation of carbon-based pollutants.

**Administration of microbial cocktail:** A culture of a microbial cocktail as mentioned above can be added to the given soil sample contaminated with CBNMs. In this process, the above mentioned microbial cocktail is added to the soil along with the necessary nutrients as prescribed (such a nutrient broth containing beef extract) to the soil sample. The sample is further aerated and well hydrated to ensure that the microbial cocktail reaches logarithmic growth phase to facilitate pollutant bio-remediation.

**Efficacy of the administered microbial cocktail:** The assessment of efficacy of the administered microbial cocktail is done by isolating and identifying remaining set of pollutants from the collected sample and re-administering a new concoction on the environmental site. The new concoction is made by adding a set of microbes which can act as partial degraders and combinatorially degrade the one or more pollutants identified in the collected sample.

The written description describes the subject matter herein to enable any person skilled in the art to make and use the embodiments. The scope of the subject matter embodiments is defined by the claims.

The embodiments of present disclosure herein address unresolved problem of degradation of pollutants, which cause severe effects on the environment. The embodiment, thus provides a method and system for complete bioremediation of one or more pollutants.

It is to be understood that the scope of the disclosure is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computerusable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that disclosed embodiments being indicated by the following claims.

## Claims

1. A method (200) for bioremediation of one or more pollutants, the method comprising:
collecting a sample from an environment site containing the one or more pollutants (202);
isolating and identifying the one or more pollutants present in the sample (204);
creating a knowledgebase, wherein the knowledgebase stores
information of the identified one or more pollutants,
information pertaining to complete degradation pathways and partial degradation pathways identified in microbes that are capable of completely degrading the one or more pollutants or partially degrading the one or more pollutants,
information about respective environmental niches in which the microbes thrive, and
a list of microbes from different environments possessing the particular complete/partial pollutant degradation pathway,
wherein the particular complete degradation pathway refers to a set of genes on a genome of a microbe and/or proteins encoded by the microbe wherein the set of genes and/ or encoded proteins are responsible for complete degradation of a pollutant either to compounds that are safe for the environment site or to compounds that can be assimilated by other microbe(s) residing within the environment,
wherein the partial degradation pathway in the microbe refers to a set of genes or encoded proteins that constitute one or more subpathways, wherein a sub-pathway is a subset of the complete degradation pathway encoded within genome of the microbe, and the sub-pathway degrades the pollutant to an intermediate compound which can be released out into the environment by the microbe and is subsequently taken up by another microbe within the environment, wherein the another microbe possesses another sub-pathway that metabolizes the released intermediate compound,
wherein the knowledgebase comprises a pollutant pathway organism matrix (PPOM), a genome pathway enzyme (GPE) map, a genome pathway master (GPM) map and a database of abundant environmental microbes (DEBG), wherein the GPE map comprises of:
microbial names listed in the DEBG, and
information about active site of each enzyme involved in a plurality of sub-pathways on each genome of the microbe, for each of the one or more pollutants identified in the collected sample (206),
wherein the step of creating the knowledgebase comprising:
employing literature mining techniques to identify degradation pathway(s) and corresponding genes/proteins in microbes, wherein the pathway(s) degrade the one or more isolated and identified pollutants, and wherein the literature mining also results in identification of a set of microbes in which the degradation pathway/ pathways are characterized, and wherein the literature mining results in obtaining information on the environmental niche in which the microbes reside and are isolated;
identifying a plurality of sub-pathways within the degradation pathway that completely or partial degrade the isolated one or more pollutants, wherein the genes and/or proteins or enzymes corresponding to each of the plurality of sub-pathways is encoded by genome of a single microbe or genomes of a plurality of microbes, and the product formed by each of the plurality of sub-pathways is released into the environment site, and wherein the product is metabolized by the single microbe or is taken up by other microbe(s) inhabiting the environment, and wherein the other plurality of microbes possess capability of metabolizing the product;
creating the PPOM using the information on identified degradation pathway for each one or more identified pollutants, the plurality of sub-pathways for the degradation pathway, the set of microbes in which the degradation pathway is characterized and the information based on literature mining and manual curation about the respective environmental niche/niches from which the set of microbes are isolated;
employing the literature mining techniques to create the DEBG, wherein the DEBG comprises of information pertaining to microbe(s) and the different environmental niches in which the microbe(s) thrive;
creating a pathway domain map (PDM) from a precreated protein family database (pfamDB), wherein the protein domains included in the PDM are those corresponding to genes/proteins constituting the plurality of sub-pathways that comprise each degradation pathway present in the created PPOM for the one or more pollutants;
creating a genome map (GM), wherein the genome map comprises information pertaining to all microbial genomes reported in National Centre of Biotechnology Information, NCBI, wherein the information also comprises of a listing of genes ordered as per their respective genomic locations in a microbe as well as the constituent protein domains encoded within these genes;
searching presence of protein domains included in PDM for each of the plurality of sub-pathways for all pathways listed in the PPOM on the genomes of microbes stored in the DEBG to determine occurrence of these sub-pathways on the genomes, wherein the search is performed using the genome map GM as a database, and wherein the subpathway from the PDM is considered to be present if a number of domains in the genome contributing to this sub-pathway as listed in the PDM occur within a window size of genes on the genome and cross a predefined threshold value;
creating the GPM with microbial names corresponding to the microbial genomes in the DEBG, and information about presence or absence of plurality of pathways and the plurality of subpathways on the genome, for each of the one or more pollutants identified in the collected sample, and wherein the GPM map has a value of 0 or 1 based on a first predefined criterion, and wherein the GPM provides the information about all sub-pathways for a given pollutant degradation pathway that are present within each of the microbial genomes listed in the GPM; and
creating the GPE map, wherein the GPE map has a value of 0 or 1 based on a second predefined criterion;
identifying a list of partial pollutant degraders and a list of complete pollutant degraders for each of the one or more pollutants identified in the sample by utilizing the information from the knowledgebase, wherein partial pollutant degraders refer to microbes that contribute one or more subpathways and the corresponding set of genes, encoded proteins or enzymes, that convert a pollutant to an intermediate compound, and wherein multiple partial degraders combinatorially contribute all sub-pathways for complete degradation of the pollutant identified in the collected sample, wherein complete pollutant degraders possess a combination of all subpathways and the corresponding set of genes, encoded proteins or enzymes within a single microbe for degradation of the pollutant identified in the collected sample (208);
creating a map of microbes using the information from the knowledgebase, wherein the map of microbes comprises information of one or more of the partial pollutant degraders and complete pollutant degraders, capable of degrading each pollutant within the one or more pollutants identified within the sample to a varying degrees of degradation, wherein the varying degrees of degradation for the pollutant refers to the degradation of a pollutant to different intermediate compounds or metabolites and wherein the intermediate compounds or metabolites are determined by final product(s) released by the degrader upon the action of genes or proteins or enzymes corresponding to the subpathway(s) present within the genome of the degrader for the degradation of the pollutant, and wherein the intermediate compounds can either be released into the environment and utilized by other microbes within the environment or can be assimilated within the same microbe which carries out this degradation (210);
designing a first microbial consortia using the created map of microbes comprising of microbes which together contribute sub-pathways required for complete degradation of the one or more pollutants identified in the sample and wherein the microbes can survive together in the same environmental niche from where the sample has been collected (212);
designing a second microbial consortia using the created map of microbes comprising of microbes which together contribute genes, proteins and enzymes for sub-pathways required for partial degradation of the one or more pollutants identified in the collected sample to desired intermediate product/products, wherein the microbes forming the second microbial consortia can survive together in the environmental niche from where the sample has been collected (214);
administering a concoction of at least one or both of the first microbial consortia and the second microbial consortia to the environmental site containing the one or more pollutants (216);
checking efficacy of the administered concoctions on the elimination of one or more pollutants in a sample collected from the environmental site, wherein the assessment of efficacy is done by isolating and identifying remaining set of pollutants from the collected sample (218); and
re-administering a new concoction on the environmental site, wherein the new concoction is made by adding a set of microbes which can act as partial degraders and combinatorially degrade the one or more pollutants identified in the collected sample (220).

2. The method according to claim 1, wherein the first predefined criteria is for each subpathway in a microbial genome:
a value of 0 is assigned if protein domains corresponding to a subpathway as recorded in 'PDM' either don't occur or do not reach a threshold value within a predefined window of genes, and
a value of 1 is assigned if sub-pathway protein domains as recorded in 'PDM' are present above the threshold within the predefined window of genes.

3. The method according to claim 2, wherein
the threshold value is decided based on literature mining and manual curation and corresponds to the threshold minimum number of domains as well as the domains whose presence is required in order to confirm existence of the sub-pathway within a microbial genome, wherein the threshold value is defined as a fraction of the required domains out of the total number of domains corresponding to this sub-pathway in the PDM, and
the predefined window of genes is defined using manual curation which states the distance in terms number of genes on the basis of genome location within which the domains can be considered constituting a subpathway, wherein the genes encoding protein domains forming a subpathway occur together on the microbial genome thereby located within a defined window size of genes on the genome.

4. The method according to claim 1, wherein the second predefined criterion for each enzyme corresponding to each step of a sub-pathway identified in the microbial genome is:
a value of 1 is assigned to the enzymes where an active site pattern for that enzyme is found, wherein the active site pattern for constituent enzymes of each candidate pathway is the pattern specific to the active site of the enzyme obtained by literature mining, wherein the active site pattern is identified by motifs which act as signature sequences which help in identifying whether the enzyme is functionally capable to bind to a substrate and are conserved across all enzymes having similar functionality, and wherein the motifs are identified by multiple sequence alignment, MSA, across all possible functionally similar homologs of the enzyme to identify conserved amino acid patterns across the enzymes which are validated in literature to assess functional importance, and
a value of 0 is assigned in case the active site pattern for the enzyme is not found.

5. The method according to claim 1, further comprising testing the presence of secretion capacity for each enzyme and updating value of 0 for absence or 1 for presence of the secretion capacity in the GPE map.

6. The method according to claim 1, wherein the desired intermediate product refers to a set of intermediate products derived during partial degradation of the one or more pollutants identified.

7. The method according to claim 1, wherein the knowledgebase stores information pertaining to one or more pollutants, wherein some of these pollutants include Polyethylene Terephthalate (PET), Styrene, Polyurethane, Polyaromatic hydrocarbons (PAH), different congeners of Polychlorobiphenyls (PCB) or carbon based nanomaterials (CBNMs).

8. The method according to claim 7, wherein complete CBNM degradation involves the presence of a bi-functional catalase-peroxidase (kat) enzyme and the sub-pathways for degradation of one or more of the intermediates formed after catalytic action of kat enzyme, wherein the intermediates comprises one or more of PAH, PCB and single aromatic hydrocarbon, SAH, degradation formed as a result of catalase-peroxidase's catalytic action on CBNM.

9. The method according to claim 7, wherein the complete PET degradation involves the sub-pathways for PETase followed by Terephthalic Acid, TPA, to Protocatechuic Acid, PCA, conversion and the candidate bacteria family involved in these sub-pathways for PET degradation comprising one or more of:
Polyangiaceae, Burkholderiaceae, Burkholderiales_incertae_sedis, Alteromonadaceae, Oceanospirillaceae, Pseudomonadaceae or Vibrionaceae,
Comamonadaceae, Bacillaceae, Bradyrhizobiaceae, Burkholderiaceae, Piscirickettsiaceae, Sphingomonadaceae, Hyphomicrobiaceae, Pseudomonadaceae, Pseudonocardiaceae, Oxalobacteraceae, Rhizobiaceae, Nocardiaceae, Rhodocyclaceae or Streptomycetaceae for Terepthalic Acid to Protocatechuic acid subpathway, and
Actinosynnemataceae, Caulobacteraceae, Oxalobacteraceae, Streptomycetaceae, Micrococcaceae, Rhizobiaceae, Myxococcaceae, Nocardiaceae, Brucellaceae, Nocardiopsaceae, Oceanospirillaceae, Planococcaceae, Pseudonocardiaceae, Actinopolysporaceae, Streptosporangiaceae, Xanthomonadaceae, Hyphomicrobiaceae, Rhodobacteraceae, Mycobacteriaceae, Microbacteriaceae, Alcaligenaceae, Geodermatophilaceae, Burkholderiaceae, Enterobacteriaceae, Halomonadaceae, Moraxellaceae, Dietziaceae, Phyllobacteriaceae, Sphingomonadaceae, Rhodospirillaceae, Micromonosporaceae, Comamonadaceae, Pseudomonadaceae, Aeromonadaceae, Alteromonadaceae, Aurantimonadaceae, Cytophagaceae, Neisseriaceae, Deinococcaceae, Nocardioidaceae, Vibrionaceae, Kiloniellaceae, Gordoniaceae, Listeriaceae, Bacillaceae, Xanthobacteraceae, Rubrobacteraceae, Tsukamurellaceae, Bradyrhizobiaceae, Saprospiraceae, Sphingobacteriaceae, Thermaceae, Clostridiaceae, Flavobacteriaceae, Brevibacteriaceae, Corynebacteriaceae, Beijerinckiaceae, Methylobacteriaceae, Cystobacteraceae, Granulosicoccaceae, Glycomycetaceae, Bacillaceae 1, Catenulisporaceae, Sphaerobacteraceae, unclassified Betaproteobacteria, unclassified Burkholderiales, unclassified Flavobacteriales, Yersiniaceae or Vicinamibacteraceae for Protocatechuic Acid to AcetylCoA sub-pathway.

10. The method according to claim 7, wherein the complete degradation of the PAHs further comprising:
degradation of Naphthalene, which comprises of two sub-pathways which degrades Naphthalene to Salicylate followed by Salicylate degradation via catechol to form Acetyl Co-A,
degradation of Anthracene, is divided into sub-pathways that convert Anthracene to Dihydroxynaphthalene sub-pathway followed by Salicyalate degradation via catechol metabolism pathway to form Acetyl Co-A, and
degradation of Phenanthrene, which involves Phenanthrene to Phthalate sub-pathway, Phthalate to dihydroxybenzoate sub-pathway and Phenanthrene to naphthalenediol sub-pathway and the candidate bacteria family involved in sub-pathways for the degradation of each type of the above mentioned PAH comprising one or more of:
Comamonadaceae, Rhizobiaceae, Alteromonadaceae, Bacillaceae, Alcaligenaceae, Bradyrhizobiaceae, Burkholderiaceae, Rhodobacteraceae, Erythrobacteraceae, Piscirickettsiaceae, Gordoniaceae, Oceanospirillaceae, Aurantimonadaceae, Oxalobacteraceae, Phyllobacteriaceae, Mycobacteriaceae, Sphingomonadaceae, Hyphomicrobiaceae, Neisseriaceae, Pseudomonadaceae, unclassified Rhizobiales, Nocardiaceae, Rhodocyclaceae, or Streptomycetaceae for Naphthalene to Salicylate subpathway,
Comamonadaceae, Alcaligenaceae, Actinosynnemataceae, Bacillaceae, Geodermatophilaceae, Sphingomonadaceae, Bradyrhizobiaceae, Burkholderiaceae, Caulobacteraceae, Rhodobacteraceae, Frankiaceae, Alteromonadaceae, Phyllobacteriaceae, Mycobacteriaceae, Hyphomicrobiaceae, Erythrobacteraceae, Pseudomonadaceae, Rhizobiaceae, Nocardiaceae, Streptomycetaceae, or Gammaproteobacteria_incertae_sedis for Anthracene to Dihydroxynaphthalene sub-pathway,
Comamonadaceae, Moraxellaceae, Alcaligenaceae, Alcanivoracaceae, Alicyclobacillaceae, Ectothiorhodospiraceae, Actinosynnemataceae, Phyllobacteriaceae, Neisseriaceae, Rhodocyclaceae, Pseudomonadaceae, Bacillaceae, Thiotrichaceae, Bradyrhizobiaceae, Burkholderiaceae, Clostridiaceae, Rhodobacteraceae, Corynebacteriaceae, Oxalobacteraceae, Piscirickettsiaceae, Frankiaceae, Gordoniaceae, Intrasporangiaceae, Enterobacteriaceae, Planococcaceae, Alteromonadaceae, Aurantimonadaceae, Mycobacteriaceae, Nakamurellaceae, Nocardiaceae, Nocardioidaceae, Sphingomonadaceae, Micrococcaceae, Rhizobiaceae, Streptomycetaceae, Sulfobacillaceae, orThermomonosporaceae for Catechol to AcetylCoA sub-pathway,
Comamonadaceae, Alteromonadaceae, Phyllobacteriaceae, Bacillaceae, Bradyrhizobiaceae, Burkholderiaceae, Erythrobacteraceae, Oxalobacteraceae, Mycobacteriaceae, Sphingomonadaceae, Hyphomicrobiaceae, Micrococcaceae, Pseudomonadaceae, Rhizobiaceae, Nocardiaceae, or Streptomycetaceae for Phenanthrene to Phthalate subpathway,
Acetobacteraceae, Comamonadaceae, Alcaligenaceae, Bacillaceae, Bradyrhizobiaceae, Brucellaceae, Burkholderiaceae, Halomonadaceae, Colwelliaceae, Corynebacteriaceae, Frankiaceae, Gordoniaceae, Phyllobacteriaceae, Rhodobiaceae, Mycobacteriaceae, Nocardioidaceae, Nostocaceae, Sphingomonadaceae, Rhodobacteraceae, Oxalobacteraceae, Pseudonocardiaceae, Pseudoalteromonadaceae, Pseudomonadaceae, Rhizobiaceae, Nocardiaceae, Alteromonadaceae, Streptomycetaceae, Gomphosphaeriaceae, Rhodospirillaceae, Enterobacteriaceae, or Gammaproteobacteria_incertae_sedis for Phthalate to dihydroxybenzoate sub-pathway, and
Comamonadaceae, Bacillaceae, Bradyrhizobiaceae, Burkholderiaceae, Caulobacteraceae, Oxalobacteraceae, Rhodocyclaceae, Frankiaceae, Halomonadaceae, Immundisolibacteraceae, Rhodobacteraceae, Alteromonadaceae, Oceanospirillaceae, Phyllobacteriaceae, Mycobacteriaceae, Nocardiaceae, Sphingomonadaceae, Hyphomicrobiaceae, Enterobacteriaceae, Erythrobacteraceae, Pseudonocardiaceae, Micrococcaceae, Pseudomonadaceae, Rhizobiaceae, or Streptomycetaceae for Phenanthrene to naphthalenediol sub-pathway.

11. The method according to claim 7, wherein the complete degradation of PCB involves the sub-pathways for reductive de-halogenation of higher chlorinated PCBs to biphenyls followed by the sub-pathway for the conversion of biphenyl to 2-hydroxypenta-2, 4-dienoate which is further degraded via lower pathway to form pyruvate and acetyl-CoA, along-with the sub-pathways for the intermediates formed, which is converted to Acetyl Co-A via Benzoyl Co-A/Catechol pathway and the candidate bacteria family involved in these sub-pathways for PCB degradation comprising one or more of:
Dehalococcoidaceae, Peptococcaceae, or Campylobacteraceae for PCB to Biphenyl sub-pathway,
Comamonadaceae, Alcaligenaceae, Alcanivoracaceae, Rhodocyclaceae, Bacillaceae, Burkholderiaceae, Conexibacteraceae, Corynebacteriaceae, Erythrobacteraceae, Frankiaceae, Aurantimonadaceae, Beijerinckiaceae, Mycobacteriaceae, Sphingomonadaceae, Paenibacillaceae, Hyphomicrobiaceae, Pseudoalteromonadaceae, Pseudomonadaceae, Pseudonocardiaceae, Xanthomonadaceae, Rhizobiaceae, Nocardiaceae, Alteromonadaceae, Planococcaceae, or Spongiibacteraceae for Biphenyl to Acetyl-CoA/Pyruvate sub-pathway,
Comamonadaceae, Alcaligenaceae, Rhodocyclaceae, Bacillaceae, Bradyrhizobiaceae, Burkholderiaceae, Rhodobacteraceae, Corynebacteriaceae, Immundisolibacteraceae, Beijerinckiaceae, Mycobacteriaceae, Nocardioidaceae, Sphingomonadaceae, Pseudomonadaceae, Rhizobiaceae, Nocardiaceae, or Streptomycetaceae for Biphenyl to 2-hydroxypenta-2,4-dienoate sub-pathway,
Comamonadaceae, Rhizobiaceae, Alcaligenaceae, Alicyclobacillaceae, Neisseriaceae, Rhodocyclaceae, Bacillaceae, Paenibacillaceae, Burkholderiaceae, Oxalobacteraceae, Gordoniaceae, Rhodospirillaceae, Aurantimonadaceae, Mycobacteriaceae, Sphingomonadaceae, Rhodobacteraceae, Planococcaceae, Pseudomonadaceae, Pseudonocardiaceae, Nocardiaceae, Streptomycetaceae, Streptosporangiaceae, or Gammaproteobacteria_incertae_sedis for 2-hydroxypenta-2,4-dienoate to Acetyl-CoA/pyruvate sub-pathway,
Moraxellaceae, Rhizobiaceae, Alteromonadaceae, Actinosynnemataceae, Micrococcaceae, Burkholderiaceae, Oxalobacteraceae, Geodermatophilaceae, Gordoniaceae, Halomonadaceae, Xanthobacteraceae, Methylobacteriaceae, Mycobacteriaceae, Aeromonadaceae, Rhodobacteraceae, Comamonadaceae, Neisseriaceae, Pseudomonadaceae, Pseudonocardiaceae, Sphingomonadaceae, or Vibrionaceae for Benzoate to Acetyl-CoA via catechol sub-pathway, and
Comamonadaceae, Moraxellaceae, Alcaligenaceae, Rhodocyclaceae, Burkholderiaceae, Polyangiaceae, Oxalobacteraceae, Labilitrichaceae, Oceanospirillales_incertae_sedis for Benzoate to Acetyl-CoA via benzoyl-CoA sub-pathway.

12. The method according to claim 1, wherein the map of microbes capable of surviving in the environmental site of the sample obtained from, and capable of degrading the pollutant to varying degrees, created using the information from the knowledgebase, wherein a first matrix is created using the microbes from the GPE matrix having value 1, a second matrix is created using the microbes from the GPM matrix having value 1 corresponding to its sub-pathways for an isolated Pollutant (Pᵢ), and a third matrix is created of candidate organisms result set with values of sub-pathways in GPM and the corresponding enzymes in GPE as 1, wherein information about the environmental niche where the microbes in the third matrix thrive can be obtained from the DEBG, wherein this information is used to create a Pollutant Organism Environment Matrix (POEM) comprising of each of the one or more pollutants identified in the sample, the organisms capable of degrading it to varying degrees (depending on complete pathway or subpathways present) and the environment from where the organism has been isolated and thrives in.

13. A system (100) for bioremediation of one or more pollutants, the system comprises:
a sample collection module (102) for collecting a sample from an environment site containing the one or more pollutants;
a pollutant isolation and identification module (104) for isolating and identifying the one or more pollutants present in the sample;
a processor (108);
a memory (106) in communication with the processor, wherein the processor configured to perform the steps of:
creating a knowledgebase, wherein the knowledgebase stores:
information of the identified one or more pollutants,
information pertaining to complete degradation pathways and partial degradation pathways identified in microbes that are capable of completely degrading the one or more pollutants or partially degrading the one or more pollutants,
information about respective environmental niches in which the microbes thrive, and
a list of microbes from different environments possessing the particular complete/partial pollutant degradation pathway,
wherein the particular complete degradation pathway refers to a set of genes on a genome of a microbe and/or proteins encoded by the microbe wherein the set of genes and/ or encoded proteins are responsible for complete degradation of a pollutant either to compounds that are safe for the environment site or to compounds that can be assimilated by other microbe(s) residing within the environment,
wherein the partial degradation pathway in the microbe refers to a set of genes or encoded proteins that constitute one or more sub-pathways, wherein a sub-pathway is a subset of the complete degradation pathway encoded within genome of the microbe, and the subpathway degrades the pollutant to an intermediate compound which can be released out into the environment by the microbe and is subsequently taken up by another microbe within the environment, wherein the another microbe possesses another sub-pathway that metabolizes the released intermediate compound,
wherein the knowledgebase comprises a pollutant pathway organism matrix (PPOM), a genome pathway enzyme (GPE) map, a genome pathway master (GPM) map and a database of abundant environmental microbes (DEBG), wherein the GPE map comprises of:
microbial names listed in the DEBG, and
information about active site of each enzyme involved in a plurality of sub-pathways on each genome of the microbe, for each of the one or more pollutants identified in the collected sample,
wherein creating the knowledgebase comprising:
employing literature mining techniques to identify degradation pathway(s) and corresponding genes/proteins in microbes, wherein the pathway(s) degrade the one or more isolated and identified pollutants, and wherein the literature mining also results in identification of a set of microbes in which the degradation pathway/ pathways are characterized, and wherein the literature mining results in obtaining information on the environmental niche in which the microbes reside and are isolated;
identifying a plurality of sub-pathways within the degradation pathway that completely or partial degrade the isolated one or more pollutants, wherein the genes and/or proteins or enzymes corresponding to each of the plurality of sub-pathways is encoded by genome of a single microbe or genomes of a plurality of microbes, and the product formed by each of the plurality of sub-pathways is released into the environment site, and wherein the product is metabolized by the single microbe or is taken up by other microbe(s) inhabiting the environment, and wherein the other plurality of microbes possess capability of metabolizing the product;
creating the PPOM using the information on identified degradation pathway for each one or more identified pollutants, the plurality of sub-pathways for the degradation pathway, the set of microbes in which the degradation pathway is characterized and the information based on literature mining and manual curation about the respective environmental niche/niches from which the set of microbes are isolated;
employing the literature mining techniques to create the DEBG, wherein the DEBG comprises of information pertaining to microbe(s) and the different environmental niches in which the microbe(s) thrive;
creating a pathway domain map (PDM) from a precreated protein family database (pfamDB), wherein the protein domains included in the PDM are those corresponding to genes/proteins constituting the plurality of sub-pathways that comprise each degradation pathway present in the created PPOM for the one or more pollutants;
creating a genome map (GM), wherein the genome map comprises information pertaining to all microbial genomes reported in National Centre of Biotechnology Information, NCBI, wherein the information also comprises of a listing of genes ordered as per their respective genomic locations in a microbe as well as the constituent protein domains encoded within these genes;
searching presence of protein domains included in PDM for each of the plurality of sub-pathways for all pathways listed in the PPOM on the genomes of microbes stored in the DEBG to determine occurrence of these sub-pathways on the genomes, wherein the search is performed using the genome map GM as a database, and wherein the subpathway from the PDM is considered to be present if a number of domains in the genome contributing to this sub-pathway as listed in the PDM occur within a window size of genes on the genome and cross a predefined threshold value;
creating the GPM with microbial names corresponding to the microbial genomes in the DEBG, and information about presence or absence of plurality of pathways and the plurality of subpathways on the genome, for each of the one or more pollutants identified in the collected sample, and wherein the GPM map has a value of 0 or 1 based on a first predefined criterion, and wherein the GPM provides the information about all sub-pathways for a given pollutant degradation pathway that are present within each of the microbial genomes listed in the GPM; and
creating the GPE map, wherein the GPE map has a value of 0 or 1 based on a second predefined criterion;
identifying a list of partial pollutant degraders and a list of complete pollutant degraders for each of the one or more pollutants identified in the sample by utilizing the information from the knowledgebase, wherein partial pollutant degraders refer to microbes that contribute one or more sub-pathways and the corresponding set of genes, encoded proteins or enzymes, that convert a pollutant to an intermediate compound, and wherein multiple partial degraders combinatorially contribute all sub-pathways for complete degradation of the pollutant identified in the collected sample, wherein complete pollutant degraders possess a combination of all sub-pathways and the corresponding set of genes, encoded proteins or enzymes within a single microbe for degradation of the pollutant identified in the collected sample;
creating a map of microbes using the information from the knowledgebase, wherein the map of microbes comprises information of one or more of the partial pollutant degraders and complete pollutant degraders, capable of degrading each pollutant within the one or more pollutants identified within the sample to a varying degrees of degradation, wherein the varying degrees of degradation for the pollutant refers to the degradation of a pollutant to different intermediate compounds or metabolites and wherein the intermediate compounds or metabolites are determined by final product(s) released by the degrader upon the action of genes or proteins or enzymes corresponding to the sub-pathway(s) present within the genome of the degrader for the degradation of the pollutant, and wherein the intermediate compounds can either be released into the environment and utilized by other microbes within the environment or can be assimilated within the same microbe which carries out this degradation;
designing a first microbial consortia using the created map of microbes comprising of microbes which together contribute subpathways required for complete degradation of the one or more pollutants identified in the sample and wherein the microbes can survive together in the same environmental niche from where the sample has been collected;
designing a second microbial consortia using the created map of microbes comprising of microbes which together contribute genes, proteins and enzymes for sub-pathways required for partial degradation of the one or more pollutants identified in the collected sample to desired intermediate product/products, wherein the microbes forming the second microbial consortia can survive together in the environmental niche from where the sample has been collected;
an administration module (114) for administering a concoction of at least one or both of the first microbial consortia and the second microbial consortia to the environmental site containing the one or more pollutants;
an efficacy module (116) for checking efficacy of the administered concoctions on the elimination of one or more pollutants in a sample collected from the environmental site, wherein the assessment of efficacy is done by isolating and identifying remaining set of pollutants from the collected sample; and
the administration module (114) for re-administering a new concoction on the environmental site, wherein the new concoction is made by adding a set of microbes which can act as partial degraders and combinatorially degrade the one or more pollutants identified in the collected sample.

14. One or more non-transitory machine readable information storage mediums comprising one or more instructions which when executed by one or more hardware processors cause:
collecting a sample from an environment site containing the one or more pollutants;
isolating and identifying the one or more pollutants present in the sample;
creating a knowledgebase, wherein the knowledgebase stores
information of the identified one or more pollutants,
information pertaining to complete degradation pathways and partial degradation pathways identified in microbes that are capable of completely degrading the one or more pollutants or partially degrading the one or more pollutants,
information about respective environmental niches in which the microbes thrive, and
a list of microbes from different environments possessing the particular complete/partial pollutant degradation pathway,
wherein the particular complete degradation pathway refers to a set of genes on a genome of a microbe and/or proteins encoded by the microbe wherein the set of genes and/ or encoded proteins are responsible for complete degradation of a pollutant either to compounds that are safe for the environment site or to compounds that can be assimilated by other microbe(s) residing within the environment,
wherein the partial degradation pathway in the microbe refers to a set of genes or encoded proteins that constitute one or more subpathways, wherein a sub-pathway is a subset of the complete degradation pathway encoded within genome of the microbe, and the sub-pathway degrades the pollutant to an intermediate compound which can be released out into the environment by the microbe and is subsequently taken up by another microbe within the environment, wherein the another microbe possesses another sub-pathway that metabolizes the released intermediate compound,
wherein the knowledgebase comprises a pollutant pathway organism matrix (PPOM), a genome pathway enzyme (GPE) map, a genome pathway master (GPM) map and a database of abundant environmental microbes (DEBG), wherein the GPE map comprises of:
microbial names listed in the DEBG, and
information about active site of each enzyme involved in a plurality of sub-pathways on each genome of the microbe, for each of the one or more pollutants identified in the collected sample,
wherein the step of creating the knowledgebase comprising:
employing literature mining techniques to identify degradation pathway(s) and corresponding genes/proteins in microbes, wherein the pathway(s) degrade the one or more isolated and identified pollutants, and wherein the literature mining also results in identification of a set of microbes in which the degradation pathway/ pathways are characterized, and wherein the literature mining results in obtaining information on the environmental niche in which the microbes reside and are isolated;
identifying a plurality of sub-pathways within the degradation pathway that completely or partial degrade the isolated one or more pollutants, wherein the genes and/or proteins or enzymes corresponding to each of the plurality of sub-pathways is encoded by genome of a single microbe or genomes of a plurality of microbes, and the product formed by each of the plurality of sub-pathways is released into the environment site, and wherein the product is metabolized by the single microbe or is taken up by other microbe(s) inhabiting the environment, and wherein the other plurality of microbes possess capability of metabolizing the product;
creating the PPOM using the information on identified degradation pathway for each one or more identified pollutants, the plurality of sub-pathways for the degradation pathway, the set of microbes in which the degradation pathway is characterized and the information based on literature mining and manual curation about the respective environmental niche/niches from which the set of microbes are isolated;
employing the literature mining techniques to create the DEBG, wherein the DEBG comprises of information pertaining to microbe(s) and the different environmental niches in which the microbe(s) thrive;
creating a pathway domain map (PDM) from a precreated protein family database (pfamDB), wherein the protein domains included in the PDM are those corresponding to genes/proteins constituting the plurality of sub-pathways that comprise each degradation pathway present in the created PPOM for the one or more pollutants;
creating a genome map (GM), wherein the genome map comprises information pertaining to all microbial genomes reported in National Centre of Biotechnology Information, NCBI, wherein the information also comprises of a listing of genes ordered as per their respective genomic locations in a microbe as well as the constituent protein domains encoded within these genes;
searching presence of protein domains included in PDM for each of the plurality of sub-pathways for all pathways listed in the PPOM on the genomes of microbes stored in the DEBG to determine occurrence of these sub-pathways on the genomes, wherein the search is performed using the genome map GM as a database, and wherein the subpathway from the PDM is considered to be present if a number of domains in the genome contributing to this sub-pathway as listed in the PDM occur within a window size of genes on the genome and cross a predefined threshold value;
creating the GPM with microbial names corresponding to the microbial genomes in the DEBG, and information about presence or absence of plurality of pathways and the plurality of subpathways on the genome, for each of the one or more pollutants identified in the collected sample, and wherein the GPM map has a value of 0 or 1 based on a first predefined criterion, and wherein the GPM provides the information about all sub-pathways for a given pollutant degradation pathway that are present within each of the microbial genomes listed in the GPM; and
creating the GPE map, wherein the GPE map has a value of 0 or 1 based on a second predefined criterion;
identifying a list of partial pollutant degraders and a list of complete pollutant degraders for each of the one or more pollutants identified in the sample by utilizing the information from the knowledgebase, wherein partial pollutant degraders refer to microbes that contribute one or more subpathways and the corresponding set of genes, encoded proteins or enzymes, that convert a pollutant to an intermediate compound, and wherein multiple partial degraders combinatorially contribute all sub-pathways for complete degradation of the pollutant identified in the collected sample, wherein complete pollutant degraders possess a combination of all subpathways and the corresponding set of genes, encoded proteins or enzymes within a single microbe for degradation of the pollutant identified in the collected sample;
creating a map of microbes using the information from the knowledgebase, wherein the map of microbes comprises information of one or more of the partial pollutant degraders and complete pollutant degraders, capable of degrading each pollutant within the one or more pollutants identified within the sample to a varying degrees of degradation, wherein the varying degrees of degradation for the pollutant refers to the degradation of a pollutant to different intermediate compounds or metabolites and wherein the intermediate compounds or metabolites are determined by final product(s) released by the degrader upon the action of genes or proteins or enzymes corresponding to the subpathway(s) present within the genome of the degrader for the degradation of the pollutant, and wherein the intermediate compounds can either be released into the environment and utilized by other microbes within the environment or can be assimilated within the same microbe which carries out this degradation;
designing a first microbial consortia using the created map of microbes comprising of microbes which together contribute sub-pathways required for complete degradation of the one or more pollutants identified in the sample and wherein the microbes can survive together in the same environmental niche from where the sample has been collected;
designing a second microbial consortia using the created map of microbes comprising of microbes which together contribute genes, proteins and enzymes for sub-pathways required for partial degradation of the one or more pollutants identified in the collected sample to desired intermediate product/products, wherein the microbes forming the second microbial consortia can survive together in the environmental niche from where the sample has been collected;
administering a concoction of at least one or both of the first microbial consortia and the second microbial consortia to the environmental site containing the one or more pollutants by using an administration module (114);
checking efficacy of the administered concoctions on the elimination of one or more pollutants in a sample collected from the environmental site, wherein the assessment of efficacy is done by isolating and identifying remaining set of pollutants from the collected sample by using an efficacy module (116); and
re-administering a new concoction on the environmental site, wherein the new concoction is made by adding a set of microbes which can act as partial degraders and combinatorially degrade the one or more pollutants identified in the collected sample by using the administration module (114).

## Patentansprüche

1. Verfahren (200) zur Biosanierung eines oder mehrerer Schadstoffe, wobei das Verfahren umfasst:
Sammeln einer Probe von einer Umgebung, die den einen oder die mehreren Schadstoffe enthält (202);
Isolieren und Identifizieren des einen oder der mehreren Schadstoffe, die in der Probe vorhanden sind (204);
Erstellen einer Wissensbasis, wobei die Wissensbasis speichert
Informationen über den identifizierten einen oder die identifizierten mehreren Schadstoffe,
Informationen über vollständige Abbauwege und teilweise Abbauwege, die in Mikroben identifiziert werden, die in der Lage sind, den einen oder die mehreren Schadstoffe vollständig abzubauen oder den einen oder die mehreren Schadstoffe teilweise abzubauen,
Informationen über jeweilige Umgebungsnischen, in denen die Mikroben gedeihen, und
eine Liste von Mikroben aus verschiedenen Umgebungen, die den bestimmten vollständigen/teilweisen Schadstoffabbauweg besitzen,
wobei sich der bestimmte vollständige Abbauweg auf einen Satz von Genen auf einem Genom einer Mikrobe und/oder von der Mikrobe codierte Proteine bezieht, wobei der Satz von Genen und/oder codierten Proteinen für den vollständigen Abbau eines Schadstoffs entweder für Verbindungen, die für die Umgebung sicher sind, oder für Verbindungen, die von anderen Mikroben, die sich in der Umgebung befinden, assimiliert werden können, verantwortlich ist,
wobei sich der teilweise Abbauweg in der Mikrobe auf einen Satz von Genen oder codierten Proteinen bezieht, die einen oder mehrere Teilwege bilden, wobei ein Teilweg eine Teilmenge des vollständigen Abbauwegs ist, der im Genom der Mikrobe codiert ist, und der Teilweg den Schadstoff zu einer Zwischenverbindung abbaut, die von der Mikrob in die Umgebung freigesetzt werden kann und anschließend von einer anderen Mikrobe in der Umgebung aufgenommen wird, wobei die andere Mikrobe einen anderen Teilweg besitzt, der die freigesetzte Zwischenverbindung metabolisiert,
wobei die Wissensdatenbank eine Schadstoffweg-Organismus-Matrix (PPOM), eine Genomweg-Enzym-Karte (GPE-Karte), eine Genomweg-Master-Karte (GPM-Karte) und eine Datenbank von zahlreichen Umgebungsmikroben (DEBG) umfasst, wobei die GPE-Karte Folgendes umfasst:
Mikrobennamen, die in der DEBG aufgelistet sind, und
Informationen über die aktive Stelle jedes Enzyms, das an einer Mehrzahl von Teilwegen auf jedem Genom der Mikrobe beteiligt ist, für jeden des einen oder der mehreren Schadstoffe, die in der gesammelten Probe identifiziert werden (206),
wobei der Schritt des Erstellens der Wissensdatenbank umfasst:
Anwenden von Literatur-Mining-Techniken, um (einen) Abbauweg(e) und entsprechende Gene/Proteine in Mikroben zu identifizieren, wobei der (die) Weg(e) den einen oder die mehreren isolierten und identifizierten Schadstoffe abbaut (abbauen), und wobei das Literatur-Mining auch zur Identifizierung eines Satzes von Mikroben führt, in denen der (die) Abbauweg(e) charakterisiert ist (sind), und wobei das Literatur-Mining zum Erhalten von Informationen über die Umgebungsnische führt, in der sich die Mikroben befinden und isoliert sind;
Identifizieren einer Mehrzahl von Teilwegen innerhalb des Abbauwegs, die den isolierten einen oder die isolierten mehreren Schadstoffe vollständig oder teilweise abbauen, wobei die Gene und/oder Proteine oder Enzyme, die jedem der Mehrzahl von Teilwegen entsprechen, durch das Genom einer einzelnen Mikrobe oder die Genome einer Mehrzahl von Mikroben codiert sind, und das Produkt, das von jedem der Mehrzahl von Teilwegen gebildet wird, in die Umgebung freigesetzt wird, und wobei das Produkt von der einzelnen Mikrobe metabolisiert wird oder von anderen Mikroben aufgenommen wird, die die Umgebung bewohnen, und wobei die andere Mehrzahl von Mikroben die Fähigkeit besitzt, das Produkt zu metabolisieren;
Erstellen der PPOM unter Verwendung der Informationen über den identifizierten Abbauweg für jeden des einen oder der mehreren identifizierten Schadstoffe, der Mehrzahl von Teilwegen für den Abbauweg, des Satzes von Mikroben, in denen der Abbauweg charakterisiert ist, und der Informationen, die auf dem Literatur-Mining und der manuellen Kuration über die jeweilige(n) Umgebungsnische(n) basieren, aus der/denen der Satz von Mikroben isoliert ist;
Anwenden der Literatur-Mining-Techniken, um die DEBG zu erstellen, wobei die DEBG Informationen umfasst, die sich auf (eine) Mikrobe(n) und die verschiedenen Umgebungsnischen beziehen, in denen die Mikrobe(n) gedeiht/gedeihen;
Erstellen einer Weg-Domänen-Karte (PDM) aus einer vorerstellten Proteinfamilien-Datenbank (pfamDB), wobei die Proteindomänen, die in der PDM enthalten sind, diejenigen sind, die Genen/Proteinen entsprechen, die die Mehrzahl von Teilwegen bilden, die jeden Abbauweg umfassen, der in der erstellten PPOM für den einen oder die mehreren Schadstoffe vorhanden ist;
Erstellen einer Genomkarte (GM), wobei die Genomkarte Informationen umfasst, die sich auf alle Mikrobengenome beziehen, die im National Centre of Biotechnology Information, NCBI, berichtet werden, wobei die Informationen auch eine Auflistung von Genen umfassen, die nach ihren jeweiligen genomischen Orten in einer Mikrobe sowie den konstituierenden Proteindomänen, die innerhalb dieser Gene codiert sind, geordnet sind;
Suchen des Vorhandenseins von Proteindomänen, die in der PDM enthalten sind, für jeden der Mehrzahl von Teilwegen für alle Wege, die in der PPOM auf den Genomen von Mikroben aufgelistet sind, die in der DEBG gespeichert sind, um das Auftreten dieser Teilwege auf den Genomen zu bestimmen, wobei die Suche unter Verwendung der Genomkarte GM als eine Datenbank durchgeführt wird, und wobei der Teilweg aus der PDM als vorhanden betrachtet wird, wenn eine Anzahl von Domänen in dem Genom, die zu diesem Teilweg beitragen, wie in der PDM aufgelistet, innerhalb einer Fenstergröße von Genen auf dem Genom auftreten und einen vordefinierten Schwellenwert überschreiten;
Erstellen der GPM mit Mikrobennamen, die den Mikrobengenomen in der DEBG entsprechen, und Informationen über das Vorhandensein oder Fehlen einer Mehrzahl von Wegen und der Mehrzahl von Teilwegen auf dem Genom für jeden des einen oder der mehreren Schadstoffe, die in der gesammelten Probe identifiziert werden, und wobei die GPM-Karte einen Wert von 0 oder 1 basierend auf einem ersten vordefinierten Kriterium aufweist, und wobei die GPM die Informationen über alle Teilwege für einen gegebenen Schadstoffabbauweg bereitstellt, die innerhalb jedes der Mikrobengenomen vorhanden sind, die in der GPM aufgelistet sind; und
Erstellen der GPE-Karte, wobei die GPE-Karte einen Wert von 0 oder 1 basierend auf einem zweiten vordefinierten Kriterium aufweist;
Identifizieren einer Liste von teilweisen Schadstoffabbaumitteln und einer Liste von vollständigen Schadstoffabbaumitteln für jeden des einen oder der mehreren Schadstoffe, die in der Probe identifiziert werden, unter Verwendung der Informationen aus der Wissensdatenbank, wobei sich teilweise Schadstoffabbaumittel auf Mikroben beziehen, die einen oder mehrere Teilwege und den entsprechenden Satz von Genen, codierten Proteinen oder Enzymen, die einen Schadstoff in eine Zwischenverbindung umwandeln, beitragen, und wobei mehrere teilweise Abbaumittel kombinatorisch alle Teilwege für den vollständigen Abbau des Schadstoffs beitragen, der in der gesammelten Probe identifiziert wird, wobei vollständige Schadstoffabbaumittel eine Kombination aller Teilwege und des entsprechenden Satzes von Genen, codierten Proteinen oder Enzymen innerhalb einer einzelnen Mikrobe für den Abbau des Schadstoffs besitzen, der in der gesammelten Probe identifiziert wird (208);
Erstellen einer Karte von Mikroben unter Verwendung der Informationen aus der Wissensdatenbank, wobei die Karte von Mikroben Informationen über einen oder mehrere der teilweisen Schadstoffabbaumittel und vollständigen Schadstoffabbaumittel umfasst, die in der Lage sind, j eden Schadstoff innerhalb des einen oder der mehreren Schadstoffe, die in der Probe identifiziert werden, auf unterschiedliche Abbaugrade abzubauen, wobei sich die unterschiedlichen Abbaugrade für den Schadstoff auf den Abbau eines Schadstoffs auf unterschiedliche Zwischenverbindungen oder Metabolite beziehen und wobei die Zwischenverbindungen oder Metabolite durch Endprodukt(e) bestimmt werden, das/die von dem Abbaumittel bei der Einwirkung von Genen oder Proteinen oder Enzymen freigesetzt wird/werden, die dem/den Teilweg(en) entsprechen, der/die innerhalb des Genoms des Abbaumittels für den Abbau des Schadstoffs vorhanden ist/sind, und wobei die Zwischenverbindungen entweder in die Umgebung freigesetzt und von anderen Mikroben innerhalb der Umgebung verwendet werden oder innerhalb derselben Mikrobe, die diesen Abbau ausführt, assimiliert werden können (210);
Entwerfen eines ersten mikrobiellen Konsortiums unter Verwendung der erstellten Karte von Mikroben, die Mikroben umfasst, die zusammen Teilwege beitragen, die für den vollständigen Abbau des einen oder der mehreren Schadstoffe, die in der Probe identifiziert werden, erforderlich sind, und wobei die Mikroben zusammen in derselben Umgebungsnische überleben können, aus der die Probe gesammelt wurde (212);
Entwerfen eines zweiten mikrobiellen Konsortiums unter Verwendung der erstellten Karte von Mikroben, die Mikroben umfasst, die zusammen Gene, Proteine und Enzyme für Teilwege beitragen, die für den teilweisen Abbau des einen oder der mehreren Schadstoffe, die in der gesammelten Probe identifiziert werden, auf das/die gewünschte(n) Zwischenprodukt(e) erforderlich sind, wobei die Mikroben, die das zweite mikrobielle Konsortium bilden, zusammen in der Umgebungsnische überleben können, aus der die Probe gesammelt wurde (214);
Verabreichen einer Mixtur von mindestens einem oder beiden des ersten mikrobiellen Konsortiums und des zweiten mikrobiellen Konsortiums an die Umgebungsstelle, die den einen oder die mehreren Schadstoffe enthält (216);
Überprüfen der Wirksamkeit der verabreichten Mixturen bei der Eliminierung von einem oder mehreren Schadstoffen in einer Probe, die von der Umgebungsstelle gesammelt wurde, wobei die Beurteilung der Wirksamkeit durch Isolieren und Identifizieren des verbleibenden Satzes von Schadstoffen aus der gesammelten Probe erfolgt (218); und
erneutes Verabreichen einer neuen Mixtur an die Umgebungsstelle, wobei die neue Mixtur durch Hinzufügen eines Satzes von Mikroben erfolgt, die als teilweise Abbaumittel wirken und den einen oder die mehreren Schadstoffe, die in der gesammelten Probe identifiziert werden, kombinatorisch abbauen können (220).

2. Verfahren nach Anspruch 1, wobei das erste vordefinierte Kriterium für jeden Teilweg in einem mikrobiellen Genom ist:
ein Wert von 0 wird zugewiesen, wenn Proteindomänen, die einem Teilweg entsprechen, wie in "PDM" aufgezeichnet, entweder nicht auftreten oder einen Schwellenwert innerhalb eines vordefinierten Fensters von Genen nicht erreichen, und
ein Wert von 1 wird zugewiesen, wenn Teilweg-Proteindomänen, wie in "PDM" aufgezeichnet, oberhalb des Schwellenwerts innerhalb des vordefinierten Fensters von Genen vorhanden sind.

3. Verfahren nach Anspruch 2, wobei
der Schwellenwert basierend auf dem Literatur-Mining und der manuellen Kuration bestimmt wird und der minimalen Schwellenanzahl von Domänen sowie den Domänen entspricht, deren Vorhandensein erforderlich ist, um das Vorhandensein des Teilwegs innerhalb eines mikrobiellen Genoms zu bestätigen, wobei der Schwellenwert als ein Bruchteil der erforderlichen Domänen aus der Gesamtanzahl von Domänen definiert ist, die diesem Teilweg in der PDM entsprechen, und
das vordefinierte Fenster von Genen unter Verwendung der manuellen Kuration definiert wird, die den Abstand in Bezug auf die Anzahl von Genen auf der Basis des Genomorts angibt, innerhalb dessen die Domänen als einen Teilweg bildend betrachtet werden können, wobei die Gene, die Proteindomänen codieren, die einen Teilweg bilden, zusammen auf dem mikrobiellen Genom auftreten, wodurch sie sich innerhalb einer definierten Fenstergröße von Genen auf dem Genom befinden.

4. Verfahren nach Anspruch 1, wobei das zweite vordefinierte Kriterium für jedes Enzym, das jedem Schritt eines Teilwegs entspricht, der in dem mikrobiellen Genom identifiziert wird, ist:
ein Wert von 1 wird den Enzymen zugewiesen, wo ein aktives Stellenmuster für dieses Enzym gefunden wird, wobei das aktive Stellenmuster für konstituierende Enzyme jedes Kandidatenwegs das Muster ist, das für die aktive Stelle des Enzyms spezifisch ist, das durch Literatur-Mining erhalten wird, wobei das aktive Stellenmuster durch Motive identifiziert wird, die als Signatursequenzen wirken, die beim Identifizieren helfen, ob das Enzym funktionell in der Lage ist, an ein Substrat zu binden, und über alle Enzyme mit ähnlicher Funktionalität konserviert sind, und wobei die Motive durch multiples Sequenzalignment, MSA, über alle möglichen funktionell ähnlichen Homologe des Enzyms identifiziert werden, um konservierte Aminosäuremuster über die Enzyme zu identifizieren, die in der Literatur validiert werden, um die funktionelle Wichtigkeit zu bewerten, und
ein Wert von 0 wird zugewiesen, falls das aktive Stellenmuster für das Enzym nicht gefunden wird.

5. Verfahren nach Anspruch 1, ferner umfassend das Testen des Vorhandenseins der Sekretionskapazität für jedes Enzym und das Aktualisieren des Werts von 0 auf das Fehlen oder 1 auf das Vorhandensein der Sekretionskapazität in der GPE-Karte.

6. Verfahren nach Anspruch 1, wobei sich das gewünschte Zwischenprodukt auf einen Satz von Zwischenprodukten bezieht, die während des teilweisen Abbaus des einen oder der mehreren identifizierten Schadstoffe abgeleitet werden.

7. Verfahren nach Anspruch 1, wobei die Wissensbasis Informationen speichert, die sich auf einen oder mehrere Schadstoffe beziehen, wobei einige dieser Schadstoffe Polyethylenterephthalat (PET), Styrol, Polyurethan, polyaromatische Kohlenwasserstoffe (PAH), verschiedene Kongenerzien von Polychlorbiphenylen (PCB) oder kohlenstoffbasierte Nanomaterialien (CBNMs) beinhalten.

8. Verfahren nach Anspruch 7, wobei der vollständige CBNM-Abbau das Vorhandensein eines bifunktionellen Katalase-Peroxidase (kat)-Enzyms und der Teilwege für den Abbau eines oder mehrerer der Zwischenprodukte, die nach der katalytischen Wirkung des kat-Enzyms gebildet werden, beinhaltet, wobei die Zwischenprodukte einen oder mehrere von PAH-, PCB- und einzelaromatischem Kohlenwasserstoff-, SAH-, Abbau umfassen, der als Ergebnis der katalytischen Wirkung von Katalase-Peroxidase auf CBNM gebildet wird.

9. Verfahren nach Anspruch 7, wobei der vollständige PET-Abbau die Teilwege für PETase, gefolgt von Terephthalsäure-, TPA-, zu Protocatechusäure-, PCA-, Umwandlung und die Kandidatenbakterienfamilie, die an diesen Teilwegen für den PET-Abbau beteiligt sind, beinhaltet, umfassend eines oder mehrere von:
Polyangiaceae, Burkholderiaceae, Burkholderiales_incertae_sedis, Alteromonadaceae, Oceanospirillaceae, Pseudomonadaceae oder Vibrionaceae,
Comamonadaceae, Bacillaceae, Bradyrhizobiaceae, Burkholderiaceae, Piscirickettsiaceae, Sphingomonadaceae, Hyphomicrobiaceae, Pseudomonadaceae, Pseudonocardiaceae, Oxalobacteraceae, Rhizobiaceae, Nocardiaceae, Rhodocyclaceae oder Streptomycetaceae für Terepthalsäure zu Protocatechusäure-Teilweg, und
Actinosynnemataceae, Caulobacteraceae, Oxalobacteraceae, Streptomycetaceae, Micrococcaceae, Rhizobiaceae, Myxococcaceae, Nocardiaceae, Brucellaceae, Nocardiopsaceae, Oceanospirillaceae, Planococcaceae, Pseudonocardiaceae, Actinopolysporaceae, Streptosporangiaceae, Xanthomonadaceae, Hyphomicrobiaceae, Rhodobacteraceae, Mycobacteriaceae, Microbacteriaceae, Alcaligenaceae, Geodermatophilaceae, Burkholderiaceae, Enterobacteriaceae, Halomonadaceae, Moraxellaceae, Dietziaceae, Phyllobacteriaceae, Sphingomonadaceae, Rhodospirillaceae, Micromonosporaceae, Comamonadaceae, Pseudomonadaceae, Aeromonadaceae, Alteromonadaceae, Aurantimonadaceae, Cytophagaceae, Neisseriaceae, Deinococcaceae, Nocardioidaceae, Vibrionaceae, Kiloniellaceae, Gordoniaceae, Listeriaceae, Bacillaceae, Xanthobacteraceae, Rubrobacteraceae, Tsukamurellaceae, Bradyrhizobiaceae, Saprospiraceae, Sphingobacteriaceae, Thermaceae, Clostridiaceae, Flavobacteriaceae, Brevibacteriaceae, Corynebacteriaceae, Beijerinckiaceae, Methylobacteriaceae, Cystobacteraceae, Granulosicoccaceae, Glycomycetaceae, Bacillaceae 1, Catenulisporaceae, Sphaerobacteraceae, unklassifizierte Betaproteobacteria, unklassifizierte Burkholderiales, unklassifizierte Flavobacteriales, Yersiniaceae oder Vicinamibacteraceae für Protocatechusäure zu AcetylCoA-Teilweg.

10. Verfahren nach Anspruch 7, wobei der vollständige Abbau der PAHs ferner Folgendes umfasst:
Abbau von Naphthalin, der zwei Teilwege umfasst, die Naphthalin zu Salicylat abbauen, gefolgt von Salicylatabbau über Catechol, um Acetyl Co-A zu bilden,
Abbau von Anthracen, der in Teilwege unterteilt ist, die Anthracen in Dihydroxynaphthalin-Teilweg umwandeln, gefolgt von Salicyalatabbau über Catechol-Stoffwechselweg, um Acetyl Co-A zu bilden, und
Abbau von Phenanthren, der Phenanthren zu Phthalat-Teilweg, Phthalat zu Dihydroxybenzoat-Teilweg und Phenanthren zu Naphthalindiol-Teilweg beinhaltet, und der Kandidatenbakterienfamilie, die an Teilwegen für den Abbau jedes Typs des oben erwähnten PAH beteiligt sind, umfassend eines oder mehrere von:
Comamonadaceae, Rhizobiaceae, Alteromonadaceae, Bacillaceae, Alcaligenaceae, Bradyrhizobiaceae, Burkholderiaceae, Rhodobacteraceae, Erythrobacteraceae, Piscirickettsiaceae, Gordoniaceae, Oceanospirillaceae, Aurantimonadaceae, Oxalobacteraceae, Phyllobacteriaceae, Mycobacteriaceae, Sphingomonadaceae, Hyphomicrobiaceae, Neisseriaceae, Pseudomonadaceae, unklassifizierte Rhizobiales, Nocardiaceae, Rhodocyclaceae oder Streptomycetaceae für Naphthalin zu Salicylat-Teilweg,
Comamonadaceae, Alcaligenaceae, Actinosynnemataceae, Bacillaceae, Geodermatophilaceae, Sphingomonadaceae, Bradyrhizobiaceae, Burkholderiaceae, Caulobacteraceae, Rhodobacteraceae, Frankiaceae, Alteromonadaceae, Phyllobacteriaceae, Mycobacteriaceae, Hyphomicrobiaceae, Erythrobacteraceae, Pseudomonadaceae, Rhizobiaceae, Nocardiaceae, Streptomycetaceae oder Gammaproteobacteria_incertae_sedis für Anthracen zu Dihydroxynaphthalin-Teilweg,
Comamonadaceae, Moraxellaceae, Alcaligenaceae, Alcanivoracaceae, Alicyclobacillaceae, Ectothiorhodospiraceae, Actinosynnemataceae, Phyllobacteriaceae, Neisseriaceae, Rhodocyclaceae, Pseudomonadaceae, Bacillaceae, Thiotrichaceae, Bradyrhizobiaceae, Burkholderiaceae, Clostridiaceae, Rhodobacteraceae, Corynebacteriaceae, Oxalobacteraceae, Piscirickettsiaceae, Frankiaceae, Gordoniaceae, Intrasporangiaceae, Enterobacteriaceae, Planococcaceae, Alteromonadaceae, Aurantimonadaceae, Mycobacteriaceae, Nakamurellaceae, Nocardiaceae, Nocardioidaceae, Sphingomonadaceae, Micrococcaceae, Rhizobiaceae, Streptomycetaceae, Sulfobacillaceae oder Thermomonosporaceae für Catechol zu AcetylCoA-Teilweg,
Comamonadaceae, Alteromonadaceae, Phyllobacteriaceae, Bacillaceae, Bradyrhizobiaceae, Burkholderiaceae, Erythrobacteraceae, Oxalobacteraceae, Mycobacteriaceae, Sphingomonadaceae, Hyphomicrobiaceae, Micrococcaceae, Pseudomonadaceae, Rhizobiaceae, Nocardiaceae oder Streptomycetaceae für Phenanthren zu Phthalat-Teilweg,
Acetobacteraceae, Comamonadaceae, Alcaligenaceae, Bacillaceae, Bradyrhizobiaceae, Brucellaceae, Burkholderiaceae, Halomonadaceae, Colwelliaceae, Corynebacteriaceae, Frankiaceae, Gordoniaceae, Phyllobacteriaceae, Rhodobiaceae, Mycobacteriaceae, Nocardioidaceae, Nostocaceae, Sphingomonadaceae, Rhodobacteraceae, Oxalobacteraceae, Pseudonocardiaceae, Pseudoalteromonadaceae, Pseudomonadaceae, Rhizobiaceae, Nocardiaceae, Alteromonadaceae, Streptomycetaceae, Gomphosphaeriaceae, Rhodospirillaceae, Enterobacteriaceae oder Gammaproteobacteria_incertae_sedis für Phthalat zu Dihydroxybenzoat-Teilweg, und
Comamonadaceae, Bacillaceae, Bradyrhizobiaceae, Burkholderiaceae, Caulobacteraceae, Oxalobacteraceae, Rhodocyclaceae, Frankiaceae, Halomonadaceae, Immundisolibacteraceae, Rhodobacteraceae, Alteromonadaceae, Oceanospirillaceae, Phyllobacteriaceae, Mycobacteriaceae, Nocardiaceae, Sphingomonadaceae, Hyphomicrobiaceae, Enterobacteriaceae, Erythrobacteraceae, Pseudonocardiaceae, Micrococcaceae, Pseudomonadaceae, Rhizobiaceae oder Streptomycetaceae für Phenanthren zu Naphthalindiol-Teilweg.

11. Verfahren nach Anspruch 7, wobei der vollständige Abbau von PCB die Teilwege für die reduktive Dehalogenierung von höher chlorierten PCBs zu Biphenylen beinhaltet, gefolgt von dem Teilweg für die Umwandlung von Biphenyl zu 2-Hydroxypenta-2,4-dienoat, der über einen niedrigeren Weg weiter abgebaut wird, um Pyruvat und Acetyl-CoA zu bilden, zusammen mit den Teilwegen für die gebildeten Zwischenprodukte, die über Benzoyl-Co-A/Catechol-Weg zu Acetyl-Co-A umgewandelt werden, und der Kandidatenbakterienfamilie, die an diesen Teilwegen für den PCB-Abbau beteiligt sind, umfassend eines oder mehrere von:
Dehalococcoidaceae, Peptococcaceae oder Campylobacteraceae für PCB zu Biphenyl-Teilweg,
Comamonadaceae, Alcaligenaceae, Alcanivoracaceae, Rhodocyclaceae, Bacillaceae, Burkholderiaceae, Conexibacteraceae, Corynebacteriaceae, Erythrobacteraceae, Frankiaceae, Aurantimonadaceae, Beijerinckiaceae, Mycobacteriaceae, Sphingomonadaceae, Paenibacillaceae, Hyphomicrobiaceae, Pseudoalteromonadaceae, Pseudomonadaceae, Pseudonocardiaceae, Xanthomonadaceae, Rhizobiaceae, Nocardiaceae, Alteromonadaceae, Planococcaceae oder Spongiibacteraceae für Biphenyl zu Acetyl-CoA/Pyruvat-Teilweg,
Comamonadaceae, Alcaligenaceae, Rhodocyclaceae, Bacillaceae, Bradyrhizobiaceae, Burkholderiaceae, Rhodobacteraceae, Corynebacteriaceae, Immundisolibacteraceae, Beijerinckiaceae, Mycobacteriaceae, Nocardioidaceae, Sphingomonadaceae, Pseudomonadaceae, Rhizobiaceae, Nocardiaceae oder Streptomycetaceae für Biphenyl zu 2-Hydroxypenta-2,4-dienoat-Teilweg,
Comamonadaceae, Rhizobiaceae, Alcaligenaceae, Alicyclobacillaceae, Neisseriaceae, Rhodocyclaceae, Bacillaceae, Paenibacillaceae, Burkholderiaceae, Oxalobacteraceae, Gordoniaceae, Rhodospirillaceae, Aurantimonadaceae, Mycobacteriaceae, Sphingomonadaceae, Rhodobacteraceae, Planococcaceae, Pseudomonadaceae, Pseudonocardiaceae, Nocardiaceae, Streptomycetaceae, Streptosporangiaceae oder Gammaproteobacteria_incertae_sedis für 2-Hydroxypenta-2,4-dienoat zu Acetyl-CoA/Pyruvat-Teilweg,
Moraxellaceae, Rhizobiaceae, Alteromonadaceae, Actinosynnemataceae, Micrococcaceae, Burkholderiaceae, Oxalobacteraceae, Geodermatophilaceae, Gordoniaceae, Halomonadaceae, Xanthobacteraceae, Methylobacteriaceae, Mycobacteriaceae, Aeromonadaceae, Rhodobacteraceae, Comamonadaceae, Neisseriaceae, Pseudomonadaceae, Pseudonocardiaceae, Sphingomonadaceae oder Vibrionaceae für Benzoat zu Acetyl-CoA über Catechol-Teilweg und
Comamonadaceae, Moraxellaceae, Alcaligenaceae, Rhodocyclaceae, Burkholderiaceae, Polyangiaceae, Oxalobacteraceae, Labilitrichaceae, Oceanospirillales_incertae_sedis für Benzoat zu Acetyl-CoA über Benzoyl-CoA-Teilweg.

12. Verfahren nach Anspruch 1, wobei die Karte von Mikroben, die in der Lage sind, an der aus der Probe erhaltenen Umgebungsstelle zu überleben, und in der Lage sind, den Schadstoff in unterschiedlichem Maße abzubauen, unter Verwendung der Informationen aus der Wissensdatenbank erstellt wird, wobei eine erste Matrix unter Verwendung der Mikroben aus der GPE-Matrix mit dem Wert 1 erstellt wird, eine zweite Matrix unter Verwendung der Mikroben aus der GPM-Matrix mit dem Wert 1 entsprechend ihren Teilwegen für einen isolierten Schadstoff (Pᵢ) erstellt wird und eine dritte Matrix von Kandidatenorganismen-Ergebnissätzen mit Werten von Teilwegen in GPM und den entsprechenden Enzymen in GPE als 1 erstellt wird, wobei Informationen über die Umgebungsnische, in der die Mikroben in der dritten Matrix gedeihen, aus der DEBG erhalten werden können, wobei diese Informationen verwendet werden, um eine Schadstofforganismus-Umgebungsmatrix (POEM) zu erstellen, die jeden des einen oder der mehreren Schadstoffe, die in der Probe identifiziert wurden, die Organismen, die in der Lage sind, ihn in unterschiedlichem Maße abzubauen (abhängig vom vorhandenen vollständigen Weg oder den vorhandenen Teilwegen) und die Umgebung, aus der der Organismus isoliert wurde und in der er gedeiht, umfasst.

13. System (100) zur Biosanierung eines oder mehrerer Schadstoffe, wobei das System umfasst:
ein Probensammelmodul (102) zum Sammeln einer Probe von einer Umgebung, die den einen oder die mehreren Schadstoffe enthält;
ein Schadstoffisolier- und -identifizierungsmodul (104) zum Isolieren und Identifizieren des einen oder der mehreren Schadstoffe, die in der Probe vorhanden sind;
einen Prozessor (108);
einen Speicher (106) in Kommunikation mit dem Prozessor, wobei der Prozessor konfiguriert ist, um die folgenden Schritte durchzuführen:
Erstellen einer Wissensbasis, wobei die Wissensbasis speichert:
Informationen über den identifizierten einen oder die identifizierten mehreren Schadstoffe,
Informationen über vollständige Abbauwege und teilweise Abbauwege, die in Mikroben identifiziert werden, die in der Lage sind, den einen oder die mehreren Schadstoffe vollständig abzubauen oder den einen oder die mehreren Schadstoffe teilweise abzubauen,
Informationen über jeweilige Umgebungsnischen, in denen die Mikroben gedeihen, und
eine Liste von Mikroben aus verschiedenen Umgebungen, die den bestimmten vollständigen/teilweisen Schadstoffabbauweg besitzen,
wobei sich der bestimmte vollständige Abbauweg auf einen Satz von Genen auf einem Genom einer Mikrobe und/oder von der Mikrobe codierte Proteine bezieht, wobei der Satz von Genen und/oder codierten Proteinen für den vollständigen Abbau eines Schadstoffs entweder für Verbindungen, die für die Umgebung sicher sind, oder für Verbindungen, die von anderen Mikroben, die sich in der Umgebung befinden, assimiliert werden können, verantwortlich ist,
wobei sich der teilweise Abbauweg in der Mikrobe auf einen Satz von Genen oder codierten Proteinen bezieht, die einen oder mehrere Teilwege bilden, wobei ein Teilweg eine Teilmenge des vollständigen Abbauwegs ist, der im Genom der Mikrobe codiert ist, und der Teilweg den Schadstoff zu einer Zwischenverbindung abbaut, die von der Mikrobe in die Umgebung freigesetzt werden kann und anschließend von einer anderen Mikrobe in der Umgebung aufgenommen wird, wobei die andere Mikrobe einen anderen Teilweg besitzt, der die freigesetzte Zwischenverbindung metabolisiert,
wobei die Wissensdatenbank eine Schadstoffweg-Organismus-Matrix (PPOM), eine Genomweg-Enzym-Karte (GPE-Karte), eine Genomweg-Master-Karte (GPM-Karte) und eine Datenbank von zahlreichen Umgebungsmikroben (DEBG) umfasst, wobei die GPE-Karte Folgendes umfasst:
Mikrobennamen, die in der DEBG aufgelistet sind, und
Informationen über die aktive Stelle jedes Enzyms, das an einer Mehrzahl von Teilwegen auf jedem Genom der Mikrobe beteiligt ist, für jeden des einen oder der mehreren Schadstoffe, die in der gesammelten Probe identifiziert werden,
wobei das Erstellen der Wissensdatenbank umfasst:
Anwenden von Literatur-Mining-Techniken, um (einen) Abbauweg(e) und entsprechende Gene/Proteine in Mikroben zu identifizieren, wobei der (die) Weg(e) den einen oder die mehreren isolierten und identifizierten Schadstoffe abbaut (abbauen), und wobei das Literatur-Mining auch zur Identifizierung eines Satzes von Mikroben führt, in denen der (die) Abbauweg(e) charakterisiert ist (sind), und wobei das Literatur-Mining zum Erhalten von Informationen über die Umgebungsnische führt, in der sich die Mikroben befinden und isoliert sind;
Identifizieren einer Mehrzahl von Teilwegen innerhalb des Abbauwegs, die den isolierten einen oder die isolierten mehreren Schadstoffe vollständig oder teilweise abbauen, wobei die Gene und/oder Proteine oder Enzyme, die jedem der Mehrzahl von Teilwegen entsprechen, durch das Genom einer einzelnen Mikrobe oder die Genome einer Mehrzahl von Mikroben codiert sind, und das Produkt, das von jedem der Mehrzahl von Teilwegen gebildet wird, in die Umgebung freigesetzt wird, und wobei das Produkt von der einzelnen Mikrobe metabolisiert wird oder von (einer) anderen Mikrobe(n) aufgenommen wird, die die Umgebung bewohnt/bewohnen, und wobei die andere Mehrzahl von Mikroben die Fähigkeit besitzt, das Produkt zu metabolisieren;
Erstellen der PPOM unter Verwendung der Informationen über den identifizierten Abbauweg für jeden des einen oder der mehreren identifizierten Schadstoffe, der Mehrzahl von Teilwegen für den Abbauweg, des Satzes von Mikroben, in denen der Abbauweg charakterisiert ist, und der Informationen, die auf dem Literatur-Mining und der manuellen Kuration über die jeweilige(n) Umgebungsnische(n) basieren, aus der/denen der Satz von Mikroben isoliert ist;
Anwenden der Literatur-Mining-Techniken, um die DEBG zu erstellen, wobei die DEBG Informationen umfasst, die sich auf (eine) Mikrobe(n) und die verschiedenen Umgebungsnischen beziehen, in denen die Mikrobe(n) gedeiht/gedeihen;
Erstellen einer Weg-Domänen-Karte (PDM) aus einer vorerstellten Proteinfamilien-Datenbank (pfamDB), wobei die Proteindomänen, die in der PDM enthalten sind, diejenigen sind, die Genen/Proteinen entsprechen, die die Mehrzahl von Teilwegen bilden, die jeden Abbauweg umfassen, der in der erstellten PPOM für den einen oder die mehreren Schadstoffe vorhanden ist;
Erstellen einer Genomkarte (GM), wobei die Genomkarte Informationen umfasst, die sich auf alle Mikrobengenome beziehen, die im National Centre of Biotechnology Information, NCBI, berichtet werden, wobei die Informationen auch eine Auflistung von Genen umfassen, die nach ihren jeweiligen genomischen Orten in einer Mikrobe sowie den konstituierenden Proteindomänen, die innerhalb dieser Gene codiert sind, geordnet sind;
Suchen des Vorhandenseins von Proteindomänen, die in der PDM enthalten sind, für jeden der Mehrzahl von Teilwegen für alle Wege, die in der PPOM auf den Genomen von Mikroben aufgelistet sind, die in der DEBG gespeichert sind, um das Auftreten dieser Teilwege auf den Genomen zu bestimmen, wobei die Suche unter Verwendung der Genomkarte GM als eine Datenbank durchgeführt wird, und wobei der Teilweg aus der PDM als vorhanden betrachtet wird, wenn eine Anzahl von Domänen in dem Genom, die zu diesem Teilweg beitragen, wie in der PDM aufgelistet, innerhalb einer Fenstergröße von Genen auf dem Genom auftreten und einen vordefinierten Schwellenwert überschreiten;
Erstellen der GPM mit Mikrobennamen, die den Mikrobengenomen in der DEBG entsprechen, und Informationen über das Vorhandensein oder Fehlen einer Mehrzahl von Wegen und der Mehrzahl von Teilwegen auf dem Genom für jeden des einen oder der mehreren Schadstoffe, die in der gesammelten Probe identifiziert werden, und wobei die GPM-Karte einen Wert von 0 oder 1 basierend auf einem ersten vordefinierten Kriterium aufweist, und wobei die GPM die Informationen über alle Teilwege für einen gegebenen Schadstoffabbauweg bereitstellt, die innerhalb jedes der Mikrobengenomen vorhanden sind, die in der GPM aufgelistet sind; und
Erstellen der GPE-Karte, wobei die GPE-Karte einen Wert von 0 oder 1 basierend auf einem zweiten vordefinierten Kriterium aufweist;
Identifizieren einer Liste von teilweisen Schadstoffabbaumitteln und einer Liste von vollständigen Schadstoffabbaumitteln für jeden des einen oder der mehreren Schadstoffe, die in der Probe identifiziert werden, unter Verwendung der Informationen aus der Wissensdatenbank, wobei sich teilweise Schadstoffabbaumittel auf Mikroben beziehen, die einen oder mehrere Teilwege und den entsprechenden Satz von Genen, codierten Proteinen oder Enzymen, die einen Schadstoff in eine Zwischenverbindung umwandeln, beitragen, und wobei mehrere teilweise Abbaumittel kombinatorisch alle Teilwege für den vollständigen Abbau des Schadstoffs beitragen, der in der gesammelten Probe identifiziert wird, wobei vollständige Schadstoffabbaumittel eine Kombination aller Teilwege und des entsprechenden Satzes von Genen, codierten Proteinen oder Enzymen innerhalb einer einzelnen Mikrobe für den Abbau des Schadstoffs besitzen, der in der gesammelten Probe identifiziert wird;
Erstellen einer Karte von Mikroben unter Verwendung der Informationen aus der Wissensdatenbank, wobei die Karte von Mikroben Informationen über einen oder mehrere der teilweisen Schadstoffabbaumittel und vollständigen Schadstoffabbaumittel umfasst, die in der Lage sind, j eden Schadstoff innerhalb des einen oder der mehreren Schadstoffe, die in der Probe identifiziert werden, auf unterschiedliche Abbaugrade abzubauen, wobei sich die unterschiedlichen Abbaugrade für den Schadstoff auf den Abbau eines Schadstoffs auf unterschiedliche Zwischenverbindungen oder Metabolite beziehen und wobei die Zwischenverbindungen oder Metabolite durch Endprodukt(e) bestimmt werden, das/die von dem Abbaumittel bei der Einwirkung von Genen oder Proteinen oder Enzymen freigesetzt wird/werden, die dem/den Teilweg(en) entsprechen, der/die innerhalb des Genoms des Abbaumittels für den Abbau des Schadstoffs vorhanden ist/sind, und wobei die Zwischenverbindungen entweder in die Umgebung freigesetzt und von anderen Mikroben innerhalb der Umgebung verwendet werden oder innerhalb derselben Mikrobe, die diesen Abbau ausführt, assimiliert werden können;
Entwerfen eines ersten mikrobiellen Konsortiums unter Verwendung der erstellten Karte von Mikroben, die Mikroben umfasst, die zusammen Teilwege beitragen, die für den vollständigen Abbau des einen oder der mehreren Schadstoffe, die in der Probe identifiziert werden, erforderlich sind, und wobei die Mikroben zusammen in derselben Umgebungsnische überleben können, aus der die Probe gesammelt wurde;
Entwerfen eines zweiten mikrobiellen Konsortiums unter Verwendung der erstellten Karte von Mikroben, die Mikroben umfasst, die zusammen Gene, Proteine und Enzyme für Teilwege beitragen, die für den teilweisen Abbau des einen oder der mehreren Schadstoffe, die in der gesammelten Probe identifiziert werden, auf das/die gewünschte(n) Zwischenprodukt(e) erforderlich sind, wobei die Mikroben, die das zweite mikrobielle Konsortium bilden, zusammen in der Umgebungsnische überleben können, aus der die Probe gesammelt wurde;
ein Verabreichungsmodul (114) zum Verabreichen einer Mixtur von mindestens einem oder beiden des ersten mikrobiellen Konsortiums und des zweiten mikrobiellen Konsortiums an die Umgebungsstelle, die den einen oder die mehreren Schadstoffe enthält;
ein Wirksamkeitsmodul (116) zum Überprüfen der Wirksamkeit der verabreichten Mixturen bei der Eliminierung von einem oder mehreren Schadstoffen in einer Probe, die von der Umgebungsstelle gesammelt wurde, wobei die Beurteilung der Wirksamkeit durch Isolieren und Identifizieren des verbleibenden Satzes von Schadstoffen aus der gesammelten Probe erfolgt; und
das Verabreichungsmodul (114) zum erneuten Verabreichen einer neuen Mixtur an die Umgebungsstelle, wobei die neue Mixtur durch Hinzufügen eines Satzes von Mikroben erfolgt, die als teilweise Abbaumittel wirken und den einen oder die mehreren Schadstoffe, die in der gesammelten Probe identifiziert werden, kombinatorisch abbauen können.

14. Ein oder mehrere nichtflüchtige maschinenlesbare Informationsspeichermedien, die eine oder mehrere Anweisungen umfassen, die, wenn sie von einem oder mehreren Hardwareprozessoren ausgeführt werden, bewirken:
Sammeln einer Probe von einer Umgebung, die den einen oder die mehreren Schadstoffe enthält;
Isolieren und Identifizieren des einen oder der mehreren Schadstoffe, die in der Probe vorhanden sind;
Erstellen einer Wissensbasis, wobei die Wissensbasis speichert
Informationen über den identifizierten einen oder die identifizierten mehreren Schadstoffe,
Informationen über vollständige Abbauwege und teilweise Abbauwege, die in Mikroben identifiziert werden, die in der Lage sind, den einen oder die mehreren Schadstoffe vollständig abzubauen oder den einen oder die mehreren Schadstoffe teilweise abzubauen,
Informationen über jeweilige Umgebungsnischen, in denen die Mikroben gedeihen, und
eine Liste von Mikroben aus verschiedenen Umgebungen, die den bestimmten vollständigen/teilweisen Schadstoffabbauweg besitzen,
wobei sich der bestimmte vollständige Abbauweg auf einen Satz von Genen auf einem Genom einer Mikrobe und/oder von der Mikrobe codierte Proteine bezieht, wobei der Satz von Genen und/oder codierten Proteinen für den vollständigen Abbau eines Schadstoffs entweder für Verbindungen, die für die Umgebung sicher sind, oder für Verbindungen, die von (einer) anderen Mikrobe(n), die sich in der Umgebung befinden, assimiliert werden können, verantwortlich ist,
wobei sich der teilweise Abbauweg in der Mikrobe auf einen Satz von Genen oder codierten Proteinen bezieht, die einen oder mehrere Teilwege bilden, wobei ein Teilweg eine Teilmenge des vollständigen Abbauwegs ist, der im Genom der Mikrobe codiert ist, und der Teilweg den Schadstoff zu einer Zwischenverbindung abbaut, die von der Mikrobe in die Umgebung freigesetzt werden kann und anschließend von einer anderen Mikrobe in der Umgebung aufgenommen wird, wobei die andere Mikrobe einen anderen Teilweg besitzt, der die freigesetzte Zwischenverbindung metabolisiert,
wobei die Wissensdatenbank eine Schadstoffweg-Organismus-Matrix (PPOM), eine Genomweg-Enzym-Karte (GPE-Karte), eine Genomweg-Master-Karte (GPM-Karte) und eine Datenbank von zahlreichen Umgebungsmikroben (DEBG) umfasst, wobei die GPE-Karte Folgendes umfasst:
Mikrobennamen, die in der DEBG aufgelistet sind, und
Informationen über die aktive Stelle jedes Enzyms, das an einer Mehrzahl von Teilwegen auf jedem Genom der Mikrobe beteiligt ist, für jeden des einen oder der mehreren Schadstoffe, die in der gesammelten Probe identifiziert werden,
wobei der Schritt des Erstellens der Wissensdatenbank umfasst:
Anwenden von Literatur-Mining-Techniken, um (einen) Abbauweg(e) und entsprechende Gene/Proteine in Mikroben zu identifizieren, wobei der (die) Weg(e) den einen oder die mehreren isolierten und identifizierten Schadstoffe abbaut (abbauen), und wobei das Literatur-Mining auch zur Identifizierung eines Satzes von Mikroben führt, in denen der (die) Abbauweg(e) charakterisiert ist (sind), und wobei das Literatur-Mining zum Erhalten von Informationen über die Umgebungsnische führt, in der sich die Mikroben befinden und isoliert sind;
Identifizieren einer Mehrzahl von Teilwegen innerhalb des Abbauwegs, die den isolierten einen oder die isolierten mehreren Schadstoffe vollständig oder teilweise abbauen, wobei die Gene und/oder Proteine oder Enzyme, die jedem der Mehrzahl von Teilwegen entsprechen, durch das Genom einer einzelnen Mikrobe oder die Genome einer Mehrzahl von Mikroben codiert sind, und das Produkt, das von jedem der Mehrzahl von Teilwegen gebildet wird, in die Umgebung freigesetzt wird, und wobei das Produkt von der einzelnen Mikrobe metabolisiert wird oder von (einer) anderen Mikrobe(n) aufgenommen wird, die die Umgebung bewohnt/bewohnen, und wobei die andere Mehrzahl von Mikroben die Fähigkeit besitzt, das Produkt zu metabolisieren;
Erstellen der PPOM unter Verwendung der Informationen über den identifizierten Abbauweg für jeden des einen oder der mehreren identifizierten Schadstoffe, der Mehrzahl von Teilwegen für den Abbauweg, des Satzes von Mikroben, in denen der Abbauweg charakterisiert ist, und der Informationen, die auf dem Literatur-Mining und der manuellen Kuration über die jeweilige(n) Umgebungsnische(n) basieren, aus der/denen der Satz von Mikroben isoliert ist;
Anwenden der Literatur-Mining-Techniken, um die DEBG zu erstellen, wobei die DEBG Informationen umfasst, die sich auf (eine) Mikrobe(n) und die verschiedenen Umgebungsnischen beziehen, in denen die Mikrobe(n) gedeiht/gedeihen;
Erstellen einer Weg-Domänen-Karte (PDM) aus einer vorerstellten Proteinfamilien-Datenbank (pfamDB), wobei die Proteindomänen, die in der PDM enthalten sind, diejenigen sind, die Genen/Proteinen entsprechen, die die Mehrzahl von Teilwegen bilden, die jeden Abbauweg umfassen, der in der erstellten PPOM für den einen oder die mehreren Schadstoffe vorhanden ist;
Erstellen einer Genomkarte (GM), wobei die Genomkarte Informationen umfasst, die sich auf alle Mikrobengenome beziehen, die im National Centre of Biotechnology Information, NCBI, berichtet werden, wobei die Informationen auch eine Auflistung von Genen umfassen, die nach ihren jeweiligen genomischen Orten in einer Mikrobe sowie den konstituierenden Proteindomänen, die innerhalb dieser Gene codiert sind, geordnet sind;
Suchen des Vorhandenseins von Proteindomänen, die in der PDM enthalten sind, für jeden der Mehrzahl von Teilwegen für alle Wege, die in der PPOM auf den Genomen von Mikroben aufgelistet sind, die in der DEBG gespeichert sind, um das Auftreten dieser Teilwege auf den Genomen zu bestimmen, wobei die Suche unter Verwendung der Genomkarte GM als eine Datenbank durchgeführt wird, und wobei der Teilweg aus der PDM als vorhanden betrachtet wird, wenn eine Anzahl von Domänen in dem Genom, die zu diesem Teilweg beitragen, wie in der PDM aufgelistet, innerhalb einer Fenstergröße von Genen auf dem Genom auftreten und einen vordefinierten Schwellenwert überschreiten;
Erstellen der GPM mit Mikrobennamen, die den Mikrobengenomen in der DEBG entsprechen, und Informationen über das Vorhandensein oder Fehlen einer Mehrzahl von Wegen und der Mehrzahl von Teilwegen auf dem Genom für jeden des einen oder der mehreren Schadstoffe, die in der gesammelten Probe identifiziert werden, und wobei die GPM-Karte einen Wert von 0 oder 1 basierend auf einem ersten vordefinierten Kriterium aufweist, und wobei die GPM die Informationen über alle Teilwege für einen gegebenen Schadstoffabbauweg bereitstellt, die innerhalb jedes der Mikrobengenomen vorhanden sind, die in der GPM aufgelistet sind; und
Erstellen der GPE-Karte, wobei die GPE-Karte einen Wert von 0 oder 1 basierend auf einem zweiten vordefinierten Kriterium aufweist;
Identifizieren einer Liste von teilweisen Schadstoffabbaumitteln und einer Liste von vollständigen Schadstoffabbaumitteln für jeden des einen oder der mehreren Schadstoffe, die in der Probe identifiziert werden, unter Verwendung der Informationen aus der Wissensdatenbank, wobei sich teilweise Schadstoffabbaumittel auf Mikroben beziehen, die einen oder mehrere Teilwege und den entsprechenden Satz von Genen, codierten Proteinen oder Enzymen, die einen Schadstoff in eine Zwischenverbindung umwandeln, beitragen, und wobei mehrere teilweise Abbaumittel kombinatorisch alle Teilwege für den vollständigen Abbau des Schadstoffs beitragen, der in der gesammelten Probe identifiziert wird, wobei vollständige Schadstoffabbaumittel eine Kombination aller Teilwege und des entsprechenden Satzes von Genen, codierten Proteinen oder Enzymen innerhalb einer einzelnen Mikrobe für den Abbau des Schadstoffs besitzen, der in der gesammelten Probe identifiziert wird;
Erstellen einer Karte von Mikroben unter Verwendung der Informationen aus der Wissensdatenbank, wobei die Karte von Mikroben Informationen über einen oder mehrere der teilweisen Schadstoffabbaumittel und vollständigen Schadstoffabbaumittel umfasst, die in der Lage sind, j eden Schadstoff innerhalb des einen oder der mehreren Schadstoffe, die in der Probe identifiziert werden, auf unterschiedliche Abbaugrade abzubauen, wobei sich die unterschiedlichen Abbaugrade für den Schadstoff auf den Abbau eines Schadstoffs auf unterschiedliche Zwischenverbindungen oder Metabolite beziehen und wobei die Zwischenverbindungen oder Metabolite durch Endprodukt(e) bestimmt werden, das/die von dem Abbaumittel bei der Einwirkung von Genen oder Proteinen oder Enzymen freigesetzt wird/werden, die dem/den Teilweg(en) entsprechen, der/die innerhalb des Genoms des Abbaumittels für den Abbau des Schadstoffs vorhanden ist/sind, und wobei die Zwischenverbindungen entweder in die Umgebung freigesetzt und von anderen Mikroben innerhalb der Umgebung verwendet werden oder innerhalb derselben Mikrobe, die diesen Abbau ausführt, assimiliert werden können;
Entwerfen eines ersten mikrobiellen Konsortiums unter Verwendung der erstellten Karte von Mikroben, die Mikroben umfasst, die zusammen Teilwege beitragen, die für den vollständigen Abbau des einen oder der mehreren Schadstoffe, die in der Probe identifiziert werden, erforderlich sind, und wobei die Mikroben zusammen in derselben Umgebungsnische überleben können, aus der die Probe gesammelt wurde;
Entwerfen eines zweiten mikrobiellen Konsortiums unter Verwendung der erstellten Karte von Mikroben, die Mikroben umfasst, die zusammen Gene, Proteine und Enzyme für Teilwege beitragen, die für den teilweisen Abbau des einen oder der mehreren Schadstoffe, die in der gesammelten Probe identifiziert werden, auf das/die gewünschte(n) Zwischenprodukt(e) erforderlich sind, wobei die Mikroben, die das zweite mikrobielle Konsortium bilden, zusammen in der Umgebungsnische überleben können, aus der die Probe gesammelt wurde;
Verabreichen einer Mixtur von mindestens einem oder beiden des ersten mikrobiellen Konsortiums und des zweiten mikrobiellen Konsortiums an die Umgebungsstelle, die den einen oder die mehreren Schadstoffe enthält, unter Verwendung eines Verabreichungsmoduls (114);
Überprüfen der Wirksamkeit der verabreichten Mixturen bei der Eliminierung von einem oder mehreren Schadstoffen in einer Probe, die von der Umgebungsstelle gesammelt wurde, wobei die Beurteilung der Wirksamkeit durch Isolieren und Identifizieren des verbleibenden Satzes von Schadstoffen aus der gesammelten Probe unter Verwendung eines Wirksamkeitsmoduls (116) erfolgt; und
erneutes Verabreichen einer neuen Mixtur an die Umgebungsstelle, wobei die neue Mixtur durch Hinzufügen eines Satzes von Mikroben erfolgt, die als teilweise Abbaumittel wirken und den einen oder die mehreren Schadstoffe, die in der gesammelten Probe identifiziert werden, kombinatorisch abbauen können, unter Verwendung des Verabreichungsmoduls (114).

## Revendications

1. Procédé (200) de bioremédiation d'un ou plusieurs polluants, le procédé comprenant :
la collecte d'un échantillon à partir d'un site environnemental contenant les un ou plusieurs polluants (202) ;
l'isolement et l'identification des un ou plusieurs polluants présents dans l'échantillon (204) ;
la création d'une base de connaissances, dans lequel la base de connaissances stocke
des informations des un ou plusieurs polluants identifiés,
des informations concernant des voies de dégradation complètes et des voies de dégradation partielles identifiées dans des microbes qui sont capables de dégrader complètement les un ou plusieurs polluants ou de dégrader partiellement les un ou plusieurs polluants,
des informations concernant des niches environnementales respectives dans lesquelles les microbes prospèrent, et
une liste de microbes provenant de différents environnements possédant la voie de dégradation de polluant complète/partielle particulière,
dans lequel la voie de dégradation complète particulière fait référence à un ensemble de gènes sur un génome d'un microbe et/ou de protéines codées par le microbe, dans lequel l'ensemble de gènes et/ou de protéines codées sont responsables d'une dégradation complète d'un polluant soit à des composés qui sont sûrs pour le site environnemental, soit à des composés qui peuvent être assimilés par d'autre(s) microbe(s) résidant dans l'environnement,
dans lequel la voie de dégradation partielle dans le microbe fait référence à un ensemble de gènes ou de protéines codées qui constituent une ou plusieurs sous-voies, dans lequel une sous-voie est un sous-ensemble de la voie de dégradation complète codée dans le génome du microbe, et la sous-voie dégrade le polluant en un composé intermédiaire qui peut être libéré dans l'environnement par le microbe et est ensuite repris par un autre microbe dans l'environnement, dans lequel l'autre microbe possède une autre sous-voie qui métabolise le composé intermédiaire libéré,
dans lequel la base de connaissances comprend une matrice d'organisme de voie de polluant (PPOM), une carte d'enzyme de voie de génome (GPE), une carte de maître de voie de génome (GPM) et une base de données de microbes environnementaux abondants (DEBG), dans lequel la carte de GPE comprend :
des noms de microbes répertoriés dans la DEBG, et
des informations concernant un site actif de chaque enzyme impliquée dans une pluralité de sous-voies sur chaque génome du microbe, pour chacun des un ou plusieurs polluants identifiés dans l'échantillon collecté (206),
dans lequel l'étape de création de la base de connaissances comprend :
l'utilisation de techniques d'exploration de littérature pour identifier une (des) voie(s) de dégradation et des gènes/protéines correspondants dans des microbes, dans lequel la (les) voie(s) dégradent les un ou plusieurs polluants isolés et identifiés, et dans lequel l'exploration de littérature conduit également à l'identification d'un ensemble de microbes dans lequel la (les) voie(s) de dégradation sont caractérisées, et dans lequel l'exploration de littérature conduit à l'obtention d'informations sur la niche environnementale dans laquelle les microbes résident et sont isolés ;
l'identification d'une pluralité de sous-voies dans la voie de dégradation qui dégradent complètement ou partiellement les un ou plusieurs polluants isolés, dans lequel les gènes et/ou protéines ou enzymes correspondant à chacune de la pluralité de sous-voies sont codés par le génome d'un microbe unique ou les génomes d'une pluralité de microbes, et le produit formé par chacune de la pluralité de sous-voies est libéré dans le site environnemental, et dans lequel le produit est métabolisé par le microbe unique ou est repris par d'autre(s) microbe(s) habitant l'environnement, et dans lequel l'autre pluralité de microbes possède la capacité de métaboliser le produit ;
la création de la PPOM en utilisant les informations sur la voie de dégradation identifiée pour chacun des un ou plusieurs polluants identifiés, la pluralité de sous-voies pour la voie de dégradation, l'ensemble de microbes dans lequel la voie de dégradation est caractérisée et les informations basées sur l'exploration de littérature et la curation manuelle concernant la (les) niche(s) environnementale(s) respective(s) à partir desquelles l'ensemble de microbes sont isolés ;
l'utilisation des techniques d'exploration de littérature pour créer la DEBG, dans lequel la DEBG comprend des informations concernant un (des) microbe(s) et les différentes niches environnementales dans lesquelles le (les) microbe(s) prospèrent ;
la création d'une carte de domaine de voie (PDM) à partir d'une base de données de familles de protéines pré-créées (pfamDB), dans lequel les domaines de protéines inclus dans la PDM sont ceux correspondant à des gènes/protéines constituant la pluralité de sous-voies qui comprennent chaque voie de dégradation présente dans la PPOM créée pour les un ou plusieurs polluants ;
la création d'une carte de génome (GM), dans lequel la carte de génome comprend des informations concernant tous les génomes de microbes rapportés dans le Centre national d'informations de biotechnologie, NCBI, dans lequel les informations comprennent également une liste de gènes ordonnés selon leurs emplacements génomiques respectifs dans un microbe ainsi que les domaines de protéines constitutifs codés dans ces gènes ;
la recherche de la présence de domaines de protéines inclus dans la PDM pour chacune de la pluralité de sous-voies pour toutes les voies répertoriées dans la PPOM sur les génomes de microbes stockés dans la DEBG pour déterminer l'occurrence de ces sous-voies sur les génomes, dans lequel la recherche est effectuée en utilisant la carte de génome GM en tant que base de données, et dans lequel la sous-voie à partir de la PDM est considérée comme étant présente si un certain nombre de domaines dans le génome contribuant à cette sous-voie telle que répertoriée dans la PDM se produisent dans une taille de fenêtre de gènes sur le génome et croisent une valeur de seuil prédéfinie ;
la création de la GPM avec des noms de microbes correspondant aux génomes de microbes dans la DEBG, et des informations concernant la présence ou l'absence d'une pluralité de voies et de la pluralité de sous-voies sur le génome, pour chacun des un ou plusieurs polluants identifiés dans l'échantillon collecté, et dans lequel la carte de GPM a une valeur de 0 ou 1 sur la base d'un premier critère prédéfini, et dans lequel la GPM fournit les informations concernant toutes les sous-voies pour une voie de dégradation de polluant donnée qui sont présentes dans chacun des génomes de microbes répertoriés dans la GPM ; et
la création de la carte de GPE, dans lequel la carte de GPE a une valeur de 0 ou 1 sur la base d'un second critère prédéfini ;
l'identification d'une liste de dégradants de polluants partiels et d'une liste de dégradants de polluants complets pour chacun des un ou plusieurs polluants identifiés dans l'échantillon en utilisant les informations provenant de la base de connaissances, dans lequel les dégradants de polluants partiels se réfèrent à des microbes qui contribuent à une ou plusieurs sous-voies et à l'ensemble correspondant de gènes, de protéines codées ou d'enzymes, qui convertissent un polluant en un composé intermédiaire, et dans lequel de multiples dégradants partiels contribuent de manière combinatoire à toutes les sous-voies pour la dégradation complète du polluant identifié dans l'échantillon collecté, dans lequel les dégradants de polluants complets possèdent une combinaison de toutes les sous-voies et de l'ensemble correspondant de gènes, de protéines codées ou d'enzymes dans un microbe unique pour la dégradation du polluant identifié dans l'échantillon collecté (208) ;
la création d'une carte de microbes en utilisant les informations provenant de la base de connaissances, dans lequel la carte de microbes comprend des informations d'un ou plusieurs des dégradants de polluants partiels et des dégradants de polluants complets, capables de dégrader chaque polluant dans les un ou plusieurs polluants identifiés dans l'échantillon à des degrés variables de dégradation, dans lequel les degrés variables de dégradation pour le polluant font référence à la dégradation d'un polluant en différents composés intermédiaires ou métabolites et dans lequel les composés intermédiaires ou métabolites sont déterminés par un(des) produit(s) final(aux) libéré(s) par le dégradant sous l'action de gènes ou de protéines ou d'enzymes correspondant à la (aux) sous-voie(s) présente(s) dans le génome du dégradant pour la dégradation du polluant, et dans lequel les composés intermédiaires peuvent être libérés dans l'environnement et utilisés par d'autres microbes dans l'environnement ou peuvent être assimilés dans le même microbe qui effectue cette dégradation (210) ;
la conception d'un premier consortium microbien en utilisant la carte de microbes créée comprenant des microbes qui contribuent ensemble aux sous-voies requises pour la dégradation complète des un ou plusieurs polluants identifiés dans l'échantillon et dans lequel les microbes peuvent survivre ensemble dans la même niche environnementale à partir de laquelle l'échantillon a été collecté (212) ;
la conception d'un second consortium microbien en utilisant la carte de microbes créée comprenant des microbes qui contribuent ensemble aux gènes, protéines et enzymes pour les sous-voies requises pour la dégradation partielle des un ou plusieurs polluants identifiés dans l'échantillon collecté en produit(s) intermédiaire(s) souhaité(s), dans lequel les microbes formant le second consortium microbien peuvent survivre ensemble dans la niche environnementale à partir de laquelle l'échantillon a été collecté (214) ;
l'administration d'une concoction d'au moins l'un ou les deux du premier consortium microbien et du second consortium microbien au site environnemental contenant les un ou plusieurs polluants (216) ;
la vérification de l'efficacité des concoctions administrées sur l'élimination d'un ou plusieurs polluants dans un échantillon collecté à partir du site environnemental, dans lequel l'évaluation de l'efficacité est effectuée en isolant et en identifiant l'ensemble restant de polluants à partir de l'échantillon collecté (218) ; et
la réadministration d'une nouvelle concoction sur le site environnemental, dans lequel la nouvelle concoction est faite en ajoutant un ensemble de microbes qui peuvent agir comme des dégradants partiels et dégrader de manière combinatoire les un ou plusieurs polluants identifiés dans l'échantillon collecté (220).

2. Procédé selon la revendication 1, dans lequel le premier critère prédéfini est pour chaque sous-voie dans un génome microbien :
une valeur de 0 est attribuée si des domaines de protéines correspondant à une sous-voie telle qu'enregistrée dans la « PDM » ne se produisent pas ou n'atteignent pas une valeur de seuil dans une fenêtre prédéfinie de gènes, et
une valeur de 1 est attribuée si des domaines de protéines de sous-voie tels qu'enregistrés dans la « PDM » sont présents au-dessus du seuil dans la fenêtre prédéfinie de gènes.

3. Procédé selon la revendication 2, dans lequel
la valeur de seuil est décidée sur la base de l'exploration de littérature et de la curation manuelle et correspond au nombre minimal de domaines de seuil ainsi qu'aux domaines dont la présence est requise afin de confirmer l'existence de la sous-voie dans un génome microbien, dans lequel la valeur de seuil est définie comme une fraction des domaines requis parmi le nombre total de domaines correspondant à cette sous-voie dans la PDM, et
la fenêtre prédéfinie de gènes est définie en utilisant la curation manuelle qui indique la distance en termes de nombre de gènes sur la base de l'emplacement du génome dans lequel les domaines peuvent être considérés comme constituant une sous-voie, dans lequel les gènes codant pour des domaines de protéines formant une sous-voie se produisent ensemble sur le génome microbien situé ainsi dans une taille de fenêtre définie de gènes sur le génome.

4. Procédé selon la revendication 1, dans lequel le second critère prédéfini pour chaque enzyme correspondant à chaque étape d'une sous-voie identifiée dans le génome microbien est :
une valeur de 1 est attribuée aux enzymes où un motif de site actif pour cette enzyme est trouvé, dans lequel le motif de site actif pour des enzymes constitutives de chaque voie candidate est le motif spécifique au site actif de l'enzyme obtenu par l'exploration de littérature, dans lequel le motif de site actif est identifié par des motifs qui agissent comme des séquences de signature qui aident à identifier si l'enzyme est fonctionnellement capable de se lier à un substrat et sont conservés sur toutes les enzymes ayant une fonctionnalité similaire, et dans lequel les motifs sont identifiés par un alignement de séquences multiples, MSA, sur tous les homologues fonctionnellement similaires possibles de l'enzyme pour identifier des motifs d'acides aminés conservés sur les enzymes qui sont validés dans la littérature pour évaluer l'importance fonctionnelle, et
une valeur de 0 est attribuée dans le cas où le motif de site actif pour l'enzyme n'est pas trouvé.

5. Procédé selon la revendication 1, comprenant en outre le test de la présence de capacité de sécrétion pour chaque enzyme et la mise à jour de la valeur de 0 pour l'absence ou de 1 pour la présence de la capacité de sécrétion dans la carte GPE.

6. Procédé selon la revendication 1, dans lequel le produit intermédiaire souhaité se réfère à un ensemble de produits intermédiaires dérivés pendant la dégradation partielle des un ou plusieurs polluants identifiés.

7. Procédé selon la revendication 1, dans lequel la base de connaissances stocke des informations se rapportant à un ou plusieurs polluants, dans lequel certains de ces polluants incluent le polyéthylène téréphtalate (PET), le styrène, le polyuréthane, des hydrocarbures polyaromatiques (PAH), différents congénères de polychlorobiphényles (PCB) ou des nanomatériaux à base de carbone (CBNM).

8. Procédé selon la revendication 7, dans lequel la dégradation complète de CBNM implique la présence d'une enzyme catalase-peroxydase (kat) bifonctionnelle et les sous-voies pour la dégradation d'un ou plusieurs des intermédiaires formés après l'action catalytique de l'enzyme kat, dans lequel les intermédiaires comprennent une ou plusieurs parmi la dégradation de PAH, de PCB et d'hydrocarbure aromatique unique, SAH, formée à la suite de l'action catalytique de catalase-peroxydase sur CBNM.

9. Procédé selon la revendication 7, dans lequel la dégradation complète de PET implique les sous-voies pour la conversion de PETase suivie par l'acide téréphtalique, TPA, en acide protocatéchuique, PCA, et la famille de bactéries candidates impliquée dans ces sous-voies pour la dégradation de PET comprenant un ou plusieurs parmi :
Polyangiaceae, Burkholderiaceae, Burkholderiales_incertae_sedis, Alteromonadaceae, Oceanospirillaceae, Pseudomonadaceae ou Vibrionaceae,
Comamonadaceae, Bacillaceae, Bradyrhizobiaceae, Burkholderiaceae, Piscirickettsiaceae, Sphingomonadaceae, Hyphomicrobiaceae, Pseudomonadaceae, Pseudonocardiaceae, Oxalobacteraceae, Rhizobiaceae, Nocardiaceae, Rhodocyclaceae ou Streptomycetaceae pour la sous-voie de l'acide téréphtalique en acide protocatéchuique, et
Actinosynnemataceae, Caulobacteraceae, Oxalobacteraceae, Streptomycetaceae, Micrococcaceae, Rhizobiaceae, Myxococcaceae, Nocardiaceae, Brucellaceae, Nocardiopsaceae, Oceanospirillaceae, Planococcaceae, Pseudonocardiaceae, Actinopolysporaceae, Streptosporangiaceae, Xanthomonadaceae, Hyphomicrobiaceae, Rhodobacteraceae, Mycobacteriaceae, Microbacteriaceae, Alcaligenaceae, Geodermatophilaceae, Burkholderiaceae, Enterobacteriaceae, Halomonadaceae, Moraxellaceae, Dietziaceae, Phyllobacteriaceae, Sphingomonadaceae, Rhodospirillaceae, Micromonosporaceae, Comamonadaceae, Pseudomonadaceae, Aeromonadaceae, Alteromonadaceae, Aurantimonadaceae, Cytophagaceae, Neisseriaceae, Deinococcaceae, Nocardioidaceae, Vibrionaceae, Kiloniellaceae, Gordoniaceae, Listeriaceae, Bacillaceae, Xanthobacteraceae, Rubrobacteraceae, Tsukamurellaceae, Bradyrhizobiaceae, Saprospiraceae, Sphingobacteriaceae, Thermaceae, Clostridiaceae, Flavobacteriaceae, Brevibacteriaceae, Corynebacteriaceae, Beijerinckiaceae, Methylobacteriaceae, Cystobacteraceae, Granulosicoccaceae, Glycomycetaceae, Bacillaceae 1, Catenulisporaceae, Sphaerobacteraceae, Bêta-protéobactéries non classées, Burkholderiales non classées, Flavobacteriales non classées, Yersiniaceae ou Vicinamibacteraceae pour la sous-voie de l'acide protocatéchuique en acétylCoA.

10. Procédé selon la revendication 7, dans lequel la dégradation complète des PAH comprend en outre :
la dégradation du naphtalène, qui comprend deux sous-voies qui dégradent le naphtalène en salicylate suivie par la dégradation du salicylate via le catéchol pour former l'acétyl Co-A,
la dégradation de l'anthracène, est divisée en sous-voies qui convertissent l'anthracène en sous-voie de dihydroxynaphtalène suivie par la dégradation du salicyalate via la voie du métabolisme du catéchol pour former l'acétyl Co-A, et
la dégradation du phénanthrène, qui implique la sous-voie du phénanthrène en phtalate, la sous-voie du phtalate en dihydroxybenzoate et la sous-voie du phénanthrène en naphtalènediol et la famille de bactéries candidates impliquée dans les sous-voies pour la dégradation de chaque type du PAH mentionné ci-dessus comprenant un ou plusieurs parmi :
Comamonadaceae, Rhizobiaceae, Alteromonadaceae, Bacillaceae, Alcaligenaceae, Bradyrhizobiaceae, Burkholderiaceae, Rhodobacteraceae, Erythrobacteraceae, Piscirickettsiaceae, Gordoniaceae, Oceanospirillaceae, Aurantimonadaceae, Oxalobacteraceae, Phyllobacteriaceae, Mycobacteriaceae, Sphingomonadaceae, Hyphomicrobiaceae, Neisseriaceae, Pseudomonadaceae, Rhizobiales non classées, Nocardiaceae, Rhodocyclaceae, ou Streptomycetaceae pour la sous-voie du naphtalène en salicylate,
Comamonadaceae, Alcaligenaceae, Actinosynnemataceae, Bacillaceae, Geodermatophilaceae, Sphingomonadaceae, Bradyrhizobiaceae, Burkholderiaceae, Caulobacteraceae, Rhodobacteraceae, Frankiaceae, Alteromonadaceae, Phyllobacteriaceae, Mycobacteriaceae, Hyphomicrobiaceae, Erythrobacteraceae, Pseudomonadaceae, Rhizobiaceae, Nocardiaceae, Streptomycetaceae, ou Gammaproteobacteria_incertae_sedis pour la sous-voie de l'anthracène en dihydroxynaphtalène,
Comamonadaceae, Moraxellaceae, Alcaligenaceae, Alcanivoracaceae, Alicyclobacillaceae, Ectothiorhodospiraceae, Actinosynnemataceae, Phyllobacteriaceae, Neisseriaceae, Rhodocyclaceae, Pseudomonadaceae, Bacillaceae, Thiotrichaceae, Bradyrhizobiaceae, Burkholderiaceae, Clostridiaceae, Rhodobacteraceae, Corynebacteriaceae, Oxalobacteraceae, Piscirickettsiaceae, Frankiaceae, Gordoniaceae, Intrasporangiaceae, Enterobacteriaceae, Planococcaceae, Alteromonadaceae, Aurantimonadaceae, Mycobacteriaceae, Nakamurellaceae, Nocardiaceae, Nocardioidaceae, Sphingomonadaceae, Micrococcaceae, Rhizobiaceae, Streptomycetaceae, Sulfobacillaceae, ou Thermomonosporaceae pour la sous-voie du catéchol en acétylCoA,
Comamonadaceae, Alteromonadaceae, Phyllobacteriaceae, Bacillaceae, Bradyrhizobiaceae, Burkholderiaceae, Erythrobacteraceae, Oxalobacteraceae, Mycobacteriaceae, Sphingomonadaceae, Hyphomicrobiaceae, Micrococcaceae, Pseudomonadaceae, Rhizobiaceae, Nocardiaceae, ou Streptomycetaceae pour la sous-voie du phénanthrène en phtalate,
Acetobacteraceae, Comamonadaceae, Alcaligenaceae, Bacillaceae, Bradyrhizobiaceae, Brucellaceae, Burkholderiaceae, Halomonadaceae, Colwelliaceae, Corynebacteriaceae, Frankiaceae, Gordoniaceae, Phyllobacteriaceae, Rhodobiaceae, Mycobacteriaceae, Nocardioidaceae, Nostocaceae, Sphingomonadaceae, Rhodobacteraceae, Oxalobacteraceae, Pseudonocardiaceae, Pseudoalteromonadaceae, Pseudomonadaceae, Rhizobiaceae, Nocardiaceae, Alteromonadaceae, Streptomycetaceae, Gomphosphaeriaceae, Rhodospirillaceae, Enterobacteriaceae, ou Gammaproteobacteria_incertae_sedis pour la sous-voie du phtalate en dihydroxybenzoate, et
Comamonadaceae, Bacillaceae, Bradyrhizobiaceae, Burkholderiaceae, Caulobacteraceae, Oxalobacteraceae, Rhodocyclaceae, Frankiaceae, Halomonadaceae, Immundisolibacteraceae, Rhodobacteraceae, Alteromonadaceae, Oceanospirillaceae, Phyllobacteriaceae, Mycobacteriaceae, Nocardiaceae, Sphingomonadaceae, Hyphomicrobiaceae, Enterobacteriaceae, Erythrobacteraceae, Pseudonocardiaceae, Micrococcaceae, Pseudomonadaceae, Rhizobiaceae, ou Streptomycetaceae pour la sous-voie du phénanthrène en naphtalènediol.

11. Procédé selon la revendication 7, dans lequel la dégradation complète de PCB implique les sous-voies pour la déshalogénation réductrice de PCB plus chlorés en biphényles suivie par la sous-voie pour la conversion du biphényle en 2-hydroxypenta-2,4-diénoate qui est davantage dégradé via la voie inférieure pour former le pyruvate et l'acétyl-CoA, avec les sous-voies pour les intermédiaires formés, qui est converti en acétyl-Co-A via la voie benzoyl Co-A/catéchol et la famille de bactéries candidates impliquée dans ces sous-voies pour la dégradation de PCB comprenant un ou plusieurs parmi :
Dehalococcoidaceae, Peptococcaceae, ou Campylobacteraceae pour la sous-voie du PCB en biphényle,
Comamonadaceae, Alcaligenaceae, Alcanivoracaceae, Rhodocyclaceae, Bacillaceae, Burkholderiaceae, Conexibacteraceae, Corynebacteriaceae, Erythrobacteraceae, Frankiaceae, Aurantimonadaceae, Beijerinckiaceae, Mycobacteriaceae, Sphingomonadaceae, Paenibacillaceae, Hyphomicrobiaceae, Pseudoalteromonadaceae, Pseudomonadaceae, Pseudonocardiaceae, Xanthomonadaceae, Rhizobiaceae, Nocardiaceae, Alteromonadaceae, Planococcaceae, ou Spongiibacteraceae pour la sous-voie du biphényle en acétyl-CoA/pyruvate,
Comamonadaceae, Alcaligenaceae, Rhodocyclaceae, Bacillaceae, Bradyrhizobiaceae, Burkholderiaceae, Rhodobacteraceae, Corynebacteriaceae, Immundisolibacteraceae, Beijerinckiaceae, Mycobacteriaceae, Nocardioidaceae, Sphingomonadaceae, Pseudomonadaceae, Rhizobiaceae, Nocardiaceae, ou Streptomycetaceae pour la sous-voie du biphényle en 2-hydroxypenta-2,4-diénoate,
Comamonadaceae, Rhizobiaceae, Alcaligenaceae, Alicyclobacillaceae, Neisseriaceae, Rhodocyclaceae, Bacillaceae, Paenibacillaceae, Burkholderiaceae, Oxalobacteraceae, Gordoniaceae, Rhodospirillaceae, Aurantimonadaceae, Mycobacteriaceae, Sphingomonadaceae, Rhodobacteraceae, Planococcaceae, Pseudomonadaceae, Pseudonocardiaceae, Nocardiaceae, Streptomycetaceae, Streptosporangiaceae, ou Gammaproteobacteria_incertae_sedis pour la sous-voie du 2-hydroxypenta-2,4-diénoate en acétyl-CoA/pyruvate,
Moraxellaceae, Rhizobiaceae, Alteromonadaceae, Actinosynnemataceae, Micrococcaceae, Burkholderiaceae, Oxalobacteraceae, Geodermatophilaceae, Gordoniaceae, Halomonadaceae, Xanthobacteraceae, Methylobacteriaceae, Mycobacteriaceae, Aeromonadaceae, Rhodobacteraceae, Comamonadaceae, Neisseriaceae, Pseudomonadaceae, Pseudonocardiaceae, Sphingomonadaceae, ou Vibrionaceae pour la sous-voie du benzoate en acétyl-CoA via le catéchol, et
Comamonadaceae, Moraxellaceae, Alcaligenaceae, Rhodocyclaceae, Burkholderiaceae, Polyangiaceae, Oxalobacteraceae, Labilitrichaceae, Oceanospirillales_incertae_sedis pour la sous-voie du benzoate en acétyl-CoA via le benzoyl-CoA.

12. Procédé selon la revendication 1, dans lequel la carte de microbes capables de survivre dans le site environnemental de l'échantillon obtenu à partir de, et capables de dégrader le polluant à des degrés variables, créée en utilisant les informations provenant de la base de connaissances, dans lequel une première matrice est créée en utilisant les microbes provenant de la matrice de GPE ayant la valeur 1, une deuxième matrice est créée en utilisant les microbes provenant de la matrice de GPM ayant la valeur 1 correspondant à ses sous-voies pour un polluant isolé (Pᵢ), et une troisième matrice est créée de résultats d'organismes candidats avec des valeurs de sous-voies dans GPM et les enzymes correspondantes dans GPE comme 1, dans lequel des informations concernant la niche environnementale dans laquelle les microbes dans la troisième matrice prospèrent peuvent être obtenues à partir de la DEBG, dans lequel ces informations sont utilisées pour créer une matrice d'environnement d'organisme de polluant (POEM) comprenant chacun des un ou plusieurs polluants identifiés dans l'échantillon, les organismes capables de le dégrader à des degrés variables (en fonction de la voie complète ou des sous-voies présentes) et l'environnement à partir duquel l'organisme a été isolé et prospère.

13. Système (100) de bioremédiation d'un ou plusieurs polluants, le système comprend :
un module de collecte d'échantillon (102) pour collecter un échantillon à partir d'un site environnemental contenant les un ou plusieurs polluants ;
un module d'isolement et d'identification de polluant (104) pour isoler et identifier les un ou plusieurs polluants présents dans l'échantillon ;
un processeur (108) ;
une mémoire (106) en communication avec le processeur, dans lequel le processeur est configuré pour effectuer les étapes de :
la création d'une base de connaissances, dans lequel la base de connaissances stocke :
des informations des un ou plusieurs polluants identifiés,
des informations concernant des voies de dégradation complètes et des voies de dégradation partielles identifiées dans des microbes qui sont capables de dégrader complètement les un ou plusieurs polluants ou de dégrader partiellement les un ou plusieurs polluants,
des informations concernant des niches environnementales respectives dans lesquelles les microbes prospèrent, et
une liste de microbes provenant de différents environnements possédant la voie de dégradation de polluant complète/partielle particulière,
dans lequel la voie de dégradation complète particulière fait référence à un ensemble de gènes sur un génome d'un microbe et/ou de protéines codées par le microbe, dans lequel l'ensemble de gènes et/ou de protéines codées sont responsables d'une dégradation complète d'un polluant soit à des composés qui sont sûrs pour le site environnemental, soit à des composés qui peuvent être assimilés par d'autre(s) microbe(s) résidant dans l'environnement,
dans lequel la voie de dégradation partielle dans le microbe fait référence à un ensemble de gènes ou de protéines codées qui constituent une ou plusieurs sous-voies, dans lequel une sous-voie est un sous-ensemble de la voie de dégradation complète codée dans le génome du microbe, et la sous-voie dégrade le polluant en un composé intermédiaire qui peut être libéré dans l'environnement par le microbe et est ensuite repris par un autre microbe dans l'environnement, dans lequel l'autre microbe possède une autre sous-voie qui métabolise le composé intermédiaire libéré,
dans lequel la base de connaissances comprend une matrice d'organisme de voie de polluant (PPOM), une carte d'enzyme de voie de génome (GPE), une carte de maître de voie de génome (GPM) et une base de données de microbes environnementaux abondants (DEBG), dans lequel la carte de GPE comprend :
des noms de microbes répertoriés dans la DEBG, et
des informations concernant un site actif de chaque enzyme impliquée dans une pluralité de sous-voies sur chaque génome du microbe, pour chacun des un ou plusieurs polluants identifiés dans l'échantillon collecté,
dans lequel la création de la base de connaissances comprend :
l'utilisation de techniques d'exploration de littérature pour identifier une (des) voie(s) de dégradation et des gènes/protéines correspondants dans des microbes, dans lequel la (les) voie(s) dégradent les un ou plusieurs polluants isolés et identifiés, et dans lequel l'exploration de littérature conduit également à l'identification d'un ensemble de microbes dans lequel la (les) voie(s) de dégradation sont caractérisées, et dans lequel l'exploration de littérature conduit à l'obtention d'informations sur la niche environnementale dans laquelle les microbes résident et sont isolés ;
l'identification d'une pluralité de sous-voies dans la voie de dégradation qui dégradent complètement ou partiellement les un ou plusieurs polluants isolés, dans lequel les gènes et/ou protéines ou enzymes correspondant à chacune de la pluralité de sous-voies sont codés par le génome d'un microbe unique ou les génomes d'une pluralité de microbes, et le produit formé par chacune de la pluralité de sous-voies est libéré dans le site environnemental, et dans lequel le produit est métabolisé par le microbe unique ou est repris par d'autre(s) microbe(s) habitant l'environnement, et dans lequel l'autre pluralité de microbes possède la capacité de métaboliser le produit ;
la création de la PPOM en utilisant les informations sur la voie de dégradation identifiée pour chacun des un ou plusieurs polluants identifiés, la pluralité de sous-voies pour la voie de dégradation, l'ensemble de microbes dans lequel la voie de dégradation est caractérisée et les informations basées sur l'exploration de littérature et la curation manuelle concernant la (les) niche(s) environnementale(s) respective(s) à partir desquelles l'ensemble de microbes sont isolés ;
l'utilisation des techniques d'exploration de littérature pour créer la DEBG, dans lequel la DEBG comprend des informations concernant un (des) microbe(s) et les différentes niches environnementales dans lesquelles le (les) microbe(s) prospèrent ;
la création d'une carte de domaine de voie (PDM) à partir d'une base de données de familles de protéines pré-créées (pfamDB), dans lequel les domaines de protéines inclus dans la PDM sont ceux correspondant à des gènes/protéines constituant la pluralité de sous-voies qui comprennent chaque voie de dégradation présente dans la PPOM créée pour les un ou plusieurs polluants ;
la création d'une carte de génome (GM), dans lequel la carte de génome comprend des informations concernant tous les génomes de microbes rapportés dans le Centre national d'informations de biotechnologie, NCBI, dans lequel les informations comprennent également une liste de gènes ordonnés selon leurs emplacements génomiques respectifs dans un microbe ainsi que les domaines de protéines constitutifs codés dans ces gènes ;
la recherche de la présence de domaines de protéines inclus dans la PDM pour chacune de la pluralité de sous-voies pour toutes les voies répertoriées dans la PPOM sur les génomes de microbes stockés dans la DEBG pour déterminer l'occurrence de ces sous-voies sur les génomes, dans lequel la recherche est effectuée en utilisant la carte de génome GM en tant que base de données, et dans lequel la sous-voie à partir de la PDM est considérée comme étant présente si un certain nombre de domaines dans le génome contribuant à cette sous-voie telle que répertoriée dans la PDM se produisent dans une taille de fenêtre de gènes sur le génome et croisent une valeur de seuil prédéfinie ;
la création de la GPM avec des noms de microbes correspondant aux génomes de microbes dans la DEBG, et des informations concernant la présence ou l'absence d'une pluralité de voies et de la pluralité de sous-voies sur le génome, pour chacun des un ou plusieurs polluants identifiés dans l'échantillon collecté, et dans lequel la carte de GPM a une valeur de 0 ou 1 sur la base d'un premier critère prédéfini, et dans lequel la GPM fournit les informations concernant toutes les sous-voies pour une voie de dégradation de polluant donnée qui sont présentes dans chacun des génomes de microbes répertoriés dans la GPM ; et
la création de la carte de GPE, dans lequel la carte de GPE a une valeur de 0 ou 1 sur la base d'un second critère prédéfini ;
l'identification d'une liste de dégradants de polluants partiels et d'une liste de dégradants de polluants complets pour chacun des un ou plusieurs polluants identifiés dans l'échantillon en utilisant les informations provenant de la base de connaissances, dans lequel les dégradants de polluants partiels se réfèrent à des microbes qui contribuent à une ou plusieurs sous-voies et à l'ensemble correspondant de gènes, de protéines codées ou d'enzymes, qui convertissent un polluant en un composé intermédiaire, et dans lequel de multiples dégradants partiels contribuent de manière combinatoire à toutes les sous-voies pour la dégradation complète du polluant identifié dans l'échantillon collecté, dans lequel les dégradants de polluants complets possèdent une combinaison de toutes les sous-voies et de l'ensemble correspondant de gènes, de protéines codées ou d'enzymes dans un microbe unique pour la dégradation du polluant identifié dans l'échantillon collecté ;
la création d'une carte de microbes en utilisant les informations provenant de la base de connaissances, dans lequel la carte de microbes comprend des informations d'un ou plusieurs des dégradants de polluants partiels et des dégradants de polluants complets, capables de dégrader chaque polluant dans les un ou plusieurs polluants identifiés dans l'échantillon à des degrés variables de dégradation, dans lequel les degrés variables de dégradation pour le polluant font référence à la dégradation d'un polluant en différents composés intermédiaires ou métabolites et dans lequel les composés intermédiaires ou métabolites sont déterminés par un(des) produit(s) final(aux) libéré(s) par le dégradant sous l'action de gènes ou de protéines ou d'enzymes correspondant à la (aux) sous-voie(s) présente(s) dans le génome du dégradant pour la dégradation du polluant, et dans lequel les composés intermédiaires peuvent être libérés dans l'environnement et utilisés par d'autres microbes dans l'environnement ou peuvent être assimilés dans le même microbe qui effectue cette dégradation ;
la conception d'un premier consortium microbien en utilisant la carte de microbes créée comprenant des microbes qui contribuent ensemble aux sous-voies requises pour la dégradation complète des un ou plusieurs polluants identifiés dans l'échantillon et dans lequel les microbes peuvent survivre ensemble dans la même niche environnementale à partir de laquelle l'échantillon a été collecté ;
la conception d'un second consortium microbien en utilisant la carte de microbes créée comprenant des microbes qui contribuent ensemble aux gènes, protéines et enzymes pour les sous-voies requises pour la dégradation partielle des un ou plusieurs polluants identifiés dans l'échantillon collecté en produit(s) intermédiaire(s) souhaité(s), dans lequel les microbes formant le second consortium microbien peuvent survivre ensemble dans la niche environnementale à partir de laquelle l'échantillon a été collecté ;
un module d'administration (114) pour administrer une concoction d'au moins l'un ou les deux du premier consortium microbien et du second consortium microbien au site environnemental contenant les un ou plusieurs polluants ;
un module d'efficacité (116) pour vérifier l'efficacité des concoctions administrées sur l'élimination d'un ou plusieurs polluants dans un échantillon collecté à partir du site environnemental, dans lequel l'évaluation de l'efficacité est effectuée en isolant et en identifiant l'ensemble restant de polluants à partir de l'échantillon collecté ; et
le module d'administration (114) pour réadministrer une nouvelle concoction sur le site environnemental, dans lequel la nouvelle concoction est faite en ajoutant un ensemble de microbes qui peuvent agir comme des dégradants partiels et dégrader de manière combinatoire les un ou plusieurs polluants identifiés dans l'échantillon collecté.

14. Un ou plusieurs supports de stockage d'informations lisibles par machine non transitoires comprenant une ou plusieurs instructions qui, lorsqu'elles sont exécutées par un ou plusieurs processeurs matériels, amènent :
la collecte d'un échantillon à partir d'un site environnemental contenant les un ou plusieurs polluants ;
l'isolement et l'identification des un ou plusieurs polluants présents dans l'échantillon ;
la création d'une base de connaissances, dans lequel la base de connaissances stocke
des informations des un ou plusieurs polluants identifiés,
des informations concernant des voies de dégradation complètes et des voies de dégradation partielles identifiées dans des microbes qui sont capables de dégrader complètement les un ou plusieurs polluants ou de dégrader partiellement les un ou plusieurs polluants,
des informations concernant des niches environnementales respectives dans lesquelles les microbes prospèrent, et
une liste de microbes provenant de différents environnements possédant la voie de dégradation de polluant complète/partielle particulière,
dans lequel la voie de dégradation complète particulière fait référence à un ensemble de gènes sur un génome d'un microbe et/ou de protéines codées par le microbe, dans lequel l'ensemble de gènes et/ou de protéines codées sont responsables d'une dégradation complète d'un polluant soit à des composés qui sont sûrs pour le site environnemental, soit à des composés qui peuvent être assimilés par d'autre(s) microbe(s) résidant dans l'environnement,
dans lequel la voie de dégradation partielle dans le microbe fait référence à un ensemble de gènes ou de protéines codées qui constituent une ou plusieurs sous-voies, dans lequel une sous-voie est un sous-ensemble de la voie de dégradation complète codée dans le génome du microbe, et la sous-voie dégrade le polluant en un composé intermédiaire qui peut être libéré dans l'environnement par le microbe et est ensuite repris par un autre microbe dans l'environnement, dans lequel l'autre microbe possède une autre sous-voie qui métabolise le composé intermédiaire libéré,
dans lequel la base de connaissances comprend une matrice d'organisme de voie de polluant (PPOM), une carte d'enzyme de voie de génome (GPE), une carte de maître de voie de génome (GPM) et une base de données de microbes environnementaux abondants (DEBG), dans lequel la carte de GPE comprend :
des noms de microbes répertoriés dans la DEBG, et
des informations concernant un site actif de chaque enzyme impliquée dans une pluralité de sous-voies sur chaque génome du microbe, pour chacun des un ou plusieurs polluants identifiés dans l'échantillon collecté,
dans lequel l'étape de création de la base de connaissances comprend :
l'utilisation de techniques d'exploration de littérature pour identifier une (des) voie(s) de dégradation et des gènes/protéines correspondants dans des microbes, dans lequel la (les) voie(s) dégradent les un ou plusieurs polluants isolés et identifiés, et dans lequel l'exploration de littérature conduit également à l'identification d'un ensemble de microbes dans lequel la (les) voie(s) de dégradation sont caractérisées, et dans lequel l'exploration de littérature conduit à l'obtention d'informations sur la niche environnementale dans laquelle les microbes résident et sont isolés ;
l'identification d'une pluralité de sous-voies dans la voie de dégradation qui dégradent complètement ou partiellement les un ou plusieurs polluants isolés, dans lequel les gènes et/ou protéines ou enzymes correspondant à chacune de la pluralité de sous-voies sont codés par le génome d'un microbe unique ou les génomes d'une pluralité de microbes, et le produit formé par chacune de la pluralité de sous-voies est libéré dans le site environnemental, et dans lequel le produit est métabolisé par le microbe unique ou est repris par d'autre(s) microbe(s) habitant l'environnement, et dans lequel l'autre pluralité de microbes possède la capacité de métaboliser le produit ;
la création de la PPOM en utilisant les informations sur la voie de dégradation identifiée pour chacun des un ou plusieurs polluants identifiés, la pluralité de sous-voies pour la voie de dégradation, l'ensemble de microbes dans lequel la voie de dégradation est caractérisée et les informations basées sur l'exploration de littérature et la curation manuelle concernant la (les) niche(s) environnementale(s) respective(s) à partir desquelles l'ensemble de microbes sont isolés ;
l'utilisation des techniques d'exploration de littérature pour créer la DEBG, dans lequel la DEBG comprend des informations concernant un (des) microbe(s) et les différentes niches environnementales dans lesquelles le (les) microbe(s) prospèrent ;
la création d'une carte de domaine de voie (PDM) à partir d'une base de données de familles de protéines pré-créées (pfamDB), dans lequel les domaines de protéines inclus dans la PDM sont ceux correspondant à des gènes/protéines constituant la pluralité de sous-voies qui comprennent chaque voie de dégradation présente dans la PPOM créée pour les un ou plusieurs polluants ;
la création d'une carte de génome (GM), dans lequel la carte de génome comprend des informations concernant tous les génomes de microbes rapportés dans le Centre national d'informations de biotechnologie, NCBI, dans lequel les informations comprennent également une liste de gènes ordonnés selon leurs emplacements génomiques respectifs dans un microbe ainsi que les domaines de protéines constitutifs codés dans ces gènes ;
la recherche de la présence de domaines de protéines inclus dans la PDM pour chacune de la pluralité de sous-voies pour toutes les voies répertoriées dans la PPOM sur les génomes de microbes stockés dans la DEBG pour déterminer l'occurrence de ces sous-voies sur les génomes, dans lequel la recherche est effectuée en utilisant la carte de génome GM en tant que base de données, et dans lequel la sous-voie à partir de la PDM est considérée comme étant présente si un certain nombre de domaines dans le génome contribuant à cette sous-voie telle que répertoriée dans la PDM se produisent dans une taille de fenêtre de gènes sur le génome et croisent une valeur de seuil prédéfinie ;
la création de la GPM avec des noms de microbes correspondant aux génomes de microbes dans la DEBG, et des informations concernant la présence ou l'absence d'une pluralité de voies et de la pluralité de sous-voies sur le génome, pour chacun des un ou plusieurs polluants identifiés dans l'échantillon collecté, et dans lequel la carte de GPM a une valeur de 0 ou 1 sur la base d'un premier critère prédéfini, et dans lequel la GPM fournit les informations concernant toutes les sous-voies pour une voie de dégradation de polluant donnée qui sont présentes dans chacun des génomes de microbes répertoriés dans la GPM ; et
la création de la carte de GPE, dans lequel la carte de GPE a une valeur de 0 ou 1 sur la base d'un second critère prédéfini ;
l'identification d'une liste de dégradants de polluants partiels et d'une liste de dégradants de polluants complets pour chacun des un ou plusieurs polluants identifiés dans l'échantillon en utilisant les informations provenant de la base de connaissances, dans lequel les dégradants de polluants partiels se réfèrent à des microbes qui contribuent à une ou plusieurs sous-voies et à l'ensemble correspondant de gènes, de protéines codées ou d'enzymes, qui convertissent un polluant en un composé intermédiaire, et dans lequel de multiples dégradants partiels contribuent de manière combinatoire à toutes les sous-voies pour la dégradation complète du polluant identifié dans l'échantillon collecté, dans lequel les dégradants de polluants complets possèdent une combinaison de toutes les sous-voies et de l'ensemble correspondant de gènes, de protéines codées ou d'enzymes dans un microbe unique pour la dégradation du polluant identifié dans l'échantillon collecté ;
la création d'une carte de microbes en utilisant les informations provenant de la base de connaissances, dans lequel la carte de microbes comprend des informations d'un ou plusieurs des dégradants de polluants partiels et des dégradants de polluants complets, capables de dégrader chaque polluant dans les un ou plusieurs polluants identifiés dans l'échantillon à des degrés variables de dégradation, dans lequel les degrés variables de dégradation pour le polluant font référence à la dégradation d'un polluant en différents composés intermédiaires ou métabolites et dans lequel les composés intermédiaires ou métabolites sont déterminés par un(des) produit(s) final(aux) libéré(s) par le dégradant sous l'action de gènes ou de protéines ou d'enzymes correspondant à la (aux) sous-voie(s) présente(s) dans le génome du dégradant pour la dégradation du polluant, et dans lequel les composés intermédiaires peuvent être libérés dans l'environnement et utilisés par d'autres microbes dans l'environnement ou peuvent être assimilés dans le même microbe qui effectue cette dégradation ;
la conception d'un premier consortium microbien en utilisant la carte de microbes créée comprenant des microbes qui contribuent ensemble aux sous-voies requises pour la dégradation complète des un ou plusieurs polluants identifiés dans l'échantillon et dans lequel les microbes peuvent survivre ensemble dans la même niche environnementale à partir de laquelle l'échantillon a été collecté ;
la conception d'un second consortium microbien en utilisant la carte de microbes créée comprenant des microbes qui contribuent ensemble aux gènes, protéines et enzymes pour les sous-voies requises pour la dégradation partielle des un ou plusieurs polluants identifiés dans l'échantillon collecté en produit(s) intermédiaire(s) souhaité(s), dans lequel les microbes formant le second consortium microbien peuvent survivre ensemble dans la niche environnementale à partir de laquelle l'échantillon a été collecté ;
l'administration d'une concoction d'au moins l'un ou les deux du premier consortium microbien et du second consortium microbien au site environnemental contenant les un ou plusieurs polluants en utilisant un module d'administration (114) ;
la vérification de l'efficacité des concoctions administrées sur l'élimination d'un ou plusieurs polluants dans un échantillon collecté à partir du site environnemental, dans lequel l'évaluation de l'efficacité est effectuée en isolant et en identifiant l'ensemble restant de polluants à partir de l'échantillon collecté en utilisant un module d'efficacité (116) ; et
la réadministration d'une nouvelle concoction sur le site environnemental, dans lequel la nouvelle concoction est faite en ajoutant un ensemble de microbes qui peuvent agir comme des dégradants partiels et dégrader de manière combinatoire les un ou plusieurs polluants identifiés dans l'échantillon collecté en utilisant le module d'administration (114).
